# EUROPEAN PATENT SPECIFICATION

(11) **EP 0 386 839 B1**
(45) Date of publication and mention of the grant of the patent: **15.01.1997**
(21) Application number: 90200499.3
(22) Date of filing: 02.03.1990
(51) Int. Cl.: C07D 215/48, A61K 31/47, C07D 215/50, C07D 405/04, C07D 401/04, C07D 413/04, C07D 401/12, C07D 405/12

(54) **Tetrahydroquinoline derivatives useful for neurodegenerative disorders**
Tetrahydroquinolin-Derivate, verwendbar bei neurodegenerativen Krankheiten
Dérivés de tétrahydroquinoléine utiles pour les affections neurodégénératives

(30) Priority: 08.03.1989 GB 8905334; 22.11.1989 GB 8926431
(43) Date of publication of application: 12.09.1990
(73) Proprietor: MERCK SHARP & DOHME LTD., Hoddesdon Hertfordshire EN11 9NU (GB)
(72) Inventor: Baker, Raymond, Dr., Much Hadham, Hertfordshire (GB); Carling, William R., Dr., Bishops Stortford, Hertfordshire (GB); Leeson, Paul D., Dr., Cambridge, Cambridgeshire (GB); Smith, Julian D., Sawbridgeworth, Hertfordshire (GB)
(74) Representative: Cole, William Gwyn

(56) References cited:
- EP-A- 0 203 891
- EP-A- 0 303 387
- JOURNAL OF ORGANIC CHEMISTRY, vol. 55, no. 2, 19th January 1990, pages 738-741, American Chemical Society, Washington, DC, US; R.T. SHUMAN et al.: "An improved synthesis of homoproline and derivatives"
- CHEMICAL ABSTRACTS, vol. 110, no.19, 8th May 1989, page 61, abstract no. 165944p, Columbus, Ohio, US; I.N. GRACHEVA et al.: "Hydrazides and sulfonyl hydrazides of quinolines as blood serum monoamine oxidase inhibitors", & KHIM.-FARM. ZH. 1988, 22(11), 1336-9
- CHEMICAL ABSTRACTS, vol. 70, no. 5, 3rd February 1969, page 1997, abstract no. 20044r, Columbus, Ohio, US; & JP-A-68 04 273

## Description

This invention relates to a class of 4-substituted 1,2,3,4-tetrahydroquinolines which are selective non-competitive antagonists of N-methyl-D-aspartate (NMDA) receptors and are therefore useful in the treatment and/or prevention of neurodegenerative disorders arising as a consequence of such pathological conditions as stroke, hypoglycaemia, cerebral palsy, transient cerebral ischaemic attack, cerebral ischaemia during cardiac pulmonary surgery or cardiac arrest, perinatal asphyxia, epilepsy, Huntington's chorea, Alzheimer's disease, Olivo-ponto-cerebellar atrophy, anoxia such as from drowning, spinal cord and head injury and poisoning by exogenous NMDA receptor agonists and neurotoxins.

Various N-acyl-substituted derivatives of 1,2,3,4-tetrahydroquinoline-2-carboxylic acid, optionally substituted on the benzo moiety of the tetrahydroquinoline structure, are known to have angiotensin converting enzyme (ACE) inhibitory activity and are thus effective antihypertensive agents. Such compounds are described in, for example, DE-A-2937779, US-4273927, US-4374246, US-4390700, US-4401818, US-4461896, EP-A-0029488, J. Med. Chem., 1983, 26, 1267 and J. Med. Chem., 1985, 28, 1606. The parent compound in this series, 1,2,3,4-tetrahydroquinoline-2-carboxylic acid, is known from Chem. Ber., 1928, 61, 2377. None of the compounds specifically disclosed in any of the abovementioned documents, however, possesses a substituent in the 4-position of the tetrahydroquinoline moiety. Moreover, there is no suggestion in any of these documents that the compounds described therein may be useful in the treatment and/or prevention of neurodegenerative disorders.

The compounds 4-oxo-1,2,3,4-tetrahydroquinoline-2-carboxylic acid and its methyl ester, and 8-hydroxy-4-oxo-1,2,3,4-tetrahydroquinoline-2-carboxylic acid, are described in J. Am. Chem. Soc., 1967, 89, 1017. However, no therapeutic utility is disclosed therein for these compounds.

The compound 2-aminocarbonyl-4-methoxycarbonyl-1,2,3,4-tetrahydroquinoline, and its preparation from methyl 2-carbamoylcinchonate, are described in *Chem. Abstr.,* 1969, **70**, 20044r. No pharmaceutical utility is, however, ascribed therein to this compound.

The preparation of 1,2,3,4-tetrahydroquinoline-2,4-dicarboxylic acid and its diethyl ester, by zinc/formic acid reduction of the corresponding quinolinedicarboxylic acid and subsequent esterification, is described in Collect. Czech. Chem. Commun., 1978, 43, 1413. Again, however, no therapeutic utility for these compounds is disclosed.

We have now found that a class of 1,2,3,4-tetrahydroquinolines, substituted at the 4-position of the tetrahydroquinoline ring system, are potent and selective non-competitive NMDA antagonists. They therefore have useful neuroprotective activity. The compounds act by selectively inhibiting the glycine modulation of NMDA receptors.

EP-A-0203891 and US-4746653 describe inter alia certain phosphonic acid derivatives of 1,2,3,4-tetrahydroquinoline-2-carboxylic acid. The compounds described in EP-A-0203891 and US-4746653 are stated to be competitive antagonists of the NMDA-sensitive amino acid receptor.

Hence, the mechanism of action of the compounds described in EP-A-0203891 and US-4746653 differs from that of the compounds according to the present invention in that the former compounds are competitive inhibitors of glutamate binding at the neurotransmitter recognition site whereas, as mentioned above, the latter compounds are non-competitive NMDA antagonists, acting as inhibitors at the glycine modulatory site on the NMDA receptor.

EP-A-0303387 describes a class of substituted kynurenic acid derivatives which were found to act as NMDA antagonists by selectively inhibiting the glycine modulation of NMDA receptors. These compounds are therefore stated therein to be useful in the treatment of neurodegenerative disorders. The kynurenic acid compounds described in EP-A-0303387 possess the 4-oxo-1,4-dihydroquinoline substitution pattern; they are not 1,2,3,4-tetrahydroquinolines.

The present invention provides a compound of formula IIC or a salt thereof: 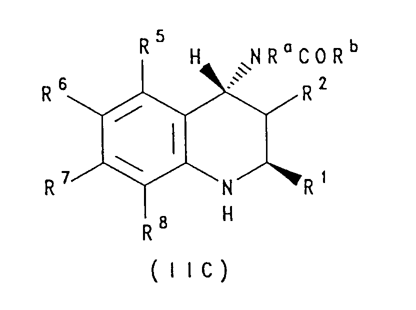 wherein
R¹ represents a carboxy group or a group which is convertible thereto in vivo;
R² represents hydrogen;
R⁵, R⁶, R⁷ and R⁸ independently represent hydrogen, halogen, cyano, trifluoromethyl, nitro, amino, carboxy, C₁₋₆ alkyl, C₁₋₆ alkoxy, C₁₋₆ alkylthio or C₁₋₆ alkoxycarbonyl; and
R^{a} and R^{b} independently represent hydrogen; or C₁₋₆ alkyl, C₂₋₆ alkenyl, C₃₋₇ cycloalkyl, C₃₋₇ cycloalkyl(C₁₋₆)alkyl, aryl selected from phenyl, naphthyl and fluorenyl, aryl(C₁₋₆)alkyl, C₃₋₇ heterocycloalkyl, C₃₋₇ heterocycloalkyl(C₁₋₆)alkyl, heteroaryl selected from pyridyl, quinolyl, isoquinolyl, pyridazinyl, pyrimidinyl, pyrazinyl, pyranyl, furyl, benzofuryl, thienyl, benzthienyl, indolinyl, imidazolyl, oxadiazolyl, thiadiazolyl and tetrazolyl, or heteroaryl(C₁₋₆)alkyl, any of which groups may be optionally substituted by one or more groups selected from methyl, ethyl, phenyl, chloro, aminomethyl, aminoethyl, aminopropyl, aminobutyl, methylaminomethyl, dimethylaminomethyl, aminopropynyl, aminobutynyl, hydroxy, methoxy, phenoxy, nitro, cyano, carboxy, acetyl, amino and acetylamino.

For use in medicine, the salts of the compounds according to the invention will be non-toxic pharmaceutically acceptable salts. Other salts may, however, be useful in the preparation of the compounds according to the invention or of their non-toxic pharmaceutically acceptable salts. Suitable pharmaceutically acceptable salts of the compounds of this invention include alkali metal salts, e.g. sodium or potassium salts; alkaline earth metal salts, e.g. calcium or magnesium salts; and salts formed with suitable organic ligands, e.g. quaternary ammonium salts. Where appropriate, acid addition salts may, for example, be formed by mixing a solution of the compound according to the invention with a solution of a pharmaceutically acceptable non-toxic acid such as hydrochloric acid, fumaric acid, maleic acid, succinic acid, acetic acid, citric acid, tartaric acid, carbonic acid or phosphoric acid.

The expression "C₁₋₆ alkyl" as used herein refers to straight-chained and branched alkyl groups containing from 1 to 6 carbon atoms. Typical examples include methyl and ethyl groups, and straight-chained or branched propyl and butyl groups. Particular alkyl groups are methyl, ethyl, n-propyl, isopropyl, n-butyl, isobutyl and t-butyl.

The expression "C₂₋₆ alkenyl" as used herein refers to straight-chained and branched alkenyl groups containing from 2 to 6 carbon atoms. Typical examples include vinyl and allyl groups.

Suitable alkynyl groups include straight-chained and branched alkynyl groups containing from 2 to 6 carbon atoms. Typical examples include ethynyl and propargyl groups.

Particular C₃₋₇ cycloalkyl groups are cyclopropyl and cyclohexyl.

As used herein, the term "aryl" refers to groups selected from phenyl, naphthyl and fluorenyl groups.

A particular aryl(C₁₋₆)alkyl group is benzyl.

Suitable heterocycloalkyl groups include piperidyl, piperazinyl and morpholinyl groups.

As used herein, the term "heteroaryl" refers to groups selected from pyridyl, quinolyl, isoquinolyl, pyridazinyl, pyrimidinyl, pyrazinyl, pyranyl, furyl, benzofuryl, thienyl, benzthienyl, indolinyl, imidazolyl, oxadiazolyl, thiadiazolyl and tetrazolyl groups. Particular heteroaryl groups are pyridyl, furyl, thienyl, indolinyl and oxadiazolyl.

The groups of formula R^{a} and R^{b}, where these are other than hydrogen, may in turn be optionally substituted by one or more groups selected from methyl, ethyl, phenyl, chloro, aminomethyl, aminoethyl, aminopropyl, aminobutyl, methylaminomethyl, dimethylaminomethyl, aminopropynyl, aminobutynyl, hydroxy, methoxy, phenoxy, nitro, cyano, carboxy, acetyl, amino and acetylamino.

The acidic group R¹ may represent carboxy, or a group convertible thereto in vivo such as an in vivo hydrolysable ester or amido group. Such groups may be represented by the moiety -(CH₂)ₙCOT wherein n is zero, and T is OR or NR^{p}R^{q}, where R is hydrogen, a hydrocarbon moiety such as C₁₋₆ alkyl, or an in vivo hydrolysable ester residue and R^{p} and R^{q} are independently hydrogen, a hydrocarbon moiety such as C₁₋₆ alkyl, or in vivo hydrolysable amido residues.

Examples of suitable in vivo hydrolysable ester and amido groups for R¹ include those which break down readily in the human body to leave the parent acid or its salt. Suitable ester and amido groups R¹ of this type include those of part formulae (i)-(vi):

-CO.Z.CH(R^{x}).O.CO.R^{y} (i)

-CO.Z.CH₂.Y.R^{f} (ii)

-CO.Z.R^{c}.NR^{d}R^{e} (iii)

-CO.Z.R^{c}.OR^{h} (iv)

-CO.Z.R^{c}.CO₂R^{h} (v)

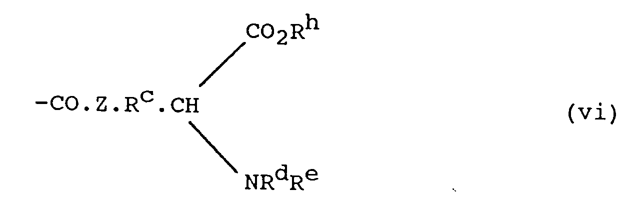

wherein Z is O or NH; R^{x} is hydrogen, C₁₋₆ alkyl or phenyl; R^{y} is C₁₋₆ alkyl, C₁₋₆ alkoxy or phenyl, any of which may be optionally substituted by a group of formula -NR^{d}R^{e}; or R^{x} and R^{y} together form a 1,2-phenylene group; R^{c} represents C₁₋₆ alkylene optionally substituted by C₁₋₆ alkyl or by a carbonyl group; R^{d} and R^{e} independently represent hydrogen, C₁₋₆ alkyl, amino(C₁₋₆)alkyl, mono- or di(C₁₋₆)alkylamino(C₁₋₆)alkyl, aryl or aryl(C₁₋₆)alkyl, or R^{d} and R^{e} together with the intervening nitrogen atom represent a pyrrolidino, piperidino or morpholino group; Y represents oxygen or sulphur; R^{f} represents C₁₋₆ alkyl, aryl or aryl(C₁₋₆)alkyl; and R^{h} represents hydrogen or C₁₋₆ alkyl. Thus, suitable in vivo hydrolysable ester and amido residues include, for example, acyloxyalkyl groups such as acetoxymethyl, pivaloyloxymethyl, α-acetoxyethyl and α-pivaloyloxyethyl groups; alkoxycarbonyloxyalkyl groups such as ethoxycarbonyloxymethyl and α-ethoxycarbonyloxyethyl; dialkylaminoalkyl especially di-loweralkylaminoalkyl groups such as dimethylaminomethyl, dimethylaminoethyl, dimethylaminopropyl, diethylaminomethyl or diethylaminoethyl; and heterocyclylalkyl groups such as pyrrolidinylethyl or morpholinoethyl.

The benzo moiety of the tetrahydroquinoline ring system shown in formula IIC above and IID below may be unsubstituted, or substituted by one or more substituents selected from halogen, cyano, trifluoromethyl, nitro, hydroxy, amino, carboxy, C₁₋₆ alkyl, C₁₋₆ alkoxy, C₁₋₆ alkylthio and C₁₋₆ alkoxycarbonyl. Suitably, R⁸ represents hydrogen and R⁵, R⁶ and R⁷ independently represent hydrogen, halogen or C₁₋₆ alkyl. Preferably, R⁶ and R⁸ each represents hydrogen and R⁵ and R⁷ independently represent methyl, ethyl or halogen, in particular chloro, bromo or iodo.

The compounds according to the invention have a number of asymmetric centres, and may accordingly exist both as enantiomers and as diastereoisomers. It is to be understood that all such isomers and mixtures thereof are encompassed within the scope of the present invention.

Preferably, in relation to formula IIC above,
R^{a} is hydrogen and R^{b} represents phenyl or benzyl, optionally substituted by an aminomethyl or aminoethyl group.

The present invention also provides compounds of formula IID and salts thereof: 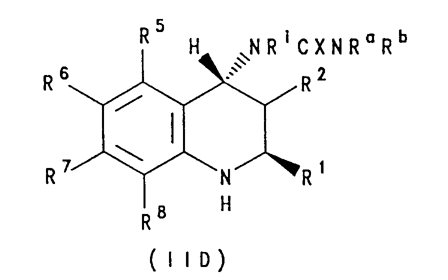 wherein R¹, R², R⁵, R⁶, R⁷ and R⁸ are as defined above;
R^{a} and Rⁱ independently represent hydrogen, C₁₋₆ alkyl or aryl selected from phenyl, naphthyl and fluorenyl;
R^{b} represents C₃₋₇ cycloalkyl, aryl selected from phenyl, naphthyl and fluorenyl, or aryl(C₁₋₆)alkyl, any of which groups may be optionally substituted by one or more groups selected from methyl, ethyl, chloro, iodo, methoxy and nitro; and X represents oxygen or sulphur.

Suitably, R^{a} and Rⁱ independently represent hydrogen, methyl, ethyl or phenyl; and R^{b} represents cyclohexyl, phenyl, naphthyl or benzyl. Preferably, R^{a} and Rⁱ both represent hydrogen; and X represents oxygen.

Specific compounds within the scope of the present invention include:
4-benzoylamino-2-carboxy-5,7-dichloro-1,2,3,4-tetrahydroquinoline;
4-acetylamino-2-carboxy-5,7-dichloro-1,2,3,4-tetrahydroquinoline;
2-carboxy-5,7-dichloro-4-cyclohexylcarbonylamino-1,2,3,4-tetrahydroquinoline;
4-benzylcarbonylamino-2-carboxy-5,7-dichloro-1,2,3,4-tetrahydroquinoline;
2-carboxy-4-(4-chlorophenylcarbonylamino)-5,7-dichloro-1,2,3,4-tetrahydroquinoline;
2-carboxy-5,7-dichloro-4-(4-pyridylcarbonylamino)-1,2,3,4-tetrahydroquinoline;
2-carboxy-5,7-dichloro-4-[2-(2-aminophenethyl)]carbonylamino-1,2,3,4-tetrahydroquinoline;
2-carboxy-5,7-dichloro-4-n-propylcarbonylamino-1,2,3,4-tetrahydroquinoline;
2-carboxy-5,7-dichloro-4-phenylaminocarbonylamino-1,2,3,4-tetrahydroquinoline;
2-carboxy-5,7-dichloro-4-(4-methoxyphenylcarbonylamino)-1,2,3,4-tetrahydroquinoline;
4-benzylaminocarbonylamino-2-carboxy-5,7-dichloro-1,2,3,4-tetrahydroquinoline;
2-carboxy-5,7-dichloro-4-(2-methoxybenzylcarbonylamino)-1,2,3,4-tetrahydroquinoline;
2-carboxy-5,7-dichloro-4-(2-methylbenzylcarbonylamino)-1,2,3,4-tetrahydroquinoline;
2-carboxy-5,7-dichloro-4-(α-methoxybenzylcarbonylamino)-1,2,3,4-tetrahydroquinoline;
2-carboxy-5,7-dichloro-4-(2-nitrobenzylcarbonylamino)-1,2,3,4-tetrahydroquinoline;
2-carboxy-5,7-dichloro-4-(2-nitrophenylaminocarbonylamino)-1,2,3,4-tetrahydroquinoline;
2-carboxy-5,7-dichloro-4-(2-methoxyphenylaminocarbonylamino)-1,2,3,4-tetrahydroquinoline;
2-carboxy-5,7-dichloro-4-(2-methylphenylaminocarbonylamino)-1,2,3,4-tetrahydroquinoline;
2-carboxy-4-(2-chlorophenylaminocarbonylamino)-5,7-dichloro-1,2,3,4-tetrahydroquinoline;
4-(4'-biphenylcarbonylamino)-2-carboxy-5,7-dichloro-1,2,3,4-tetrahydroquinoline;
2-carboxy-5,7-dichloro-4-isopropylcarbonylamino-1,2,3,4-tetrahydroquinoline;
2-carboxy-4-(2-chlorophenylcarbonylamino)-5,7-dichloro-1,2,3,4-tetrahydroquinoline;
2-carboxy-5,7-dichloro-4-(1-naphthylcarbonylamino)-1,2,3,4-tetrahydroquinoline;
2-carboxy-5,7-dichloro-4-(2-naphthylcarbonylamino)-1,2,3,4-tetrahydroquinoline;
2-carboxy-5,7-dichloro-4-(2-furylcarbonylamino)-1,2,3,4-tetrahydroquinoline;
2-carboxy-5,7-dichloro-4-(2-methylphenylcarbonylamino)-1,2,3,4-tetrahydroquinoline;
2-carboxy-5,7-dichloro-4-(2-phenethylcarbonylamino)-1,2,3,4-tetrahydroquinoline;
2-carboxy-5,7-dichloro-4-(2-phenylethenylcarbonylamino)-1,2,3,4-tetrahydroquinoline;
2-carboxy-5,7-dichloro-4-(2-thienylmethylcarbonylamino)-1,2,3,4-tetrahydroquinoline;
2-carboxy-4-(3-chlorophenylcarbonylamino)-5,7-dichloro-1,2,3,4-tetrahydroquinoline;
2-carboxy-5,7-dichloro-4-(3-phenylpropylcarbonylamino)-1,2,3,4-tetrahydroquinoline;
2-carboxy-5,7-dichloro-4-(9-fluorenylcarbonylamino)-1,2,3,4-tetrahydroquinoline;
2-carboxy-4-cyclohexylmethylcarbonylamino-5,7-dichloro-1,2,3,4-tetrahydroquinoline;
2-carboxy-4-(2-chlorobenzylcarbonylamino)-5,7-dichloro-1,2,3,4-tetrahydroquinoline;
2-carboxy-4-(3-chlorobenzylcarbonylamino)-5,7-dichloro-1,2,3,4-tetrahydroquinoline;
2-carboxy-4-(4-chlorobenzylcarbonylamino)-5,7-dichloro-1,2,3,4-tetrahydroquinoline;
2-carboxy-5,7-dichloro-4-(4-methylbenzylcarbonylamino)-1,2,3,4-tetrahydroquinoline;
2-carboxy-5,7-dichloro-4-(4-methoxybenzylcarbonylamino)-1,2,3,4-tetrahydroquinoline;
2-carboxy-5,7-dichloro-4-(4-nitrobenzylcarbonylamino)-1,2,3,4-tetrahydroquinoline;
2-carboxy-4-(3-cyanophenylcarbonylamino)-5,7-dichloro-1,2,3,4-tetrahydroquinoline;
2-carboxy-4-(4-chlorophenylaminocarbonylamino)-5,7-dichloro-1,2,3,4-tetrahydroquinoline;
2-carboxy-5,7-dichloro-4-(4-methylphenylaminocarbonylamino)-1,2,3,4-tetrahydroquinoline;
2-carboxy-5,7-dichloro-4-(4-methoxyphenylaminocarbonylamino)-1,2,3,4-tetrahydroquinoline;
2-carboxy-5,7-dichloro-4-(4-nitrophenylaminocarbonylamino)-1,2,3,4-tetrahydroquinoline;
2-carboxy-5,7-dichloro-4-(4-iodophenylaminocarbonylamino)-1,2,3,4-tetrahydroquinoline;
2-carboxy-5,7-dichloro-4-[phenylaminocarbonyl(N-methyl)amino]-1,2,3,4-tetrahydroquinoline,
2-carboxy-5,7-dichloro-4-[(N-methyl-N-phenyl)aminocarbonylamino]-1,2,3,4-tetrahydroquinoline;
2-carboxy-5,7-dichloro-4-(2,3-dihydroindol-1-ylcarbonylamino)-1,2,3,4-tetrahydroquinoline;
2-carboxy-4-[2-(carboxyethyl)carbonylamino]-5,7-dichloro-1,2,3,4-tetrahydroquinoline;
4-[3-(aminomethyl)phenylcarbonylamino]-2-carboxy-5,7-dichloro-1,2,3,4-tetrahydroquinoline;
4-[3-(aminomethyl)phenylcarbonylamino]-5,7-dichloro-2-methoxycarbonyl-1,2,3,4-tetrahydroquinoline;
4-[4-(aminomethyl)phenylcarbonylamino]-2-carboxy-5,7-dichloro-1,2,3,4-tetrahydroquinoline;
4-[4-(aminomethyl)phenylcarbonylamino]-5,7-dichloro-2-methoxycarbonyl-1,2,3,4-tetrahydroquinoline;
4-[4-(2-aminoethyl)phenylcarbonylamino]-2-carboxy-5,7-dichloro-1,2,3,4-tetrahydroquinoline;
4-[4-(2-aminoethyl)phenylcarbonylamino]-5,7-dichloro-2-methoxycarbonyl-1,2,3,4-tetrahydroquinoline;
2-carboxy-5,7-dichloro-4-(3-methylbenzylcarbonylamino)-1,2,3,4-tetrahydroquinoline;
2-carboxy-5,7-dichloro-4-(3-nitrobenzylcarbonylamino)-1,2,3,4-tetrahydroquinoline;
2-carboxy-5,7-dichloro-4-(3-methoxybenzylcarbonylamino)-1,2,3,4-tetrahydroquinoline;
2-carboxy-5,7-dichloro-4-(1-naphthylmethylcarbonylamino)-1,2,3,4-tetrahydroquinoline;
2-carboxy-5,7-dichloro-4-(2-naphthylmethylcarbonylamino)-1,2,3,4-tetrahydroquinoline;
2-carboxy-5,7-dichloro-4-(3-thienylmethylcarbonylamino)-1,2,3,4-tetrahydroquinoline;
2-carboxy-5,7-dichloro-4-(2,6-dichlorobenzylcarbonylamino)-1,2,3,4-tetrahydroquinoline;
2-carboxy-5,7-dichloro-4-phenylaminothiocarbonylamino-1,2,3,4-tetrahydroquinoline;
2-carboxy-5,7-dichloro-4-(3-methoxyphenylaminocarbonylamino)-1,2,3,4-tetrahydroquinoline;
2-carboxy-5,7-dichloro-4-(3-methylphenylaminocarbonylamino)-1,2,3,4-tetrahydroquinoline;
2-carboxy-4-(3-chlorophenylaminocarbonylamino)-5,7-dichloro-1,2,3,4-tetrahydroquinoline;
2-carboxy-5,7-dichloro-4-(3-nitrophenylaminocarbonylamino)-1,2,3,4-tetrahydroquinoline;
4-(3-aminopropyl)carbonylamino-2-carboxy-5,7-dichloro-1,2,3,4-tetrahydroquinoline;
4-(2-aminoethyl)carbonylamino-2-carboxy-5,7-dichloro-1,2,3,4-tetrahydroquinoline;
4-(4-aminobutyl)carbonylamino-2-carboxy-5,7-dichloro-1,2,3,4-tetrahydroquinoline;
2-carboxy-5,7-dichloro-4-(4-piperidylmethylcarbonylamino)-1,2,3,4-tetrahydroquinoline;
4-[4-(aminomethyl)benzylcarbonylamino]-2-carboxy-5,7-dichloro-1,2,3,4-tetrahydroquinoline;
4-[4-(aminomethyl)benzylcarbonylamino]-5,7-dichloro-2-methoxycarbonyl-1,2,3,4-tetrahydroquinoline;
4-[4-(2-aminoethyl)benzylcarbonylamino]-2-carboxy-5,7-dichloro-1,2,3,4-tetrahydroquinoline;
4-[4-(2-aminoethyl)benzylcarbonylamino]-5,7-dichloro-2-methoxycarbonyl-1,2,3,4-tetrahydroquinoline;
2-carboxy-5,7-dichloro-4-[4-(N-methylaminomethyl)benzylcarbonylamino]-1,2,3,4-tetrahydroquinoline;
5,7-dichloro-2-methoxycarbonyl-4-[4-(N-methylaminomethyl)benzylcarbonylamino]-1,2,3,4-tetrahydroquinoline;
5,7-dichloro-4-[4-(N,N-dimethylaminomethyl)benzylcarbonylamino]-2-methoxycarbonyl-1,2,3,4-tetrahydroquinoline;
2-carboxy-5,7-dichloro-4-[4-(N,N-dimethylaminomethyl)benzylcarbonylamino]-1,2,3,4-tetrahydroquinoline;
4-[4-(3-aminoprop-2-ynyl)benzylcarbonylamino]-2-carboxy-5,7-dichloro-1,2,3,4-tetrahydroquinoline;
4-[4-(3-aminoprop-2-ynyl)benzylcarbonylamino]-5,7-dichloro-2-methoxycarbonyl-1,2,3,4-tetrahydroquinoline;
4-[4-(3-aminopropyl)benzylcarbonylamino]-2-carboxy-5,7-dichloro-1,2,3,4-tetrahydroquinoline;
4-[4-(3-aminopropyl)benzylcarbonylamino]-5,7-dichloro-2-methoxycarbonyl-1,2,3,4-tetrahydroquinoline;
4-[4-(4-aminobut-2-ynyl)benzylcarbonylamino]-2-carboxy-5,7-dichloro-1,2,3,4-tetrahydroquinoline;
4-[4-(4-aminobut-2-ynyl)benzylcarbonylamino)-5,7-dichloro-2-methoxycarbonyl-1,2,3,4-tetrahydroquinoline;
4-[4-(4-aminobutyl)benzylcarbonylamino]-2-carboxy-5,7-dichloro-1,2,3,4-tetrahydroquinoline;
4-[4-(4-aminobutyl)benzylcarbonylamino]-5,7-dichloro-2-methoxycarbonyl-1,2,3,4-tetrahydroquinoline;
4-[3-(aminomethyl)benzylcarbonylamino]-2-carboxy-5,7-dichloro-1,2,3,4-tetrahydroquinoline;
4-[3-(aminomethyl)benzylcarbonylamino]-5,7-dichloro-2-methoxycarbonyl-1,2,3,4-tetrahydroquinoline;
4-[2-(aminomethyl)benzylcarbonylamino]-2-carboxy-5,7-dichloro-1,2,3,4-tetrahydroquinoline;
4-[2-(aminomethyl)benzylcarbonylamino]-5,7-dichloro-2-methoxycarbonyl-1,2,3,4-tetrahydroquinoline;
4-[2-(4-(aminomethyl)phenyl)ethylcarbonylamino]-2-carboxy-5,7-dichloro-1,2,3,4-tetrahydroquinoline;
4-[2-(4-(aminomethyl)phenyl)ethylcarbonylamino]-5,7-dichloro-2-methoxycarbonyl-1,2,3,4-tetrahydroquinoline;
4-(4-aminobenzylcarbonylamino)-2-carboxy-5,7-dichloro-1,2,3,4-tetrahydroquinoline;
2-carboxy-4-(2-carboxyphenylcarbonylamino)-5,7-dichloro-1,2,3,4-tetrahydroquinoline;
2-carboxy-4-(4-chloro-3-nitrobenzylcarbonylamino)-5,7-dichloro-1,2,3,4-tetrahydroquinoline;
2-carboxy-5,7-dichloro-4-(4-hydroxy-3-nitrobenzylcarbonylamino)-1,2,3,4-tetrahydroquinoline;
2-carboxy-5,7-dichloro-4-(diphenylmethylcarbonylamino)-1,2,3,4-tetrahydroquinoline;
2-carboxy-5,7-dichloro-4-(1-phenylethyl)carbonylamino-1,2,3,4-tetrahydroquinoline;
4-(4-acetylbenzylcarbonylamino)-2-carboxy-5,7-dichloro-1,2,3,4-tetrahydroquinoline;
2-carboxy-5,7-dichloro-4-phenoxymethylcarbonylamino-1,2,3,4-tetrahydroquinoline;
2-carboxy-5,7-dichloro-4-(4-ethylbenzylcarbonylamino)-1,2,3,4-tetrahydroquinoline;
2-carboxy-5,7-dichloro-4-(4-hydroxybenzylcarbonylamino)-1,2,3,4-tetrahydroquinoline;
4-(4-acetamidobenzylcarbonylamino)-2-carboxy-5,7-dichloro-1,2,3,4-tetrahydroquinoline;
2-carboxy-5,7-dichloro-4-(1-naphthylaminocarbonylamino)-1,2,3,4-tetrahydroquinoline;
2-carboxy-4-cyclohexylaminocarbonylamino-5,7-dichloro-1,2,3,4-tetrahydroquinoline;
2-carboxy-5,7-dichloro-4-[N-methyl-N-(4-methylphenyl)amino]carbonylamino-1,2,3,4-tetrahydroquinoline;
2-carboxy-5,7-dichloro-4-(N,N-diphenylamino)carbonylamino-1,2,3,4-tetrahydroquinoline;
2-carboxy-5,7-dichloro-4-(4-ethylphenyl)aminocarbonylamino-1,2,3,4-tetrahydroquinoline;
2-carboxy-5,7-dichloro-4-(N-ethyl-N-phenyl)aminocarbonylamino-1,2,3,4-tetrahydroquinoline;
4-benzylcarbonylamino-2-(t-butylcarbonyloxy)methoxycarbonyl-5,7-dichloro-1,2,3,4-tetrahydroquinoline;
4-benzylcarbonylamino-5,7-dichloro-2-(methylaminocarbonyl)methoxycarbonyl-1,2,3,4-tetrahydroquinoline;
4-benzylcarbonylamino-5,7-dichloro-2-[2-(N,N-dimethylamino)ethylaminocarbonyl]methoxycarbonyl-1,2,3,4-tetrahydroquinoline;
4-benzylcarbonylamino-5,7-dichloro-2-[2-(N,N-dimethylamino)ethoxycarbonyl]-1,2,3,4-tetrahydroquinoline;
4-benzylcarbonylamino-5,7-dichloro-2-[3-(N,N-dimethylamino)propoxycarbonyl]-1,2,3,4-tetrahydroquinoline;
4-benzylcarbonylamino-5,7-dichloro-2-[2-(N,N-dimethylamino)ethylaminocarbonyl]-1,2,3,4-tetrahydroquinoline;
5,7-dichloro-2-[2-(N,N-dimethylamino)ethylaminocarbonyl]-4-phenylaminocarbonylamino-1,2,3,4-tetrahydroquinoline;
4-[4-(aminomethyl)benzylcarbonylamino]-5,7-dichloro-2-(methylaminocarbonyl)methoxycarbonyl-1,2,3,4-tetrahydroquinoline;
4-[4-(aminomethyl)benzylcarbonylamino]-5,7-dichloro-2-hexyloxycarbonyl-1,2,3,4-tetrahydroquinoline;
and salts thereof; wherein the relative orientation of the substituents at the 2- and 4-positions of the tetrahydroquinoline ring system is trans.

The invention also provides pharmaceutical compositions comprising one or more compounds of this invention in association with a pharmaceutically acceptable carrier. Preferably these compositions are in unit dosage forms such as tablets, pills, capsules, powders, granules, sterile parenteral solutions or suspensions, or suppositories, for oral, parenteral or rectal administration. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatin.

In the treatment of neurodegeneration, a suitable dosage level is about 0.01 to 250 mg/kg per day, preferably about 0.05 to 100 mg/kg per day, and especially about 0.05 to 5 mg/kg per day. The compounds may be administered on a regimen of 1 to 4 times per day.

The compounds according to the invention may be prepared by a process which comprises reducing a quinoline derivative of formula IV: 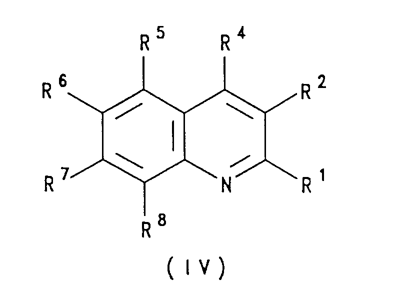 wherein R¹, R², R⁵, R⁶, R⁷ and R⁸ are as defined above, and R⁴ represents -NR^{a}COR^{b} or -NRⁱCXNR^{a}R^{b} as defined above.

A suitable reducing agent for carrying out the above transformation is sodium cyanoborohydride. The reaction is conveniently carried out in the presence of a suitable solvent, for example acetic acid, at an elevated temperature, e.g. a temperature in the region of 50°C.

The compounds of formula IIC may be prepared by reacting a compound of formula A-COR^{b} with a compound of formula V: 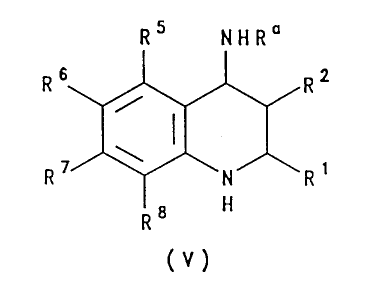 wherein the relative orientation of the R¹ and -NHR^{a} substituents is trans;
R¹, R², R⁵, R⁶, R⁷, R⁸, R^{a} and R^{b} are as defined above; and A represents a leaving group. In this context, A suitably represents halogen, C₁₋₆ alkoxy or -OCOR^{b}.

The reaction is conveniently carried out by treating the compound of formula V with the appropriate acid chloride in the presence of triethylamine in an inert solvent, e.g. dichloromethane. Alternatively, the reaction may be effected by treating the compound of formula V with the appropriate carboxylic acid in the presence of a suitable condensing agent, e.g. 1,3-dicyclohexylcarbodiimide. Further methods for carrying out this transformation include standard amide bond-forming reactions used in peptide synthesis.

The compounds of formula V above wherein R^{a} is hydrogen may be prepared by reducing a compound of formula VI: 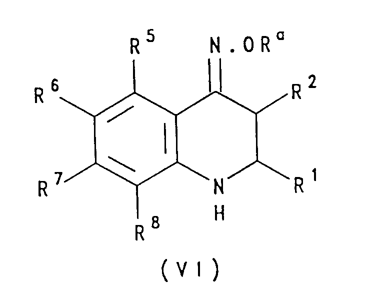 wherein R¹, R², R⁵, R⁶, R⁷, R⁸ and R^{a} are as defined above.

A suitable reducing agent for this purpose is zinc in acetic acid, and the reaction is conveniently carried out at room temperature.

The compounds of formula VI above may be prepared by treating a compound of formula VII: 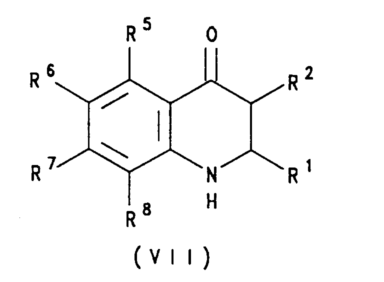 wherein R¹, R², R⁵, R⁶, R⁷ and R⁸ are as defined above; with a hydroxylamine derivative of formula H₂N.OR^{a} wherein R^{a} is as defined above, or a salt thereof, particularly the hydrochloride salt.

The reaction is conveniently carried out in a suitable solvent, e.g. methanol, at reflux temperature, in the presence of a stoichiometric quantity of pyridine.

In an alternative procedure, the compounds of formula V may be prepared directly from the compounds of formula VII by reductive amination. This process comprises treating a compound of formula VII as defined above with an amine of formula R^{a}NH₂, in which R^{a} is as defined above, in the presence of a reducing agent. Suitable reducing agents include sodium borohydride, sodium cyanoborohydride, and hydrogen in the presence of a catalyst such as palladium on charcoal, or platinum oxide.

The compounds of formula VII above may be prepared by a process which comprises cyclising a reactive derivative of a carboxylic acid of formula VIII: 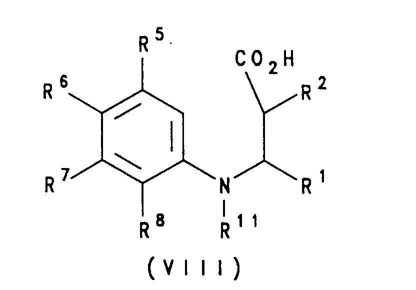 wherein R¹, R², R⁵, R⁶, R⁷ and R⁸ are as defined above and R¹¹ represents an amino-protecting group; and subsequently removing the amino-protecting group R¹¹.

The reactive derivatives of the carboxylic acid VIII suitably include esters, for example C₁₋₄ alkyl esters; acid anhydrides, for example mixed anhydrides with C₁₋₄ alkanoic acids; acid halides, for example acid chlorides; orthoesters; and primary, secondary and tertiary amides.

Suitable examples of amino-protecting groups for the substituent R¹¹ include carboxylic acid groups such as acetyl, chloroacetyl, trifluoroacetyl, formyl, benzoyl, phthaloyl, phenylacetyl or pyridinecarbonyl; acid groups derived from carbonic acid such as ethoxycarbonyl, benzyloxycarbonyl, t-butoxycarbonyl, biphenylisopropoxycarbonyl, p-methylbenzyloxycarbonyl, p-nitrobenzyloxycarbonyl, p-bromobenzyloxycarbonyl, p-phenylazobenzyloxycarbonyl, p-(p'-methoxyphenylazo)benzyloxycarbonyl or t-amyloxycarbonyl; acid groups derived from sulphonic acid, e.g. p-toluenesulphonic acid; and other groups such as benzyl, trityl, o-nitrophenylsulphenyl or benzylidene.

A preferred amino-protecting group is acetyl.

The removal of the amino-protecting group present in the resultant compound may be effected by an appropriate procedure depending upon the nature of the protecting group. A typical procedure for the acetyl group involves heating at reflux temperature in the presence of 3N hydrochloric acid.

When R¹ in the compounds according to the invention represents carboxy, it is convenient to replace the carboxylic acid VIII in the above reaction with a cyclic anhydride of formula IX: 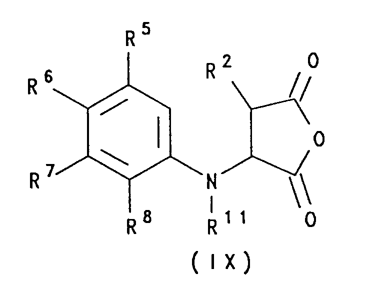 wherein R², R⁵, R⁶, R⁷, R⁸ and R¹¹ are as defined above.

The reaction is suitably carried out in the presence of a Lewis acid catalyst such as aluminium chloride; and is conveniently effected at an elevated temperature, for example a temperature in the region of 160°C, suitably in the melt.

In an alternative process, the compounds according to the invention may be prepared by reacting a compound of formula X, or a protected derivative thereof, with a compound of formula XI, or a protected derivative thereof: 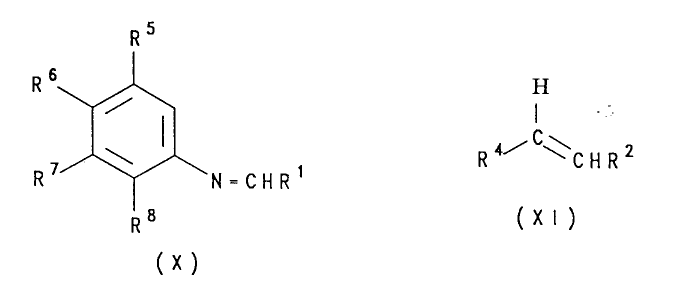 wherein R¹, R² and R⁴ to R⁸ are as defined above; followed, where necessary, by removal of the protecting groups.

The reaction is conveniently carried out at room temperature in an inert solvent, e.g. dichloromethane, in the presence of a Lewis acid catalyst such as boron trifluoride etherate. Illustrative experimental details for this reaction are described in J. Heterocycl. Chem., 1988, 25, 1831.

As will be appreciated, any compound of formula I initially obtained may, where appropriate, subsequently be elaborated into a further compound of formula I by techniques known from the art.

The intermediates of formulae IV, VIII and IX may conveniently be prepared by methods analogous to those described in the accompanying Examples.

Except where explicitly stated otherwise, the above-described processes for the preparation of the compounds according to the invention are likely to give rise to mixtures of stereoisomers. At an appropriate stage, these isomers may be separated by conventional techniques such as preparative chromatography.

The novel compounds may be prepared in racemic form, or individual enantiomers may be prepared either by enantiospecific synthesis or by resolution. The novel compounds may, for example, be resolved into their component enantiomers by standard techniques, such as the formation of diastereomeric pairs by salt formation with an optically active acid, such as (-)-di-p-toluoyl-d-tartaric acid and/or (+)-di-p-toluoyl-l-tartaric acid followed by fractional crystallization and regeneration of the free base. The novel compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene, Protective Groups in Organic Synthesis, John Wiley & Sons, 1981. The protecting groups may be removed at a convenient subsequent stage using methods known from the art.

The compounds useful in this invention potently and selectively block responses to NMDA in a brain slice from rat cortex, and inhibit glycine binding to the strychnine-insensitive site present on the NMDA receptor.

### Cortical Slice Studies

The effects of compounds of the invention on responses to NMDA were assessed using the rat cortical slice as described by Wong et al., Proc. Natl. Acad. Sci. USA, 1986, 83, 7104. The apparent equilibrium constant (K_{b}) was calculated from the righthand shift in the NMDA concentration-response curve produced by the compound under test. The compounds of the accompanying Examples were tested and each was found to possess a K_{b} value below 100 µM.

### Inhibition of Glycine Binding

The ability of test compounds to displace ³H-glycine binding to the strychnine-insensitive site present on the NMDA receptor of rat forebrain membranes was determined by the method of Donald et al., Proceedings of The British Pharmacological Society, University of Nottingham, September 1988, Abstract P122. The concentration of the compounds of each of the accompanying Examples required to displace 50% of the specific binding (IC₅₀) is below 100 µM.

### PREPARATIVE EXAMPLE A

### 2-Carboxy-5,7-dichloro-4-oxo-1,2,3,4-tetrahydroquinoline

### a) Methyl 2-(3,5-dichlorophenylamino)-3-methoxycarbonylpropionate

3,5-Dichloroaniline (104g) and dimethylacetylene dicarboxylate (79ml) were dissolved in dry methanol (100ml) and heated under reflux for 14h. On cooling a yellow solid crystallised out and this was collected by filtration. The mother liquors were concentrated in vacuo to leave a residue from which a second crop of product was obtained by recrystallisation from diethyl ether/60-80 petrol. The combined crops of material weighed 167.5g (after drying at 40°C under a pressure of 20mmHg for 18h). A portion of this material (105g) was dissolved in ethyl acetate (1000ml) and hydrogenated at 50 psi over 10% palladium on carbon catalyst (4g) for 40h. The catalyst was removed by filtration and the solvent evaporated under vacuum to leave a residue which was recrystallised from diethyl ether/hexane to give the title compound as a white crystalline solid (84.8g, m.p. 66-67°C).

### b) 3-Carboxy-2-(3,5-dichlorophenylamino)-propionic acid

Methyl 2-(3,5-Dichlorophenyl)amino-3-methoxycarboxyl-propionate (131.4g) was dissolved in methanol (800ml) and sodium hydroxide (51.5g in 600ml of water) was added. The reaction mixture was stirred at room temperature for 5h then the methanol was removed under vacuum and the residual solution was acidified to pH l by the cautious addition of concentrated hydrochloric acid. The aqueous solution was extracted with ethyl acetate (2 x 600ml) and the combined organic layers were dried (MgSO₄), filtered and concentrated in vacuo to give the required compound as a white solid (11.6g, m.p. 217°C dec).

### c) 2-[N-Acetyl-N-(3,5-dichlorophenyl)amino]-3-carboxy-propionic acid

3-Carboxy-2-(3,5-dichlorophenylamino)-propionic acid (116g) was suspended in acetic anhydride (1000ml) and heated at 60-70°C under nitrogen for 6h then cooled and concentrated in vacuo. The residue which was obtained was dissolved in methanol (500ml) and sodium hydroxide solution (500ml of 2.5N) was added. The reaction mixture was stirred at room temperature for 14h then the methanol was removed by evaporation. The residual aqueous solution was washed with diethyl ether (2 x 200ml) the acidified to pH l with concentrated hydrochloric acid and extracted into ethyl acetate (2 x 500ml), dried (MgSO₄), filtered and concentrated under vacuum. The residue obtained was triturated with diethyl ether and collected by filtration to give the the title compound as a white solid (128.5g, m.p. 181°C dec).

### d) 1-Acetyl-2-carboxy-5,7-dichloro-4-oxo-1,2,3,4-tetrahydroquinoline

2-[N-Acetyl-N-(3,5-dichlorophenyl)amino]-3-carboxy-propionic acid (128g) was suspended in dichloromethane (1000ml) and acetyl chloride (300ml) was added. The reaction mixture was stirred overnight at room temperature under an inert atmosphere, filtered, then concentrated in vacuo and then dried under high vacuum to give as an off white solid the required substituted succinic anhydride (119.8g). A portion of this material (60g) and finely ground aluminium trichloride (168g) were blended together and heated at 170°C for 30 min. After this time the reaction mixture was allowed to cool and ice cold 1N hydrochloric acid was added cautiously until effervescence ceased. The aqueous layer was decanted and the residual solid retained and combined with the product from a second reaction carried out on the remaining anhydride (50.8g). The solid was triturated with absolute ethanol and the title compound was collected by filtration (20.2g, m.p. 212-213°C).

### e) 2-Carboxy-5,7-dichloro-4-oxo-1,2,3,4-tetrahydroquinoline

1-Acetyl-2-carboxy-5,7-dichloro-4-oxo-1,2,3,4-tetrahydroquinoline (21.17g) was suspended in 3N hydrochloric acid (500ml) and heated at reflux overnight. After cooling, the aqueous solution was extracted with ethyl acetate (3 x 300ml) and the combined organic layers were washed with brine (1 x 300ml), dried (MgSO₄), filtered and evaporated in vacuo to give the title compound as a yellow solid (17.45g, m.p. 218-219°C).

### PREPARATIVE EXAMPLE B

### Trans-4-tertiary-butyloxycarbonylamino-2-carboxy-5,7-dichloro-1,2,3,4-tetrahydroquinoline

### a) 5,7-Dichloro-4-hydroxyimino-2-methoxycarbonyl-1,2,3,4-tetrahydroquinoline

To a solution of 2-carboxy-5,7-dichloro-4-oxo-1,2,3,4-tetrahydroquinoline (Preparative Example A) (14.85g, 57.1mmol) in methanol (300ml) was added hydroxylamine hydrochloride (4.17g, 60.0mmol) followed by dry pyridine (4.85ml, 60.0mmol) and the resulting mixture was heated at reflux under an atmosphere of nitrogen for 2 hours. The mixture was then cooled, and a solution of methanol saturated with hydrogen chloride (50ml) was added and the reaction was stirred at room temperature under an atmosphere of nitrogen for 17 hours. The solvent was removed in vacuo and the residue was partitioned between water (300ml) and diethyl ether (300ml). The aqueous phase was further extracted with diethyl ether (2 x 300ml) and the combined organic layers were washed with 0.5M aqueous citric acid solution (1 x 200ml), saturated hydrogen sodium bicarbonate solution (2 x 200ml), brine (1 x 200ml), then dried (MgSO₄) and the solvent was removed under vacuum to give the title compound (15.1g) as a brown solid (m.p. 216-217°C).

### b) Cis and trans-4-tertiary-butyloxcarbonylamino-5,7- dichloro-2-methoxycarbonyl-1,2,3,4-tetrahydroquinoline

To a suspension of 5,7-dichloro-4-hydroxyimino-2-methoxycarbonyl-1,2,3,4-tetrahydroquinoline (8.0g, 27.7mmol) in glacial acetic acid (240ml) was added zinc dust (12.0g, 18mmol) and the resulting mixture was heated at 60-65°C, under an atmosphere of nitrogen, with stirring for 4 hours. The reaction mixture was allowed to cool, then filtered and the filtrate was evaporated in vacuo. The residue was redissolved in ethyl acetate (400ml), washed with saturated sodium bicarbonate solution (2 x 200ml) and saturated brine solution (1 x 200ml) then dried (MgSO₄), filtered and concentrated under vacuum to give a brown foam (6.60g). To a solution of this foam in dichloromethane (350ml) was added di-tertiary-butyl-dicarbonate (12.0g, 55mmol) and the mixture was stirred at room temperature for 65 hours under an atmosphere of nitrogen. To the reaction mixture was added N,N-dimethylethylenediamine (6.6ml, 60mmol) and stirring was continued for a further 2 hours. The reaction mixture was washed successively with 0.5M citric acid solution (2 x 200ml) and brine (1 x 200ml), dried (MgSO₄), filtered and the solvent was removed under vacuum to give an oily solid (5.8g). This was purified by flash chromatography using 20% ethyl acetate in petroleum ether (bp 60-80°C) as eluent to give a mixture of cis and trans isomers. Trituration of this mixture with diethyl ether gave a solid which was collected by filtration and was recrystallised from hot ethyl acetate/petroleum ether (bp 60-80°C) to give, as colourless needles, the less polar Trans-4-tertiarybutyloxycarbonylamino-5,7-dichloro-2-methoxycarbonyl-1,2,3,4-tetrahydroquinoline (1.33g, m.p. 210-211°C).

### c) Trans-4-tertiary-butyloxycarbonylamino-2-carboxy-5,7-dichloro-1,2,3,4-tetrahydroquinoline

To a solution of trans-4-tertiary-butyloxycarbonylamino-5,7-dichloro-2-methoxycarbonyl-1,2,3,4-tetrahydroquinoline (0.153g, 0.408mmol) in tetrahydrofuran (10ml) was added aqueous lithium hydroxide (0.45ml of a 1.0M solution, 0.45mmol) followed by distilled water (3ml) and the resulting mixture was stirred at room temperature for 4 hours. The mixture was then evaporated to dryness under vacuum and the residue was redissolved in water (40ml). The solution was adjusted to pH 1 with dilute hydrochloric acid and the mixture was extracted with ethyl acetate (2 x 30ml). The combined organic extracts were washed with brine then dried (MgSO₄), and concentrated in vacuo to give an oil from which two crops of crude product were obtained by recrystallisation from diethyl ether/petroleum ether (bp 60-80°C). The combined material was dissolved in methanol and evaporated under vacuum to give an oil which was recrystallised from diethyl ether to give the pure title compound as colourless crystals (0.071g, m.p. 193-195°C).

### PREPARATIVE EXAMPLE C

### Trans-4-amino-2-carboxy-5,7-dichloro-1,2,3,4-tetrahydroquinoline hydrochloride

To a suspension of trans-4-tertiary-butyloxy-carbonylamino-2-carboxy-5,7-dichloro-1,2,3,4-tetrahydroquinoline (80mg, 0.222mmol) in ethyl acetate (3ml) was added hydrogen chloride in ethyl acetate (3ml of an approximately 5M solution) and the resulting mixture was stirred at room temperature for 5 hours. The solvent was removed under vacuum and the residue was triturated with ethyl acetate. The solid obtained was collected by filtration then washed with ethyl acetate and dried to give as a colourless solid, the title compound (0.054g, m.p. 184-188°C).

### EXAMPLE 1

### Trans-2-carboxy-5,7-dichloro-4-benzoylamino-1,2,3,4-tetrahydroquinoline

### a) Trans-4-amino-5,7-dichloro-2-methoxycarbonyl-1,2,3,4-tetrahydroquinoline hydrochloride

To a suspension of trans-4-tertiary-butyloxy-carbonylamino-5,7-dichloro-2-methoxycarbonyl-1,2,3,4-tetrahydroquinoline (1.2g) in ethyl acetate (25ml) was added a solution of hydrogen chloride in ethyl acetate (25M of an approx 5M solution) and the resulting mixture was stirred at room temperature for 3 hours after which time the solvent was removed in vacuo. The residue was triturated with ethyl acetate and the solid was collected to give the title compound as a colourless solid (0.96g, m.p. 192-194°C [sublimes]).

### b) Trans-5,7-dichloro-2-methoxycarbonyl-4-benzoylamino-1,2,3,4-tetrahydroquinoline

To a suspension of trans-4-amino-5,7-dichloro-2-methoxycarbonyl-1,2,3,4-tetrahydroquinoline hydrochloride (0.2g, 0.642mmol) in anhydrous dichloromethane (15ml) was added dry triethylamine (220ml, 1.4mmol) and the resulting mixture was stirred at room temperature under an atmosphere of nitrogen until dissolution was complete. To this solution was added benzoyl chloride (82ml, 0.706mmol) and stirring was continued for 17 hours. The solvent was removed in vacuo and the residue was partitioned between ethyl acetate (100ml) and dilute citric acid (150ml). The organic layer was washed successively with saturated sodium bicarbonate solution (2 x 50ml) and brine (50ml) then dried (MgSO₄) and evaporated to give the crude product (0.26g) which was recrystallised from ethyl acetate/petroleum ether (bp 60-80°C) to give the pure title compound as colourless crystals (0.201g, m.p. 258-259°C).

### c) Trans-2-carboxy-5,7-dichloro-4-benzoylamino-1,2,3,4-tetrahydroquinoline

To a solution of trans-5,7-dichloro-2-methoxy-carbonyl-4-benzoylamino-1,2,3,4-tetrahydroquinoline (0. 189g, 0.499mmol) in tetrahydrofuran (10ml) was added distilled water (5ml) followed by aqueous lithium hydroxide (1.10ml of a 0.50M solution, 0.549mmol) and the resulting mixture was stirred at room temperature for 3 hours. The organic solvent was removed under vacuum and to the aqueous residue was added saturated sodium bicarbonate solution (20ml) and deionised water (50ml). The mixture was washed with ethyl acetate (2 x 50ml), then acidified to pH 1 with dilute hydrochloric acid and extracted with ethyl acetate (2 x 50ml). The combined organic extracts were washed with brine (50ml) then dried (Na₂SO₄) and evaporated to give the crude product (0.16g) which was triturated with diethyl ether to give two crops of the pure title compound as a colourless crystalline solid (0.11g, m.p. 233-236°C).

### EXAMPLE 2

### Trans-2-carboxy-5,7-dichloro-4-acetylamino-1,2,3,4-tetrahydroquinoline

### a) Trans-5,7-dichloro-2-methoxycarbonyl-4-acetyl-amino-1,2,3,4-tetrahydroquinoline

To a suspension of trans-4-amino-5,7-dichloro-2-methoxycarbonyl-1,2,3,4-tetrahydroquinoline hydrochloride (0.15g, 0.481mmol) in anhydrous dichloromethane (15ml) was added dry triethylamine (0.141ml, 1.01mmol) and the resulting mixture was stirred at room temperature under an atmosphere of nitrogen until dissolution was complete. To this solution was added acetyl chloride (38ml, 0.529mmol) and the reaction mixture was stirred under an atmosphere of nitrogen for a further 5 hours at room temperature. The precipitate was collected by filtration and was washed with dichloromethane to give the title compound as a colourless crystalline solid (0.133g, m.p. 280-282°C) (dec.).

### b) Trans-2-Carboxy-5,7-dichloro-4-acetylamino-1,2,3,4-tetrahydroquinoline

To a suspension of trans-5,7-dichloro-2-methoxy-carbonyl-4-acetylamino-1,2,3,4-tetrahydroquinoline (0.12g, 0.379mmol) in methanol (5ml) was added aqueous lithium hydroxide (0.42ml of a 1.0M solution, 0.42mmol) and the resulting mixture was stirred at room temperature for 7 hours. To this mixture was added additional aqueous lithium hydroxide (0.20ml of a 1.0M solution, 0.20mmol) and the mixture was stirred for a further 16 hours. The solution was evaporated to dryness in vacuo and the residue obtained was redissolved in water (50ml). The solution was acidified to pH l with dilute hydrochloric acid and extracted with ethyl acetate (2 x 30ml). The combined extracts were washed with brine (30ml), dried (Na₂SO₄) and evaporated to give the pure title compound as colourless crystals (0.108g, 223-225°C).

### EXAMPLE 3

### Trans-2-carboxy-4-cyclohexylcarbonylamino-5,7-dichloro-1,2,3,4-tetrahydroquinoline

### a) Trans-4-cyclohexylcarbonylamino-5,7-dichloro-2-methoxycarbonyl-1,2,3,4-tetrahydroquinoline

This material was prepared by the same method as described in Example 1b using cyclohexane carboxylic acid to give the title compound (m.p. 280-282°C dec).

### b) Trans-2-carboxy-4-cyclohexylcarbonylamino-5,7-dichloro-1,2,3,4-tetrahydroquinoline

This material was prepared using the same method as described in Example 1c to give the title compound (Et₂O, m.p. 190-195°C).

### EXAMPLE 4

### Trans-2-carboxy-5,7-dichloro-4-phenylmethylcarbonylamino-1,2,3,4-tetrahydroquinoline

### a) Trans-5,7-dichloro-2-methoxycarbonyl-4-phenylmethylcarbonylamino-1,2,3,4-tetrahydroquinoline

This material was prepared by the same method as described in Example 1b using phenylacetyl chloride to give the title compound as colourless crystals (m.p. 226-228°C).

### b) Trans-2-carboxy-5,7-dichloro-4-phenylmethylcarbonylamino-1,2,3,4-tetrahydroquinoline

This material was prepared by the same method as described in Example 1c to give the title compound as colourless crystals (m.p. 186-188°C dec).

### EXAMPLE 5

### Trans-2-carboxy-5,7-dichloro-4-(4-pyridyl)carbonyl amino-1,2,3,4-tetrahydroquinoline

### a) Trans-5,7-dichloro-2-methoxycarbonyl-4(4-pyridyl)carbonylamino-1,2,3,4-tetrahydroquinoline

To a suspension of trans-4-amino-5,7-dichloro-2-methoxycarbonyl-1,2,3,4-tetrahydroquinoline hydrochloride (example 1a) (0.200g, 0.642 mmol) and isonicotinoyl chloride hydrochloride (0. 124g, 0.706mmol) in anhydrous dichloromethane (20ml) under an atmosphere of nitrogen was added dry triethylamine (0.307ml, 2.2mmol) and the resulting mixture was stirred at room temperature for 1h. The mixture was then evaporated to dryness in vacuo and the residue was partitioned between water (100ml) and ethyl acetate (250ml). The organic layer was separated, washed successively with water (3 x 100ml), saturated sodium bicarbonate solution (1 x 100ml), brine (1 x 100ml), dried (MgSO₄) and evaporated. The resulting solid was triturated with ethyl acetate and collected by filtration to give the title compound as colourless crystals (0.200g) mp 200°C (dec).

### b) Trans-2-carboxy-5,7-dichloro-4(4-pydridyl)carbonylamino-1,2,3,4-tetrahydroquinoline

To a suspension of trans-5,7-dichloro-2-methoxycarbonyl- 4(4-pyridyl)-carbonylamino-1,2,3,4-tetrahydroquinoline (0.18g, 0.47mmol) in a mixture of tetrahydrofuran (15ml) and water (5ml) was added aqueous lithium hydroxide (1.04ml of a 0.50M solution, 0.521mmol) and the resulting mixture was stirred at room temperature for 3h. The organic solvent was removed in vacuo then the aqueous residue was diluted with dilute sodium bicarbonate solution (100ml) and washed with ethyl acetate (2 x 75ml). The aqueous layer was then acidified to pH 6 with dilute hydrochloric acid and extracted with ethyl acetate (4 x 75ml). The combined extracts were washed with brine (1 x 100ml), dried (Na₂SO₄) and evaporated to give the title compound as colourless crystals (0.116g), m.p. 270-275°C [dec].

### EXAMPLE 6

### Trans-2-carboxy-5,7-dichloro-4-n-propylcarbonylamino-1,2,3,4-tetrahydroquinoline

### a) Trans-5,7-dichloro-2-methoxycarbonyl-4-n-propylcarbonylamino-1,2,3,4-tetrahydroquinoline

To a suspension of trans-4-amino-5,7-dichloro-2-methoxycarbonyl-1,2,3,4-tetrahydroquinoline hydrochloride (example 1a) (0.200g, 0.642mmol) in anhydrous dichloromethane (15ml) under an atmosphere of nitrogen was added dry triethylamine (0. 197ml, 1.41mmol) and the mixture was stirred until dissolution was complete. To this solution was then added butyryl chloride (0.073ml, 0.706mmol) and the reaction was stirred at room temperature for 4h. The mixture was then evaporated to dryness in vacuo and the residue was partitioned between ethyl acetate (50ml) and 0.5M aqueous citric acid (30ml). The organic layer was separated, washed successively with saturated sodium bicarbonate solution (1 x 30ml) and brine (1 x 30ml), then dried (MgSO₄) and evaporated to give a solid which was triturated with diethyl ether. The solid was collected by filtration to give the pure title compound as colourless crystals (0. 187g), m.p. 205-206°C.

### b) Trans-2-carboxy-5,7-dichloro-4-n-pronylcarbonylamino-1,2,3,4-tetrahydroquinoline

To a solution of trans-5,7-dichloro-2-methoxycarbonyl-4-n-propylcarbonyl-amino-1,2,3,4-tetrahydroquinoline (0.70g, 0.493mmol) in a mixture of tetrahydrofuran (15ml) and water (5ml) was added aqueous lithium hydroxide (1.1ml of a 0.50M solution, 0.55mmol) and the resulting mixture was stirred at room temperature for 20h. The organic solvent was then removed under vacuum and the aqueous residue was diluted with dilute sodium bicarbonate solution (50ml) then washed with ethyl acetate (1 x 30ml). The aqueous phase was separated, acidified with dilute hydrochloric acid and extracted with ethylacetate (2 x 50ml). The combined extracts were washed with brine (1 x 50ml), dried (MgSO₄) and evaporated to give a solid which was triturated with diethyl ether to give the title compound as colourless crystals (0.123g) m.p. 174-176°C (dec).

### EXAMPLE 7

### Trans-2-carboxy-5,7-dichloro-4-phenylmethylcarbonylamino-1,2,3,4-tetrahydroquinoline

### a) Trans-5,7-dichloro-2-methoxycarbonyl-4-phenylmethylcarbonylamino-1,2,3,4-tetrahydroquinoline

To a suspension of 4-amino-5,7-dichloro-2-methoxycarbonyl-1,2,3,4-tetrahydroquinoline hydrochloride (example 1a) (3.0g, 9.63mmol) in anhydrous tetrahydrofuran (300ml) under an atmosphere of nitrogen was added dry triethylamine (2.95ml, 21.2mmol) and the resulting mixture was stirred at room temperature for 0.5h. To this suspension was added phenylacetic acid (1.44g, 10.6mmol), 1-hydroxybenzotriazole (1.43g, 10.6mmol) and 1-(3-dimethylaminopropyl)-3-ethylcarbodiimide hydrochloride (2.03g, 10.6mmol) and stirring was continued for 65h. The reaction mixture was concentrated in vacuo and the residue obtained was partitioned between ethyl acetate (1000ml) and 1M aqueous citric acid (300ml). The organic layer was collected, washed successively with 1M aqueous citric acid (1 x 300ml), saturated sodium bicarbonate solution (2 x 300ml) and brine (200ml) then dried (MgSO₄) and evaporated. The resulting crude product was recrystallised from methanol to give the title compound as colourless needles (2 crops, 3.26g) identical in physical properties to the material obtained in example 4 step a.

### b) Trans-2-carboxy-5,7-dichloro-4-phenylmethylcarbonylamino-1,2,3,4-tetrahydroquinoline

To a suspension of trans-5,7-dichloro-2-methoxy-carbonyl-4-phenylmethylcarbonylamino-1,2,3,4-tetrahydroquinoline (3.11g, 7.89mmol) in a mixture of tetrahydrofuran (100ml) and water (50ml) was added aqueous lithium hydroxide (17.4ml of a 0.5M solution, 8.70mmol) and the resulting mixture was stirred at room temperture for 3h. The organic solvent was removed in vacuo and the aqueous residue was acidified with concentrated hydrochloric acid and extracted with ethyl acetate (2 x 100ml). The combined extracts were washed with saturated brine (1 x 100ml), dried (MgSO₄) and evaporated to give the crude product which was recrystallised from ethyl acetate/petroleum ether 60-80 to give the title compound as colourless crystals (2 crops, 2.65g) identical in physical properties to example 4 step b.

### EXAMPLE 8

### Trans-2-carboxy-5,7-dichloro-4-phenylaminocarbonylamino-1,2,3,4-tetrahydroquinoline

### a) Trans-5,7-dichloro-2-methoxycarbonyl-4-phenylaminocarbonylamino-1,2,3,4-tetrahydroquinoline

To a suspension of trans-4-amino-5,7-dichloro-2-methoxy-carbonyl-1,2,3,4-tetrahydroquinoline hydrochloride (0.200g, 0.642mmol) in anhydrous dichloromethane (20ml) under an atmosphere of nitrogen was added dry triethylamine (0.098ml, 0.706mmol) and the mixture was stirred until dissolution was complete. To this solution was then added phenyl isocyanate (0.077ml, 0.706mmol) and the resulting mixture was stirred at room temperature for 2h. The solvent was removed under vacuum and the residue was partitioned between ethyl acetate (150ml) and 1M aqueous citric acid (75ml). The organic layer was successively washed with 1M aqueous citric acid (1 x 75ml), saturated sodium bicarbonate solution (2 x 75ml) and saturated brine ( 1 x 75ml), then dried (MgSO₄) and evaporated. The crude product was recrystallised from methanol to give the title compound as colourless crystals (0.185g), m.p. 228-229°C (dec).

### b) Trans-2-carboxy-5,7-dichloro-4-phenylaminocarbonylamino-1,2,3,4-tetrahydroquinoline.

To a suspension of trans-5,7-dichloro-2-methoxycarbonyl-4-phenylaminocarbonylamino-1,2,3,4-tetrahydroquinoline (0.185g, 0.47mmol) in a mixture of tetrahydrofuran (10ml) and water (5ml) was added aqueous lithium hydroxide (1.04ml of a 0.5M solution, 0.52 mmol) and the resulting mixture was stirred at room temperature for 2h. The reaction mixture was concentrated in vacuo and the aqueous residue was dissolved in dilute sodium bicarbonate solution (100ml) then washed with diethyl ether (100ml). The aqueous phase was separated, acidified with concentrated hydrochloric acid and extracted with ethyl acetate (2 x 100ml). The combined extracts were washed with brine (100ml), dried (MgSO₄) and evaporated to give the crude product which was redissolved in methanol and evaporated to give an oil which was crystallised with diethyl ether. The title compound was collected by filtration to give colourless crystals (0.110g), m.p. 148-150°C (starts to decompose above 120°C).

### EXAMPLE 9

### Trans-2-carboxy-4(4-chlorophenyl)carbonylamino-5,7-dichloro-1,2,3,4-tetrahydroquinoline

This compound was prepared by the same method given for example 6 using 4-chlorobenzoyl chloride in place of butyryl chloride to give the title compound as colourless crystals m.p. 228-229°C (dec).

### EXAMPLE 10

### Trans-2-carboxy-5,7-dichloro-4(4-methoxyphenyl)carbonylamino-1,2,3,4-tetrahydroquinoline

This compound was prepared by the same method given for example 6 using para-anisoyl chloride in place of butyryl chloride to give the title compound as colourless crystals, m.p. 209-210°C (dec).

### EXAMPLE 11

### Trans-2-carboxy-5,7-dichloro-4-phenylmethylaminocarbonylamino-1,2,3,4-tetrahydroquinoline

This compound was prepared using the same method given for example 8 using benzyl isocyanate in place of phenyl isocyanate to give the title compound as colourless crystals, m.p. 163-164°C (dec).

### EXAMPLE 12

### Trans-2-carboxy-5,7-dichloro-4(2-methoxyphenyl) methylcarbonylamino-1,2,3,4-tetrahydroquinoline

This compound was prepared using the same method given for example 7 using 2-methoxyphenylacetic acid in place of phenylacetic acid to give the title compound as colourless crystals, m.p. 222-223°C (dec).

### EXAMPLE 13

### Trans-2-carboxy-5,7-dichloro-4(2-methylphenyl)methylcarbonylamino-1,2,3,4-tetrahydroquinoline

This compound was prepared using the same method given for example 7 using ortho-tolylacetic acid in place of phenylacetic acid to give the title compound as colourless crystals, m.p. 214-215°C (dec).

### EXAMPLE 14

### Trans-2-carboxy-5,7-dichloro-4-phenyl(methoxy) methylcarbonylamino-1,2,3,4-tetrahydroquinoline (Isomer A)

### Step a): Trans-5,7-dichloro-2 methoxycarbonyl-4-phenyl(methoxy) methylcarbonylamino-1,2,3,4-tetrahydroquinoline (Isomers A and B)

To a suspension of trans-4-amino-5,7-dichloro-2-methoxy-carbonyl-1,2,3,4-tetrahydroquinoline hydrochloride (example 1a) (0.400g, 1.28mmol) in anhydrous THF (50ml) was added dry triethylamine (0.393ml, 2.82mmol) and the resulting mixture was stirred at room temperature under an atmosphere of nitrogen for 0.25h. To this suspension was then added (+)-α-methoxyphenylacetic acid (0.234g, 1.41mmol), 1-hydroxybenzotriazole (0.191g, 1.41mmol) and 1-(3-dimethylaminopropyl)-3-ethylcarbodiimide hydrochloride (0.270g, 1.41mmol) and stirring was continued for 4h. The solvent was then removed under vacuum and the residue was partitioned between ethyl acetate (100ml) and 1M aqueous citric acid (100ml). The organic layer was separated and washed successively with 1M aqueous citric acid (1 x 75ml), saturated sodium bicarbonate solution (2 x 75ml), saturated brine (1 x 75ml) then dried (MgSO₄) and evaporated to give a solid. The two diastereoisomers were separated by flash chromatography using 30-40% ethyl acetate in petroleum ether (bp 60-80°) as eluent to give, after recrystallisation from hot ethyl acetate-petroleum (ether bp 60-80°), the less polar isomer A (0.234g, m.p. 182-184°C) and the more polar isomer B (0.162g, m.p. 228-229°C).

### Step b: Trans-2-carboxy-5,7-dichloro-4-phenyl(methoxy) methylcarbonylamino-1,2,3,4-tetrahydroquinoline (Isomer A)

To a solution of trans-5,7-dichloro-2-methoxycarbonyl-4-phenyl(methoxy)methylcarbonylamino-1,2,3,4-tetrahydroquinoline (Isomer A, 0.180g, 0.426mmol) in a mixture of tetrahydrofuran (10ml) and water (5ml) was added aqueous lithium hydroxide (0.904ml of a 0.50M solution, 0.47mmol), and the resulting mixture was stirred at room temperature for 3h. The solvent was then removed under vacuum and the residue was diluted with sodium bicarbonate solution (50ml) and washed with diethyl ether (1 x 30ml). The aqueous layer was separated, acidified with concentrated hydrochloric acid and extracted with ethyl acetate (2 x 40ml). The combined extracts were washed with saturated brine (1 x 40ml), dried (MgSO₄) and evaporated to give a solid which was recrystallised from hot ethyl acetate/petroleum ether (bp 60-80°) to give the title compound as colourless crystals (0.102g), m.p. 232-233°C.

### EXAMPLE 15

### Trans-2-carboxy-5,7-dichloro-4-phenyl(methoxy) methylcarbonylamino-1,2,3,4-tetrahydroquinoline (Isomer B)

To a solution of trans-5,7-dichloro-2-methoxycarbonyl-4-phenyl(methoxy)methylcarbonylamino-1,2,3,4-tetrahydroquinoline (Isomer B) (0.140g, 0.331mmol) (Example 14a) in a mixture of tetrahydrofuran (10ml) and water (5ml) was added aqueous lithium hydroxide (0.73ml of a 0.5M solution, 0.364mmol) and the resulting mixture was stirred at room temperature for 2h. The organic solvent was removed under vacuum and the residue was diluted with sodium bicarbonate solution (50ml) then washed with diethyl ether (1 x 30ml). The aqueous layer was separated, acidified with concentrated hydrochloric acid and extracted with ethyl acetate (2 x 40ml). The combined extracts were washed with saturated brine (1 x 40ml), dried (MgSO₄) and evaporated to give the crude product which was recrystallised from hot ethyl acetate/petroleum ether (bp 60-80°) to give the title compound as colourless crystals (0.070g), m.p. 238-239°C.

### EXAMPLE 16

### Trans-2-carboxy-5,7-dichloro-4-(2-nitrophenyl)methyl carbonylamino-1,2,3,4-tetrahydroquinoline

This compound was prepared using the same method given for example 7 using 2-nitrophenylacetic acid in place of phenylacetic acid to give the title compound as colourless crystals, m.p. 220-221°C (dec).

### EXAMPLE 17

### Trans-2-carboxy-5,7-dlchloro-4-(2-nitrophenyl)amino carbonylamino-1,2,3,4-tetrahydroquinoline

This compound was prepared using the same method given for example 8 using 2-nitrophenyl isocyanate in place of phenyl isocyanate to give the title compound as yellow crystals, mp 214-215°C (dec).

### EXAMPLE 18

### Trans-2-carboxy-5,7-dichloro-4-(2-methoxyphenyl)amino carbonylamino-1,2,3,4-tetrahydroquinoline

This compound was prepared using the same method given for example 8 using 2-methoxyphenyl isocyanate in place of phenylisocyanate to give the title compound as colourless crystals, m.p. 198-199°C (dec).

### EXAMPLE 19

### Trans-2-carboxy-5,7-dichloro-4-(2-methylphenyl)aminocarbonylamino-1,2,3,4-tetrahydroquinoline

This compound was prepared using the method given for example 8 using ortho-tolyl isocyanate in place of phenyl isocyanate to give the title compound as colourless crystals, m.p. 196-197°C (dec).

### EXAMPLE 20

### Trans-2-carboxy-5,7-dichloro-4(2-chlorophenyl)amino carbonylamino-1,2,3,4-tetrahydroquinoline

This compound was prepared using the method given for example 8 using ortho-chlorophenyl isocyanate in place of phenyl isocyanate to give the title compound as colourless crystals, m.p. 203-204°C (dec).

Examples 21 to 34 hereinbelow were prepared by the method of Example 6 utilising the appropriate acid chloride in place of butynyl chloride.

### EXAMPLE 21

### Trans-2-carboxy-5,7-dichloro-4(4-phenyl)phenylcarbonyl amino-1,2,3,4-tetrahydroquinoline

Colourless crystals, m.p. 267°C.

### EXAMPLE 22

### Trans-2-carboxy-5,7-dichloro-4-isopropylcarbonylamino- 1,2,3,4-tetrahydroquinoline

Colourless crystals, m.p. 197-198°C.

### EXAMPLE 23

### Trans-2-carboxy-5,7-dichloro-4-(2-chlorophenyl)carbonylamino-1,2,3,4-tetrahydroquinoline

Colourless crystals, m.p. 141-143°C.

### EXAMPLE 24

### Trans-2-carboxy-5,7-dichloro-4-(1-naphthalenyl)carbonyl amino1,2,3,4-tetrahydroquinoline

Colourless crystals, m.p. 263-264°C.

### EXAMPLE 25

### Trans-2-carboxy-5,7-dichloro-4-(2-naphthalenyl)carbonylamino-1,2,3,4-tetrahydroquinoline

Colourless crystals, m.p. 193-194°C.

### EXAMPLE 26

### Trans-2-carboxy-5,7-dichloro-4-(2-furanylcarbonyl)amino-1,2,3,4-tetrahydroquinoline

Colourless crystals, m.p. 219-220°C.

### EXAMPLE 27

### Trans-2-carboxy-5,7-dichloro-4-(2-methylphenyl)carbonyl amino-1,2,3,4-tetrahydroquinoline

Colourless crystals, m.p. 153-156°C.

### EXAMPLE 28

### Trans-2-carboxy-5,7-dichloro-4-(phenethylcarbonyl)amino-1,2,3,4-tetrahydroquinoline

Colourless crystals, m.p. 188°C.

### EXAMPLE 29

### Trans-2-carboxy-5,7-dichloro-4-(phenethenyl carbonyl)amino-1,2,3,4-tetrahydroquinoline

Colourless crystals, m.p. 214-216°C.

### EXAMPLE 30

### Trans-2-carboxy-5,7-dichloro-4-(2-thiophenylmethyl carbonyl)amino-1,2,3,4-tetrahydroquinoline

Colourless crystals, m.p. 166-169°C.

### EXAMPLE 31

### Trans-2-carboxy-5,7-dichloro-4-(3-chlorophenyl)carbonyl amino-1,2,3,4-tetrahydroquinoline

Colourless crystals, m.p. 262-263°C.

### EXAMPLE 32

### Trans-2-carboxy-5,7-dichloro-4-(3-phenylpropyl) carbonylamino-1,2,3,4-tetrahydroquinoline

Colourless crystals, m.p. 189-191°C.

### EXAMPLE 33

### Trans-2-carboxy-5,7-dichloro-4-(9-fluorenyl)carbonyl amino-1,2,3,4-tetrahydroquinoline

Colourless crystals, m.p. 266-268°C.

### EXAMPLE 34

### Trans-2-carboxy-5,7-dichloro-4-(cyclohexylmethylcarbonyl) amino-1,2,3,4-tetrahydroquinoline

Colourless crystals m.p. 197-200°C.
Examples 35 to 41 hereinbelow were prepared by the method of Example 7 utilising the appropriate carboxylic acid in place of phenylacetic acid.

### EXAMPLE 35

### Trans-2-carboxy-5,7-dichloro-4-(2-chlorophenyl)methylcarbonylamino- 1,2,3,4-tetrahydroquinoline

Colourless crystals, m.p. 244-245°C.

### EXAMPLE 36

### Trans-2-carboxy-5,7-dichloro-4-(3-chlorophenylmethylcarbonyl)amino-1,2,3,4-tetrahydroquinoline

Colourless crystals, m.p. 193-194°C.

### EXAMPLE 37

### Trans-2-carboxy-5,7-dichloro-4-(4-chlorophenylmethylcarbonyl)amino-1,2,3,4-tetrahydroquinoline

Colourless crystals, m.p. 244-245°C.

### EXAMPLE 38

### Trans-2-carboxy-5,7-dichloro-4-(4-methylphenylmethylcarbonyl)amino-1,2,3,4-tetrahydroquinoline

Colourless crystals, m.p. 229-230°C.

### EXAMPLE 39

### Trans-2-carboxy-5,7-dichloro-4-(4-methoxyphenylmethyl)carbonylamino-1,2,3,4-tetrahydroquinoline

Colourless crystals, m.p. 245-247°C.

### EXAMPLE 40

### Trans-2-carboxy-5,7-dichloro-4-(4-nitrophenylmethylcarbonyl)amino-1,2,3,4-tetrahydroquinoline

Yellow crystals m.p. 246-248°C.

### EXAMPLE 41

### Trans-2-carboxy-5,7-dichloro-4-(3-cyanophenylcarbonyl)amino-1,2,3,4-tetrahydroquinoline

Colourless crystals, m.p. 229-231°C.

### EXAMPLE 42

### Trans-2-carboxy-5,7-dichloro-4-(4-chlorophenyl)aminocarbonylamino-1,2,3,4-tetrahydroquinoline

This compound was prepared by the method given for Example 8 using 4-chlorophenyl isocyanate in place of phenyl isocyanate to give the title compound as colourless crystals, m.p. 199-200°C.

### EXAMPLE 43

### Trans-2-carboxy-5,7-dichloro-4-(4-methylphenyl)aminocarbonylamino-1,2,3,4-tetrahydroquinoline

This compound was prepared by the method given for Example 8 using 4-methylphenyl isocyanate in place of phenyl isocyanate to give the title compound as colourless crystals, m.p. 197-198°C.

### EXAMPLE 44

### Trans-2-carboxy-5,7-dichloro-4-(4-methoxyphenyl)aminocarbonylamino-1,2,3,4-tetrahydroquinoline

This compound was prepared by the method given for example 8 using 4-methoxyphenyl isocyanate in the place of phenyl isocyanate to give the title compound as colourless crystals, m.p. 189-190°C.

### EXAMPLE 45

### Trans-2-carboxy-5,7-dichloro-4-(4-nitrophenyl)amino carbonylamino-1,2,3,4-tetrahydroquinoline

This compound was prepared by the method given for Example 8 using 4-nitrophenyl isocyanate in place of phenyl isocyanate to give the title compound as yellow crystals, m.p. 185-186°C.

### EXAMPLE 46

### Trans-2-carboxy-5,7-dichloro-4-(4-iodophenyl)amino carbonylamino-1,2,3,4-tetrahydroquinoline

### Step a: 1-Phenyl-3-(4-Iodophenyl)carbamate

To a solution of 4-iodoaniline (1.94g, 8.85mmol) in anhydrous dichloromethane (40ml) was added phenylchloroformate (1.11ml, 8.85mmol) and triethylamine (1.42ml, 10.28mmol). The mixture was stirred under an atmosphere of nitrogen for 18h. The dichloromethane was removed in vacuo and the solid residue partitioned between ethyl acetate (100ml) and 0.5M citric acid (150ml). The organic layer was retained and washed succesively with 0.5M citric acid (100ml) saturated sodium bicarbonate solution (2 x 150ml) and brine (100ml) before drying (Na₂SO₄). The solvent was removed by rotary evaporation and the solid residue recrystallised from ethyl acetate/hexane to give the title compound as colourless crystals (2.61g) m.p. 155-157°C.

### Step b: Trans-2-methoxycarbonyl-5,7-dichloro-4-(4-iodophenyl) aminocarbonylamino-1,2,3,4-tetrahydroquinoline

To a solution of 1-phenyl-3-(4-iodophenyl)carbamate (770mg, 7.61mmol), in anhydrous toluene (15ml) was added dry triethylamine (0.670ml, 4.33mmol). The solution was heated, with stirring, in an oil bath to 120°C and then allowed to cool to (0.55ml, 4.33mmol). The solution was heated at reflux (120°C) for 2h. The mixture was cooled below 30°C and a solution of trans-2-methoxycarbonyl-5,7-dichloro-4-amino-tetrahydroquinoline-hydrochloride (example 1a) (750mg, 2.41mmol) was added as a solution in anhydrous dichloromethane (10ml) with triethylamine (0.403ml, 2.87mmol). The mixture was stirred under an atmosphere of nitrogen for 18h. The organic solvents were removed in vacuo and the solid residue partitioned between 0.5M citric acid (100ml) and ethyl acetate (100ml). The organic layer was retained and washed succesively with 0.5M citric acid (100ml), saturated sodium bicarbonate solution (2 x 100ml) and brine (100ml) before drying (Na₂SO₄). The solvent was removed and the product recrystallised from ethyl acetate/hexane to give the title compound as colourless crystals (668mg), m.p. 253-255°C.

### Step c: Trans-2-carboxy-5,7-dichloro-4-(4-iodophenyl) aminocarbonylamino-1,2,3,4-tetrahydroquinoline

This compound was prepared by the method given for Example 8 step b using trans-2-methoxycarbonyl-5,7-dichloro-4(4-iodophenylaminocarbonyl)amino-1,2,3,4-tetrahydroquinoline in place of trans-2-methoxycarbonyl-5,7-dichloro-4(phenylaminocarbonyl)amino-1,2,3,4-tetrahydroquinoline to give the title compound as colourless crystals, m.p. 224-226°C.

### EXAMPLE 47

### Trans-2-carboxy-5,7-dichloro-4-(phenyl)aminocarbonyl-N-methylamino)-1,2,3,4-tetrahydroquinoline

### Step a: Trans-2-methoxycarbonyl-5,7-dichloro-4-N-methylamino-1,2,3,4-tetrahydroquinoline

Trans-2-carboxy-5,7-dichloro-4-amino-1,2,3,4-tetrahydroquinoline hydrochloride (Example 1a) (760mg, 2.44mmol) was shaken with a solution of potassium carbonate for 5 min. The solid in suspension was extracted into ethyl acetate (2 x 50ml). The combined organic extracts were evaporated and the solid residue redissolved in anhydrous tetrahydrofuran (300ml). This solution was cooled (-5°C) and a solution of methyl iodide (159µl, 2.56mmol) in tetrahydrofuran (50ml) was added dropwise, under an atmosphere of nitrogen. The mixture was allowed to warm to room temperature and was stirred for 70h. The THF was removed and the solid residue redissolved in 30ml of THF. A further quantity of methyl iodide (159µl, 2.56mmol) was added as a solution in THF (5ml) and the mixture stirred for a further 16h in an atmosphere of nitrogen. The solvent was evaporated and the residue separated by silica chromatography (eluent; methanol/dichloromethane) to yield the title compound as a colourless crystalline solid.

### Step b: Trans-2-carboxy-5,7-dichloro-4-(phenyl) aminocarbonyl-N-(methylamino)-1,2,3,4-tetrahydroquinoline

This compound was prepared by the method given for Example 8 using trans-2-methoxycarbonyl-5,7-dichloro-4-N-methylamino-1,2,3,4-tetrahydroquinoline in place of trans-2-methoxycarbonyl-5,7-dichloro-4-amino- 1,2,3,4-tetrahydroquinoline hydrochloride and triethylamine, to give the title compound as colourless crystals, m.p. 139-140°C.

### EXAMPLE 48

### Trans-2-carboxy-5,7-dichloro-4-(-N-methyl)-(N-phenyl)aminocarbonylamino-1,2,3,4-tetrahydroquinoline

### Step a: Trans-2-methoxycarbonyl-5,7-dichloro-4-(phenyloxy)carbonylamino-1,2,3,4-tetrahydroquinoline

This compound was prepared by the method given for Example 6, step a, using phenylchloroformate in the place of butyryl chloride to give the title compound as colourless crystals, m.p. 159-161°C.

### Step b: Trans-2-methoxycarbonyl-5,7-dichloro-4(N-methyl)-(N-phenyl)aminocarbonylamino-1,2,3,4-tetrahydroquinoline

To a solution of trans-2-methoxycarbonyl-5,7-dichloro-4-(phenyloxy)carbonylamino-1,2,3,4-tetrahydroquinoline (225mg, 0.587mmol) in anhydrous toluene (7ml) was added dry triethylamine (245µl, 1.76mmol). The mixture was heated with stirring, in an oil bath to 120°C and then allowed to cool to (186µl, 1.47mmol). The solution was heated at reflux (120°C) for 2h. After cooling to triethylamine was added (81µl, 0.587mmol) followed by N-methylaniline (126mg, 1.174mmol). The mixture was stirred at room temperature under an atmosphere of nitrogen for 18h. The organic solvent was removed and the solid residue paritioned between ethyl acetate (75ml) and 0.5M citric acid (100ml). The organic layer was retained and washed successively with 0.5M citric acid (100ml), saturated sodium bicarbonate solution (2 x 100ml) and brine (100ml) before drying (Na₂SO₄). The solvent was removed in vacuo to yield an orange oil which was isolated by flash chromatography (solvent; ethyl acetate/hexane) to give the title compound as colourless crystals (89mg) m.p. 185-189°C.

### Step c: Trans-2-carboxy-5,7-dichloro-4-(N-methyl)-)N-phenyl)amino)carbonylamino-1,2,3,4-tetrahydroquinoline

This compound was prepared by the method given for Example 8, step b, using trans-2-methoxycarbonyl-5,7-dichloro-4-(N-methyl)-(N-phenyl)aminocarbonylamino-1,2,3,4-tetrahydroquinoline in place of trans-2-methoxycarbonyl-5,7-dichloro-4(phenyl)N-methylaminocarbonylamino-1,2,3,4-tetrahydroquinoline to give the title compound as colourless crystals, m.p. 204-205°C.

### EXAMPLE 49

### Trans-2-carboxy-5,7-dichloro-4-(2,3-dihydroindole-1-carbonyl)amino-1,2,3,4-tetrahydroquinoline

This compound was prepared by the method given for Example 48 using indoline in place of N-methylaniline to give the title compound as colourless crystals, m.p. 251-252°C.

### EXAMPLE 50

### Trans-2-carboxy-5,7-dichloro-4-(2-carboxyethyl)carbonyl)amino-1,2,3,4-tetrahydroquinoline

### Step a: trans-2-methoxycarbonyl-5,7-dichloro-4-(2-carboxyethyl)carbonylamino-1,2,3,4-tetrahydroquinoline

To a suspension of trans-2-methoxycarbonyl-5,7-dichloro-4-amino-1,2,3,4-tetrahydroquinoline hydrochloride (Example 1a) (225mg, 0.819mmol) in ethyl acetate (50ml) was added a solution of potassium carbonate (50ml). The mixture was shaken until no solid remained (5min). The organic layer was retained and washed with brine (50ml) before drying (Na₂SO₄). The solvent was removed in vacuo. The residue was redissolved in xylene (20ml) and succinic anhydride (90mg, 0.901mmol) was added. The mixture was heated, with stirring, in an oil bath (80°C) for 16h. The precipitate was removed by filtration and washed with ether to give the title compound as a colourless crystalline solid.

### Step b: Trans-2-carboxy-5,7-dichloro-4-(2-carboxyethyl)carbonylamino-1,2,3,4-tetrahydroquinoline

This compound was prepared by the method given for Example 6, step b, using trans-2-methoxycarbonyl-5,7-dichloro-4-(2-carboxyethyl)carbonylamino-1,2,3,4-tetrahydroquinoline in place of trans-2-methoxycarbonyl-5,7-dichloro-4-butylcarbonylamino-1,2,3,4-tetrahydroquinoline to give the title compound as colourless crystals, m.p. 221-223°C.

### EXAMPLE 51

### Trans-2-carboxy-5,7-dichloro-4-(3-aminomethylphenyl)carbonylamino-1,2,3,4-tetrahydroquinoline hydrochloride

### Step a: 3-(tert-Butyloxycarbonylamino)methyl benzoic acid

To a solution of 3-cyanobenzoic acid (1.0g, 6.79mmol) in ethanol (150ml) was added 100mg of 10% palladium on carbon and the mixture was hydrogenated at 50 psi for approximately 18h. Di-tert-butyl dicarbonate (1.63g, 7.47mmol) was added to the mixture, with a further 110mg of catalyst and hydrogenation was continued under the same conditions for a further 20h. After this time all the starting material had been consumed by t.l.c. (solvent: 1% MeOH, 0.5% AcoH, 98.5% CH₂Cl₂). The catalyst was removed by filtration, and the ethanol was removed by rotary evaporation to give an orange oil which crystallised after drying under high vacuum (1h). The product was recrystallised from ether/hexane to give the title compound as colourless crystals, m.p. 127-128°C.

### Step b: Trans-2-methoxycarbonyl-5,7-dichloro-4-(3-tert-butyloxycarbonylaminomethylphenyl)carbonylamino-1,2,3,4-tetrahydroquinoline

To a suspension of 2-methoxycarbonyl-5,7-dichloro-4-amino-1,2,3,4-tetrahydroquinoline hydrochloride (Example 1) (300mg, 0.963mmol) in anhydrous dichloromethane (15ml) under an atmosphere of nitrogen was added dry triethylamine (403µl, 2.89mol) and the resulting mixture was stirred at room temperature for 5 min. To this was added 3-(tert-butyloxycarbonylamino)methyl benzoic acid (step a) (290mg, 1.16mmol), 1-hydroxybenzotriazole (156mg, 1.16mol) followed by 1-(3-dimethylaminopropyl)-3-ethylcarbodiimide hydrochloride (222mg, 1.16mmol) and stirring was continued for approximately 18h. The mixture was then concentrated in vacuo and the residue partitioned between ethyl acetate (100ml) and 0.5M citric acid (150ml). The organic layer was retained and washed successively with 0.5M citric acid (150ml), saturated sodium bicarbonate solution (2 x 150ml) and brine (100ml) before drying (Na₂SO₄). The solvent was removed and the residue was recrystallised from ethyl acetate/hexane to give the title compound as colourless crystals (351mg), m.p. 222-223°C.

### Step c: Trans-2-carboxy-5,7-dichloro-4-(3-tert-butyloxycarbonylaminomethylphenyl)carbonylamino-1,2,3,4-tetrahydroquinoline

To a solution of trans-2-methoxycarbonyl-5,7-dichloro- 4-(3-tert-butyloxycarbonylaminomethylphenyl)carbonylamino-1,2,3,4-tetrahydroquinoline (step b) (430mg, 0.846mmol) in a mixture of tetrahydrofuran (20ml) and water (10ml) was added aqueous lithium hydroxide (1.95ml of a 0.5M solution, 0.973mmol), and the resulting mixture was stirred at room temperature for 3h. The organic solvent was removed in vacuo and the aqueous residue was diluted to 50ml before acidifying to pH 1 with 1N HCl. The white precipitate was extracted into ethyl acetate (150ml), washed with brine and dried (Na₂SO₄). The solution was evaporated to give a residue which was washed with diethyl ether and collected by filtration to give the title compound as a colourless crystalline solid, m.p. 192-193°C.

### Step d: Trans-2-carboxy-5,7-dichloro-4-(3-aminomethylphenyl)carbonylamino-1,2,3,4-tetrahydroquinoline hydrochloride

To a solution of trans-2-carboxy-5,7-dichloro-4-(3-tert-butyloxycarbonylaminomethylphenyl)carbonylamino-1,2,3,4-tetrahydroquinoline (85mg) in ethyl acetate (20ml) was added 25ml of ethyl acetate saturated with hydrogen chloride gas and the mixture was stirred in a stoppered flask for 6h. The ethyl acetate and excess hydrogen chloride were removed in vacuo and the solid residue was recrystallised from ethyl acetate/methanol to give the title compound as colourless crystals (46mg), m.p. 199-200°C.

### EXAMPLE 52

### Trans-2-methoxycarbonyl-5,7-dichloro-4-(3-aminomethylphenyl) carbonylamino-1,2,3,4-tetrahydroquinoline hydrochloride

A solution of hydrogen chloride in methanol was prepared by the cautious addition of acetyl chloride (6ml) to ice-cooled methanol (25ml). Trans-2-carboxy-5,7-dichloro-4-(3-aminomethylphenyl)carbonylamino-1,2,3,4-tetrahydroquinoline hydrochloride (87mg, 0.202mmol) (Example 51) was dissolved in the HCl/methanol solution and the resulting mixture was stirred in a stoppered flask for 2h. The methanol and excess hydrogen chloride were removed in vacuo and the product recrystallised from methanol/ether to give the title compound as a colourless crystalline solid (80mg) m.p. 173°C (dec).

### EXAMPLE 53

### Trans-2-carboxy-5,7-dichloro-4-(4-aminomethylphenyl) carbonylamino-1,2,3,4-tetrahydroquinoline hydrochloride

### step a: 4-(tert-Butyloxycarbonylamino)methyl benzoic acid

To a stirred suspension of 4-aminomethyl benzoic acid (5.0g, 0.0331mol) in 10% sodium carbonate solution (42ml, 0.0394mol) was added a solution of di-tert-butyl dicarbonate (7.43g, 0.0364mol) in dioxan (42ml) and the mixture was stirred at room temperature for 2h. The mixture was diluted with water to 300ml and washed with ether (100ml). The aqueous fraction was retained and acidified to pH3 with 1N citric acid solution. The precipitate was extracted into ethyl acetate, washed with water (2 x 100ml), brine (1 x 100ml) and dried (Na₂SO₄). The solvent was removed in vacuo to give a white solid m.p. 170-171°C.

### Step b: Trans-2-carboxy-5,7-dichloro-4- (4-aminomethylphenyl)carbonylamino-1,2,3,4-tetrahydroquinoline hydrochloride

This compound was prepared by the method given for Example 51, steps b, c, and d, using 4-(tert-butyloxycarbonylamino)methyl benzoic acid in place of 3-(tert-butyloxycarbonylamino)methyl benzoic acid to give the title compound as colourless crystals, m.p. 219°C (dec).

### EXAMPLE 54

### Trans-2-methoxycarbonyl-5,7-dichloro-4-(4-aminomethylphenyl)carbonylamino-1,2,3,4-tetrahydroquinoline hydrochloride

This compound was prepared by the method given for Example 52 using trans-2-carboxy-5,7-dichloro-4(4-aminomethylphenyl)carbonylamino-1,2,3,4-tetrahydroquinoline hydrochloride, Example 53, in place of trans-2-carboxy-5,7-dichloro-4(3-aminomethylphenyl)carbonylamino-1,2,3,4-tetrahydroquinoline hydrochloride to give the title compound as a colourless crystalline solid m.p. 238-240°C.

### EXAMPLE 55

### Trans-2-carboxy-5,7-dichloro-4-(4-aminoethylphenyl)carbonylamino-1,2,3,4-tetrahydroquinoline hydrochloride

### step a: (4-tert-Butyloxycarbonylamino)ethyl benzoic acid

To a suspension of 4-aminoethylbenzoic acid hydrochloride (4.32g, 0.0214mol), in anhydrous dichloromethance (50ml) was added triethylamine (8.94ml, 0.0642mol) and the mixture was stirred at room temperature for 10 min. To this solution was added di-tert-butyl-dicarbonate (5.14g, 0.236mol) and stirring was continued for 18h. Dimethyl ethylenediamine (2ml) was added and after stirring for a further 20 min the dichloromethane was removed in vacuo. The residue was partitioned between ethyl acetate (150ml) and 0.5M citric acid (100ml). The organic layer was retained and washed successively with 0.5M citric acid (100ml), water (3 x 150ml) and brine (100ml) before drying (Na₂SO₄). The solvent was removed in vacuo and the residue recrystallised from ethyl acetate/hexane to give the title compound as a colourless crystalline solid, m.p. 163-164°C.

### step b: Trans-2-carboxy-5,7-dichloro-4-(4-aminoethylphenyl)carbonylamino-1,2,3,4-tetrahydroquinoline hydrochloride

This compound was prepared by the method given for Example 51, steps b, c, and d, using 4-(tert-butyloxycarbonylamino)ethyl benzoic acid as the starting material in the place of 3-(tert-butyloxycarbonylamino)methyl benzoic acid to give the title compound as colourless crystals, m.p. 204°C (dec).

### EXAMPLE 56

### Trans-2-methoxycarbonyl-5,7-dichloro-4-(4-aminoethylphenyl)carbonylamino-1,2,3,4-tetrahydroquinoline hydrochloride

This compound was prepared by the method given for Example 52 using trans-2-carboxy-5,7-dichloro-4-(4-aminoethylphenyl)carbonylamino-1,2,3,4-tetrahydroquinoline hydrochloride Example 55, in the place of trans-2-carboxy-5,7-dichloro-4(3-aminomethylphenyl)carbonylamino-1,2,3,4-tetrahydroquinoline hydrochloride to give the title compound as a colourless crystalline solid, m.p. 218°C (dec).

### EXAMPLE 57

### Trans-2-carboxy-4-(3-methylphenyl)methylcarbonylamino-5,7-dichloro-1,2,3,4-tetrahydroquinoline

This compound was prepared by the method given for Example 7 using 3-methylphenylacetic acid in place of phenylacetic acid to give the title compound as colourless crystals, m.p. 169-171°C.

### EXAMPLE 58

### Trans-2-carboxy-5,7-dichloro-4-(3-nitrophenyl)methylcarbonylamino-1,2,3,4-tetrahydroquinoline

This compound was prepared using the same method given for Example 7 using 3-nitrophenylacetic acid in place of phenylacetic acid to give the title compound as yellow crystals, m.p. 260°C.

### EXAMPLE 59

### Trans-2-carboxy-5,7-dichloro-4-(3-methoxyphenyl)methylcarbonylamino-1,2,3,4-tetrahydroquinoline

This compound was prepared by the same method given for Example 6 using 3-methoxyphenylacetyl chloride in place of butyryl chloride to give the title compound as colourless crystals, m.p. 242-244°C.

### EXAMPLE 60

### Trans-2-carboxy-4-(1-naphthyl)methylcarbonylamino-5,7-dichloro-1,2,3,4-tetrahydroquinoline

This compound was prepared by the method given for Example 7 using 1-naphthylacetic acid in place of phenylacetic acid to give the title compound as colourless crystals, m.p. 257-258°C.

### EXAMPLE 61

### Trans-2-carboxy-4-(2-naphthyl)methylcarbonylamino-5,7-dichloro-1,2,3,4-tetrahydroquinoline

This compound was prepared by the method given for Example 7 using 2-naphthylacetic acid in place of phenylacetic acid to give the title compound as colourless crystals, m.p. 217-219°C.

### EXAMPLE 62

### Trans-2-carboxy-4-(3-thiophenyl)methylcarbonylamino-5,7-dichloro-1,2,3,4-tetrahydroquinoline

This compound was prepared by the method given for Example 7 using 3-thiopheneacetic acid in place of phenylacetic acid to give the title compound as colourless crystals, m.p. 198-200°C.

### EXAMPLE 63

### Trans-2-carboxy-4-(2,6-dichlorophenyl)methylcarbonyl amino-5,7-dichloro-1,2,3,4-tetrahydroquinoline

This compound was prepared by the method given for Example 7 using 2,6-dichlorophenylacetic acid in place of phenylacetic acid to give the title compound as colourless crystals, m.p. 235-237°C.

### EXAMPLE 64

### Trans-2-carboxy-4-(phenylamino)thiocarbonylamino-5,7-dichloro-1,2,3,4-tetrahydroquinoline

This compound was prepared by the method given for Example 8 using phenylisothiocyanate in place of phenylisocyanate to give the title compound as colourless crystals, m.p. 186-187°C.

### EXAMPLE 65

### Trans-2-carboxy-4-phenylmethoxycarbonylamino-5,7-dichloro-1,2,3,4-tetrahydroquinoline

Trans-4-amino-2-methoxycarbonyl-5,7-dichloro-1,2,3,4-tetrahydroquinoline hydrochloride (Example 1a) (0.3g, 0.00096ml) was dissolved in dichloromethane (30ml) together with triethylamine (0.4ml, 3 molar equivalents) and dibenzyl dicarbonate (0.33g, 1.2molar equivalents). The reaction mixture was stirred at room temperature for 14h then dimethylethylenediamine (0.3ml) was added and the reaction stirred for a further 2h. The solvents were removed by evaporation and the residue redissolved in ethyl acetate (40ml) and washed with 1 molar citric acid solution (2 x 30ml), saturated sodium hydrogen carbonate solution (2 x 30ml) and brine (1 x 30ml). The organic layer was dried (Na₂SO₄), filtered and concentrated in vacuo to leave a residue which was purified by chromatography on silica gel using 20% ethyl acetate in hexane as eluent to give a white solid (0.24g). This was suspended in 50% aqueous methanol (100ml) and stirred at room temperature for 60h in the presence of sodium hydroxide (0.2g). The methanol was removed in vacuo and the aqueous residue acidified to pH 1 with 1M hydrochloric acid. The solid which was precipitated was extracted into ethyl acetate (2 x 50ml), washed with brine (1 x 40ml), dried (Na₂SO₄), filtered and concentrated in vacuo. The residue was recrystallised from diethyl ether/hexane to give the title compound as colourless crystals, m.p. 169°C.

### EXAMPLE 66

### Trans-2-carboxy-5,7-dichloro-4-(3-methoxyphenyl)aminocarbonylamino-1,2,3,4-tetrahydroquinoline

This compound was prepared by the method given in Example 8 using 3-methoxyphenylisocyanate in place of phenylisocyanate to give the title compound as colourless crystals, m.p. 181-183°C.

### EXAMPLE 67

### Trans-2-carboxy-5,7-dichloro-4-(3-methylphenyl) aminocarbonylamino-1,2,3,4-tetrahydroquinoline

This compound was prepared by the method given in Example 8 using 3-methylphenylisocyanate instead of phenylisocyanate to give the title compound as colourless crystals, m.p. 172-174°C.

### EXAMPLE 68

### Trans-2-carboxy-5,7-dichloro-4-(3-chlorophenyl)aminocarbonylamino-1,2,3,4-tetrahydroquinoline

This compound was prepared by the method outlined in Example 8 using 3-chlorophenylisocyanate instead of phenylisocyanate to give the title compound as colourless crystals, m.p. 173-175°C.

### EXAMPLE 69

### Trans-2-carboxy-5,7-dichloro-4-(3-nitrophenyl) aminocarbonylamino-1,2,3,4-tetrahydroquinoline

This compound was prepared by the method given in Example 8 using 3-nitrophenylisocyanate instead of phenylisocyanate to give the title compound as yellow crystals, m.p. 209-210°C.

### EXAMPLE 70

### Trans-2-carboxy-4-(3-aminopropyl)carbonylamino-5,7-dichloro-1,2,3,4-tetrahydroquinoline hydrochloride

### a) 4-(Tertiarybutyloxycarbonylamino)butanoic acid

4-Amino butyric acid (2.06g, 0.02M) was suspended in a solution of tetrahydrofuran (30ml) and dichloromethane (20ml) with triethylamine (2.77ml, 1 molar equivalent) and ditertiarybutyl dicarbonate (4.36g, 1 molar equivalent) was added. After stirring at room temperature for 14h the solvents were evaporated and the residue was chromatographed on silica gel using 1% acetic acid, 4% methanol and 95% dichloromethane as eluent to give the required compound (1.9g) as an oily solid.

### b) Trans-2-carboxy-4-(3-aminopropyl)carbonylamino-5,7-dichloro-1,2,3,4-tetrahydroquinoline hydrochloride

4-(Tertiarybutyloxycarbonylamino)butanoic acid (0.3g, 1.5 molar equivalents), 2-methoxycarbonyl-5,7-dichloro-4-amino-1,2,3,4-tetrahydroquinoline hydrochloride (Example 1a) (0.3g, 0.000963M), hydroxybenzotriazole (0.156g, 1.2 molar equivalents) and triethylamine (0.4ml, 3 molar equivalents) were dissolved in anhydrous tetrahydrofuran (40ml) followed by 1-(3-dimethylaminopropyl)-3-ethylcarbodiimide hydrochloride (0.22g, 1.2 molar equivalents). After stirring at room temperature for 14h the solvents were evaporated, the residue was dissolved in ethyl acetate (100ml) and washed with 0.5M citric acid solution (2 x 50ml), saturated sodium hydrogen carbonate solution (2 x 50ml) and brine (1 x 50ml). The organic layer was dried (Na₂SO₄), filtered and concentrated in vacuo to leave a residue which was dissolved in 50% aqueous methanol and treated with sodium hydroxide (0.5g). After stirring the solution for 36h at room temperature the methanol was removed under vacuum and the residual aqueous solution was treated with 1M HCl until a pH of 1 was attained. The precipitated solid was extracted into ethyl acetate (2 x 20ml) and the combined organic layers were dried (Na₂SO₄), filtered and concentrated in vacuo. The residue obtained was treated with 5 molar HCl in ethyl acetate (30ml) and stirred at room temperature for 60h. The solvents were evaporated and the residue recrystallised from methanol/diethyl ether/ethyl acetate to give the title compound as a white crystalline solid, m.p. 238-240°C.

### EXAMPLE 71

### Trans-2-carboxy-4-(3-aminoethyl)carbonylamino-5,7-dichloro-1,2,3,4-tetrahydroquinoline hydrochloride

This compound was prepared by the method described in Example 70b using 3-(tertiarybutyloxycarbonylamino)-propanoic acid instead of 4-(tertiarybutyloxycarbonylamino)-butanoic acid to give the title compound as colourless crystals, m.p. 214-215°C.

### EXAMPLE 72

### Trans-2-carboxy-4-(4-aminobutyl)carbonylamino-5,7-dichloro-1,2,3,4-tetrahydroquinoline hydrochloride

This compound was prepared by the method described in Example 70a and 70b using 5-amino valeric acid instead of 4-aminobutyric acid to give the title compound as colourless crystals, m.p. 160°C (dec).

### EXAMPLE 73

### Trans-2-carboxy-4-(4-piperidylmethyl)carbonylamino-5,7-dichloro-1,2,3,4-tetrahydroquinoline hydrochloride

This compound was prepared by the method described in Example 70a and 70b using 4-piperidine acetic acid in place of 4-aminobutyric acid to give the title compound as colourless crystals, m.p. 175°C (dec).

### EXAMPLE 74

### Trans-2-carboxy-4-(4-aminomethylphenyl)methyl-carbonylamino-5,7-dichloro-1,2,3,4-tetrahydroquinoline hydrochloride

This compound was prepared by the route outlined in Example 70a and 70b using 4-aminomethylphenylacetic acid in place of 4-aminobutyric acid to give the title compound as colourless crystals, m.p. 248-250°.

### EXAMPLE 75

### Trans-2-methoxycarbonyl-4-(4-aminomethylphenyl)methylcarbonylamino-5,7-dichloro-1,2,3,4-tetrahydroquinoline hydrochloride

This compound was prepared by the method given in Example 52 using trans-2-carboxy-4(-4-aminomethylphenyl)-methylcarbonylamino-5,7-dichloro-1,2,3,4-tetrahydroquinoline hydrochloride (Example 74) in place of trans-2-carboxy-4(3-aminomethylphenyl)carbonylamino-5,7-dichloro-1,2,3,4-tetrahydroquinoline hydrochloride to give the title compound as colourless crystals, m.p. 172-174°C.

### EXAMPLE 76

### Trans-4-(4-aminoethylphenyl)methylcarbonylamino-2-carboxy-5,7-dichloro-1,2,3,4-tetrahydroquinoline hydrochloride

### Step a Methyl-4-tertiary-butyloxycarbonylamino ethylphenylacetate

To a solution of 4-cyanomethylphenylacetic acid (1.0g, 5.7mmol) in a mixture of water (150ml), 15% aqueous potassium hydroxide (20ml) and concentrated aqueous ammonia (50ml) was added a slurry of Raney nickel in water (approx. 0.5g) and the resulting mixture was hydrogenated at room temperature under 50 psi for 2.25h. The mixture was then filtered and evaporated to dryness in vacuo. The residue was then dissolved in water (100ml), acidified with concentrated hydrochloric acid, washed with ethyl acetate (2 x 75ml) and the aqueous phase was freeze- dried to give a solid. To a solution of this solid in a mixture of water (40ml) and 1,4-dioxan (40ml) was added anhydrous sodium carbonate (2.01g, 19.9mmol) and di-tertiary-butyldicarbonate (2.62g, 12.0mmol) and the mixture was stirred at room temperature for 2 hours. The organic solvent was then removed under vacuum and the aqueous residue was diluted with water (100ml) and washed with diethyl ether (2 x 75ml). The aqueous phase was acidified with concentrated hydrochloric acid and extracted with ethyl acetate (2 x 75ml). The combined extracts were washed with saturated brine solution (1 x 75ml), dried (MgSO₄) and evaporated to give the crude intermediate as an oil. This preparation was repeated using 1.13g of 4-cyanomethylphenylacetic acid (6.46mmol) and the resulting oil was combined with that from above. To a solution of this crude material in diethyl ether (300ml) was added a solution of diazomethane (16mmol) in diethyl ether (80ml) and the mixture was allowed to stand at room temperature for 0.25h. The reaction was then quenched with glacial acetic acid (0.5ml) and the mixture was washed with saturated sodium bicarbonate solution (3 x 100ml), saturated brine solution (1 x 100ml), dried (MgSO₄) and evaporated. The resulting solid was purified using flash chromatography (using 30% ethyl acetate in petroleum ether bp 60-80° as eluent) to give the title compound as colourless crystals (3.00g), m.p. 77-78°C.

### Step b 4-Tertiary-butyloxycarbonylaminoethylphenylacetic acid

To a solution of methyl 4-(tertiarybutyloxycarbonylaminoethyl)phenylacetate (0.500g, 1.71mmol) in a mixture of tetrahydrofuran (20ml) and water (10ml) was added aqueous lithium hydroxide (3.8ml of a 0.50M solution, 1.90mmol) and the resulting mixture was stirred at room temperature for 16h. The organic solvent was removed under vacuum and the residue was diluted with water (40ml), acidified with concentrated hydrochloric acid and extracted with ethyl acetate (2 x 30ml). The combined extracts were washed with brine (1 x 30ml), dried (MgSO₄) and evaporated to give the pure title compound as colourless crystals (0.460g), m.p. 94-95°C.

### Step c. Trans-(4-(4-tertiary-butyloxycarbonylaminoethylphenyl)methylcarbonylamino-5,7-dichloro-2-methoxycarbonyl-1,2,3,4-tetrahydroquinoline

This material was prepared using the method given for Example 7 Step a using 4-(tertiarybutyloxycarbonylaminoethyl)phenylacetic acid in place of phenylacetic acid to give the title compound as colourless crystals, m.p. 219-221°C.

### Step d. Trans-4-(4-aminoethylphenyl)methylcarbonylamino-2-carboxy-5,7-dichloro-1,2,3,4-tetrahydroquinoline hydrochloride

To a solution of trans-4(4-tertiary-butyloxycarbonyl aminoethylphenyl)methylcarbonylamino-5,7-dichloro-2-methoxycarbonyl-1,2,3,4-tetrahydroquinoline (0.095g, 0.177mmol) in a mixture of tetrahydrofuran (10ml) and water (5ml) was added aqueous lithium hydroxide (0.40ml of a 0.50M solution, 0.20mmol) and the resulting mixture was stirred at room temperature for 1h. The organic solvent was then removed under vacuum, and the residue was diluted with sodium bicarbonate solution (40ml) and washed with diethyl ether (1 x 30ml). The aqueous phase was then acidified with concentrated hydrochloric acid and extracted with ethyl acetate (2 x 40ml). The combined extracts were washed with brine (1 x 40ml), dried (MgSO₄) and evaporated to give an oil. To a solution of this oil in ethyl acetate (5ml) was added a saturated solution of hydrogen chloride in ethyl acetate (5ml) and the mixture was stirred at room temperature for 35 min. The solid was collected by filtration, washed with ethyl acetate and dried to give the title compound as colourless crystals (0.075g), m.p. 142-145°C.

### EXAMPLE 77

### Trans-4-(4-aminoethylphenyl)methylcarbonylamino-5,7-dichloro-2-methoxycarbonyl-1,2,3,4-tetrahydroquinoline hydrochloride

To a solution of trans-4(4-tertiary-butyloxycarbonylaminoethylphenyl)methylcarbonylamino-5,7-dichloro-2-methoxycarbonyl-1,2,3,4-tetrahydroquinoline (Example 76c) (0.150g, 0.28mmol) in ethyl acetate (5ml) was added a saturated solution of hydrogen chloride in ethyl acetate (5ml) and the resulting mixture was stirred at room temperature for 40 minutes. The solvent was then removed under vacuum and the residue was triturated with hot ethyl acetate. A gummy solid was collected by filtration and this was dissolved in water and freeze dried to give the title compound as a colourless solid (0.121g), m.p. decomposes above 200°C.

### EXAMPLE 78

### Trans-2-carboxy-5,7-dichloro-4-(4-N-methylaminomethylphenyl)methyl-carbonylamino-1,2,3,4-tetrahydroquinoline hydrochloride

### Step a) 4-N-tertiary-butyloxycarbonyl-N-methylaminomethylphenylacetic acid

To a solution of 4-(bromomethyl)phenylacetic acid (8.58g, 37.5mmol) in anhydrous N,N-dimethylformamide (200ml) was added methylamine hydrochloride (25.3g, 375mmol) and dry triethylamine (63.0ml, 450mmol). The resulting mixture was stirred under an atmosphere of nitrogen at room temperature for 16.5h. The reaction was then concentrated in vacuo and the resulting residue was partitioned between ethyl acetate (200ml) and 1M hydrochloric acid (200ml). The aqueous layer was separated, washed with ethyl acetate (200ml) and the pH adjusted to 14 by the addition of solid sodium hydroxide. The alkaline solution was washed with diethyl ether (2 x 100ml), then reacidified with concentrated hydrochloric acid and evaporated in vacuo to give the crude amino acid hydrochloride as a mixture with inorganic material. To a solution of this solid in water (100ml) was added 1,4-dioxan (100ml), sodium carbonate (13.1g, 124mmol) and di-tertiarybutyldicarbonate (18.0g, 82mmol) and the reaction was stirred at room temperature for 3h. The organic solvent was removed under vacuum and the aqueous residue was washed with diethyl ether (2 x 75ml), acidified by the addition of concentrated hydrochloric acid and extracted with ethyl acetate (2 x 75ml). The combined extracts were washed with brine (1 x 75ml), dried (MgSO₄) and evaporated in vacuo to give the crude N-protected amino acid as an oil (0.91g) To a solution of this oil (0.91g, 3.3mmol) in ethanol (50ml) at room temperature was added sufficient of a solution of diazomethane (∼ 5mmol) in diethyl ether (∼ 90ml) to allow a yellow colour to persist. The excess diazomethane was then destroyed by the addition of glacial acetic acid (1ml) and the mixture was concentrated in vacuo. The residue was dissolved in diethyl ether (5ml), washed with saturated sodium bicarbonate solution (3 x 25ml), brine (1 x 25ml), dried (MgSO₄) and evaporated to give an oil which was purified by flash chromatography on silica gel using a gradient of 10-15% ethyl acetate in petroleum ether bp (60-80°) as eluent, to give the pure fully protected amino acid as a clear oil (0.549g). To a solution of this oil (0.532g, 1.82mmol) in a mixture of tetrahydrofuran (30ml) and water (15ml) was added aqueous lithium hydroxide (4.0ml of a 0.50M solution) and the reaction was stirred at room temperature for 3h. The organic solvent was removed under vacuum and the aqueous mixture was diluted with water (40ml), acidified with concentrated hydrochloric acid and extracted with ethyl acetate (2 x 25ml). The combined extracts were washed with brine (1 x 25ml), dried (MgSO₄) and evaporated in vacuo to give the title compound as colourless crystals (0.50g), mp 122-125°C.

### Step b) Trans-4-(4-N-tertiary-butyloxycarbonyl-N-methylaminomethylphenyl)methylcarbonylamino-5,7-dichloro-2-methoxycarbonyl-1,2,3,4-tetrahydroquinoline

This material was prepared using the method given for Example 7, Step a) using 4-N-tertiary-butyloxycarbonyl-N-methylaminomethylphenylacetic acid in place of phenylacetic acid, yielding the crude product which was purified by flash chromatography on silica gel, using 45% ethyl acetate in petroleum ether bp (60-80°). Recrystallisation from ethyl acetate-petroleum ether bp (60-80°) gave the title compound as colourless crystals, mp 128-129.5°C.

### Step c) Trans-2-carboxy-5,7-dichloro-4-(4-N-methylaminomethylphenyl)methylcarbonylamino-1,2,3,4-tetrahydroquinoline hydrochloride

This material was prepared using the method given for Example 76, Step d) using trans-4-(4N-tertiary-butyloxy-carbonyl-N-methylaminomethylphenyl)methylcarbonylamino-5,7-dichloro-2- methoxycarbonyl-1,2,3,4-tetrahydroquinoline to give the title compound as colourless crystals, mp 140°C dec.

### EXAMPLE 79

### Trans-5,7-dichloro-2-methoxycarbonyl-4(4-N-methylaminomethylphenyl)methylcarbonylamino-1,2,3,4-tetrahydroquinoline hydrochloride

This material was prepared using the method given for Example 77 using trans-4-(4-N-tertiary-butyloxycarbonyl-N-methylaminomethylphenyl) methylcarbonylamino-5,7-dichloro-2-methoxycarbonyl-1,2,3,4-tetrahydroquinoline in place of trans-4-(4-tertiary-butyloxycarbonylaminoethylphenyl)-methylcarbonylamino-5,7-dichloro-2-methoxycarbonyl-1,2,3,4-tetrahydroquinoline to give the title compound as colourless crystals, mp. 140°C dec.

### EXAMPLE 80

### Trans-5,7-dichloro-4-(4-N,N-dimethylamino-methylphenyl) methylcarbonylamino-2-methoxycarbonyl-1,2,3,4-tetrahydroquinoline

### Step a) Ethyl 4-(tertiary-butyloxycarbonylaminomethyl)phenylacetate

To a refluxing suspension of 4-(bromomethyl)phenylacetic acid (20.63g, 90.1mmol) in chloroform (160ml) was slowly added a solution of hexamethylenetetramine (13.4g, 95.5mmol) in chloroform (110ml). The resulting mixture was heated at reflux under an atmosphere of nitrogen for 2.5h, then cooled to room temperature and the colourless solid which had formed was collected by filtration. A solution of this solid in a mixture of concentrated hydrochloric acid (50ml) and ethanol (140ml) was heated at reflux for 1h, then cooled, filtered and the filtrate was concentrated in vacuo to yield an oily solid. This solid was dissolved in ethanol (300ml) and the solution was saturated with hydrogen chloride gas with cooling in ice then stirred at room temperature for 18h. After evaporation of the solvent under vacuum, the residue was dissolved in water (500ml) and washed with ethyl acetate (2 x 200ml). The aqueous phase was separated, basified with solid sodium bicarbonate, extracted with ethyl acetate (3 x 200ml), then basified further with solid sodium carbonate and again extracted with ethyl acetate (5 x 100ml). The combined extracts were washed with brine (1 x 300ml), dried (MgSO₄) and evaporated to give the crude amine ester as an oil (13.5g). To a solution of this oil (13.5g) in dichloromethane (350ml) was added di-tertiary-butyldicarbonate (21.0g, 96mmol) and the resulting mixture was stirred at room temperature under an atmosphere of nitrogen for 4h. To the reaction was then added N,N-dimethylethylenediamine (8.4ml, 76mmol) and stirring was continued for 0.5h. The mixture ws then washed successively with 1M aqueous citric acid (2 x 700ml), saturated aqueous sodium bicarbonate solution (2 x 700ml), brine (1 x 200ml) then dried (MgSO₄) and evaporated to give a brown oil. This was purified by flash chromatography on silica gel using 20% ethyl acetate in petroleum ether bp (60-80°) as eluent to yield the title compound as a colourless oil (13.46g).

### Step b) Ethyl 4-(aminomethyl)phenylacetate hydrochloride

To a solution of ethyl 4-(tertiary-butyloxycarbonylaminomethyl)phenylacetate (1.50g, 5.1mmol) in ethyl acetate (10ml) was added a saturated solution of hydrogen chloride in ethyl acetate (20ml) and the resulting mixture was stirred at room temperature in a stoppered flask for 2h. The reaction was then concentrated in vacuo and the solid residue was triturated with hot ethyl acetate and collected by filtration to give the title compound as colourless crystals (1.11g), mp 184-186°C.

### Step c) Ethyl 4-(N,N-dimethylaminomethyl)phenylacetate

A solution of ethyl 4-(aminomethyl)phenylacetate hydrochloride (2.00g, 8.71mmol) in water (100ml) was basified by the addition of sodium carbonate, then saturated with sodium chloride and extracted with ethyl acetate (3 x 50ml). The combined extracts were dried (MgSO₄) and evaporated to give an oil. To a solution of this oil in acetonitrile (50ml) was added aqueous formaldehyde (3.5ml of a 37% solution, 43mmol) and sodium cyanoborohydride (approx. 0.9g, 14mmol) and the resulting cloudy mixture was stirred at room temperature for 0.5h. Sufficient glacial acetic acid to give a pH of 5 (when tested on wet indicator paper) was then added and stirring was continued for 1.2h. The reaction was quenched with dilute aqueous sodium carbonate (50ml), and the organic solvent was removed under vacuum to give an aqueous residue which was extracted with ethyl acetate (3 x 20ml). The combined extracts were concentrated in vacuo and partitioned between 1M hydrochloric acid (75ml) and diethyl ether (30ml). The aqueous phase was separated, washed with ethyl acetate (2 x 30ml) then basified with sodium carbonate, saturated with sodium chloride and extracted with ethyl acetate (3 x 20ml). The combined extracts were washed with saturated brine (30ml), dried (MgSO₄) and evaporated to give the title compound as an oil (0.545g).

### Step d) 4-(N,N-dimethylaminomethyl)phenylacetic acid

To a solution of ethyl 4-(N,N-dimethylaminomethyl) phenylacetate (0.470g, 2.13mmol) in a mixture of tetrahydrofuran (15ml) and water (5ml) was added aqueous lithium hydroxide (4.7ml, 2.34mmol) and the resulting mixture was stirred at room temperature for 4h. The organic solvent was removed under vacuum and the aqueous residue was freeze dried to give the crude lithium salt. This material was applied to an ion-exchange resin (Dowex 50W x 8), eluted with dilute aqueous ammonia and freeze dried to give the title compound as a non-crystalline solid (0.368g).

### Step e) Trans-5,7-dichloro-4-(4-N,N-dimethylaminomethylphenyl) methylcarbonylamino-2-methoxycarbonyl-1,2,3,4-tetrahydroquinoline

A solution of trans-4-amino-5,7-dichloro-2-methoxycarbonyl-1,2,3,4-tetrahydroquinoline hydrochloride (0.35g, 1.12mmol) in water (50ml) was basified with sodium carbonate, saturated with sodium chloride and extracted with ethyl acetate (2 x 30ml). The combined extracts were washed with brine (1 x 30ml), dried (MgSO₄) and evaporated to give the free base as a solid (0.28g). To a solution of this material (0.28g, 1.02mmol) in anhydrous tetrahydrofuran (30ml) were added 4-N,N-dimethylaminomethylphenylacetic acid (0.216g, 1.12mmol), 1-hydroxybenzotriazole (0.151g, 1.12mmol) and N,N'-dicyclohexylcarbodiimide (0.231g, 1.12mmol) and the resulting mixture was stirred at room temperature under an atmosphere of nitrogen for 18h. The reaction was concentrated in vacuo and the residue was suspended in ethyl acetate (50ml) and extracted with 1M hydrochloric acid (4 x 20ml). The combined aqueous extracts were washed with ethyl acetate (1 x 20ml), filtered, basified with sodium carbonate, saturated with sodium chloride and extracted with ethyl acetate (3 x 20ml). The combined extracts were washed with brine (20ml), dried (MgSO₄) and evaporated under vacuum to give the crude product which was purified by flash chromatography on silica gel using dichloromethane containing 8% methanol and 0.1% ammonia as eluent, followed by recrystallisation from ethyl acetate/petroleum ether bp (60-80°), to give the title compound as colourless crystals (0.261g), mp 182-183°C.

### EXAMPLE 81

### Trans-2-carboxy-5,7-dichloro-4(4-N,N-dimethylaminomethylphenyl)methylcarbonylamino-1,2,3,4-tetrahydroquinoline

To a solution of trans-5,7-dichloro-4(4-N,N-dimethylaminomethylphenyl)methylcarbonylamino-2-methoxycarbonyl- 1,2,3,4-tetrahydroquinoline (Example 80, 0.120g, 0.267mmol) in a mixture of tetrahydrofuran (10ml) and water (5ml) was added aqueous lithium hydroxide (0.58ml of a 0.50M solution, 0.29mmol) and the resulting mixture was stirred at room temperature for 2h. The reaction was then evaporated to dryness in vacuo to give a solid which was applied to an ion-exchange resin (Dowex 50W x 8), and eluted with dilute aqueous ammonia. The solution was then freeze dried to give the crude product which was triturated with hot methanol and collected by filtration to yield the title compound as a colourless solid (0.032g), mp 190-193°C.

### EXAMPLE 82

### Trans-2-carboxy-4-(4-(3-aminoprop-2-ynyl)phenyl)methylcarbonylamino-5,7-dichloro-1,2,3,4-tetrahydroquinoline hydrochloride

### a) 4-(3-Hydroxyprop-2-ynyl)phenylacetic acid methyl ester

4-Bromophenylacetic acid methyl ester (23g, 0.1mol) was dissolved in dry triethylamine (80ml) with propynol (5.82ml, 0.1mol). After the addition of cuprous iodide (0.3g) and bis (triphenylphosphine)palladium (II) chloride (1g) the reaction mixture was heated at 65°C under nitrogen for 14h. The solvent was evaporated under vacuum and the residue was dissolved in ethyl acetate (500ml) and washed successively with 0.5M citric acid solution (2 x 200ml), saturated sodium hydrogen carbonate solution (2 x 200ml) and brine (1 x 150ml). The organic solution was dried (Na₂SO₄), filtered and concentrated in vacuo to give an oil which was purified by chromatography on silica gel using 20% ethyl acetate in hexane as eluent to give the required compound (5.6g) as an oil.

### b) 4-(3-Chloroprop-2-ynyl)phenylacetic acid methyl ester

To a solution of 4-(3-Hydroxyprop-2-ynyl)phenylacetic acid methyl ester (5.5g, 0.027M) in tetrahydrofuran (110ml) and carbon tetrachloride (110ml) was added triphenylphosphine (7.81g, 1.1 molar equivalents). The reaction mixture was heated at 60°C for 3h. After cooling, the solid obtained was removed by filtration and the mother liquors were concentrated under vacuum. The residue was purified by chromatography on silica gel using 50% diethyl ether in hexane as eluent to give the required product (5.35g) as a colourless oil.

### c) 4-(3-Azidoprop-2-ynyl)phenylacetic acid methyl ester

4-(3-Chloroprop-2-ynyl)phenylacetic acid methyl ester (5.3g, 0.023M) was dissolved in dry dimethylformamide (30ml) with sodium azide (1.7g, 1.1 molar equivalents) and stirred at room temperature for 14h. The reaction mixture was diluted with water (200ml) and washed with dichloromethane (2 x 150ml). The combined organic layers were washed with water (2 x 100ml) and brine (1 x 100ml) then dried (Na₂SO₄), filtered and concentrated in vacuo to give a residue which was purified by passage down a silica gel column, using as eluents 20% dichloromethane in hexane and finally neat dichloromethane, to give the required product (5.3g) as a colourless oil.

### d) 4-(3-(Tertiary-butyloxycarbonylamino)-prop-2-ynyl)phenylacetic acid methyl ester

4-(3-Azidoprop-2-ynyl)phenylacetic acid methyl ester (2.6g, 0.0113mol) was dissolved in methanol (60ml) in the presence of triethylamine (7.86ml, 5 molar equivalents) and propane 1,3-dithiol (5.67ml, 5 molar equivalents). After stirring at room temperature for 14h the reaction mixture was concentrated under vacuum and the residue was purified by silica gel chromatography, using initially neat dichloromethane then finally 5% methanol in dichloromethane. After evaporation of the solvents the residue obtained was dissolved in dichloromethane (100ml) and triethylamine (3.13ml, 2 molar equivalents) and ditertiary-butyldicarbonate (3.0g, 1.2 molar equivalents) were added. After stirring at room temperature for 14h the solvent was removed in vacuo and the oil obtained was purified by chromatography on silica gel using dichloromethane as eluent to give the required product as a viscous oil (1.95g).

### e) 4-(3-(Tertiary-butyloxycarbonylamino)prop-2-ynyl)phenylacetic acid

4-(3-Tertiarybutyloxycarbonylamino)-prop-2-ynylphenylacetic acid methyl ester (1.9g) was dissolved in 50% aqueous methanol (100ml) with sodium hydroxide (1g) and the solution stirred at room temperature for 14h. After this time the methanol was removed under vacuum and the aqueous residue was treated with 1N hydrochloric acid until a pH of 1 was obtained. The precipitate produced was extracted into ethyl acetate (2 x 200ml), the organic layer was dried (Na₂SO₄), filtered and concentrated in vacuo to give the required product (1.7g) as a white solid.

### f) Trans-2-carboxy-4-(4-(3-aminoprop-2-ynyl)phenyl) methylcarbonylamino-5,7-dichloro-1,2,3,4-tetrahydroquinoline hydrochloride

This compound was prepared by the route outlined in Example 70b using 4-(3-(tertiarybutyloxycarbonylamino)prop-2-ynyl)phenylacetic acid in place of 4-(tertiarybutyloxy-carbonylamino)butanoic acid to give the title compound as colourless crystals, m.p. 162-164°C.

### EXAMPLE 83

### Trans-2-methoxycarbonyl-4-(4-(3-aminoprop-2-ynyl)phenyl) methylcarbonylamino-5,7-dichloro-1,2,3,4-tetrahydroquinoline hydrochloride

This compound was prepared by the method given in Example 52 using trans-2-carboxy-4-(4-(3-aminoprop-2-ynyl)phenyl)methylcarbonylamino-5,7-dichloro-1,2,3,4- tetrahydroquinoline hydrochloride (Example 82) in place of trans-2-carboxy-4-(3-aminomethylphenyl)carbonylamino-5,7-dichloro-1,2,3,4-tetrahydroquinoline hydrochloride to give the title compound as colourless crystals, m.p. 202°C dec.

### EXAMPLE 84

### Trans-2-carboxy-4-(4-(3-aminopropyl)phenyl)methylcarbonylamino-5,7-dichloro-1,2,3,4-tetrahydroquinoline hydrochloride

### a) 4-(3-(Tertiary-butyloxycarbonylamino)propyl)-phenylacetic acid methyl ester

4-(3-Azidoprop-2-ynyl)phenylacetic acid methyl ester (Example 82c) (2.6g, 0.0113m) was dissolved in ethyl acetate (100ml) with ditertiarybutyl dicarbonate (5.9g, 2.4 molar equivalents) and after the addition of 10% palladium on carbon catalyst (0.25g) the reaction mixture was shaken under a 50 p.s.i. pressure of hydrogen for 14h. The catalyst was removed by filtration and the organic solution was concentrated in vacuo to leave a residue which was purified by chromatography on silica gel, using 10% ethyl acetate in dichloromethane as eluent, to give the required product (2.07g) as an oil.

### b) 4-(3-(Tertiarybutyloxycarbonylamino)propyl)phenylacetic acid

4-(3-(Tertiarybutoxycarbonylamino)propyl)phenylacetic acid methyl ester (2.0g) was dissolved in 50% aqueous methanol (100ml) with sodium hydroxide (1g) and stirred at room temperature for 14h. The methanol was removed under vacuum and the residual aqueous solution was acidified to pH1 using 1N hydrochloric acid, and the resulting precipitate extracted into ethyl acetate (2 x 100ml). The combined organic extracts were dried (Na₂SO₄), filtered and concentrated under vacuum to give the required product as a white solid (1.82g).

### c) Trans-2-carboxy-4-(4-(3-aminopropyl)phenyl)methylcarbonylamino-5,7-dichloro-1,2,3,4-tetrahydroquinoline hydrochloride

This compound was prepared by the route outlined in Example 70b using 4-(3-(tertiarybutyloxycarbonylamino)-propyl)phenylacetic acid in place of 4-(tertiarybutyloxy-carbonylamino)butanoic acid to give the title compound as colourless crystals, m.p. 161-164°C.

### EXAMPLE 85

### Trans-4-methoxycarbonyl-4-(4-(3-aminopropyl)phenyl)methylcarbonylamino-5,7-dichloro-1,2,3,4-tetrahydroquinoline hydrochloride

This compound was prepared by the method described in Example 52 using trans-2-carboxy-4-(4-(3-aminopropyl)phenyl)methylcarbonylamino-1,2,3,4-tetrahydroquinoline hydrochloride in place of trans-2-carboxy-4-(3-aminomethyl-phenyl)carbonylamino-5,7-dichloro-1,2,3,4-tetrahydroquinoline hydrochloride to give the title compound as colourless crystals, m.p. 140°C dec.

### EXAMPLE 86

### Trans-2-carboxy-4(4-(4-aminobut-2-ynyl)phenyl) methylcarbonylamino-5,7-dichloro-1,2,3,4-tetrahydroquinoline hydrochloride

This compound was prepared by the methods given in Example 82a), b), c), d), and e) and Example 70b) using but-3-yn-l-ol in place of propynol (in Example 82a) to give the title compound as colourless crystals, m.p. 162°C dec.

### EXAMPLE 87

### Trans-2-methoxycarbonyl-4(4-(4-aminobut-2-ynyl)phenyl) methylcarbonylamino-5,7-dichloro-1,2,3,4-tetrahydroquinoline hydrochloride

This compound was prepared by the method given in Example 52 using trans-2-carboxy-4-(4-(4-aminobut-2-ynyl)phenyl)methylcarbonylamino-5,7-dichloro-1,2,3,4-tetrahydroquinoline hydrochloride in place of trans-2-carboxy-4-(3-aminomethylphenyl)carbonylamino-5,7-dichloro-1,2,3,4-tetrahydroquinoline hydrochloride to give the title compound as colourless crystals, m.p. 162°C dec.

### EXAMPLE 88

### Trans-2-carboxy-4(4-(4-aminobutyl)phenyl)methyl-carbonylamino-5,7-dichloro-1,2,3,4-tetrahydroquinoline hydrochloride

This compound was prepared by the methods given in Examples 82a), b), c), 84a), b) and 70b) using but-3-yn-1-ol in place of propynol (in Example 82a) to give the title compound as colourless crystals, m.p. 110°C dec.

### EXAMPLE 89

### Trans-2-methoxycarbonyl-4-(4-(4-aminobutyl)phenyl)methylcarbonylamino-5,7-dichloro-1,2,3,4-tetrahydroquinoline hydrochloride

This compound was prepared by the method given in Example 52 using trans-2-carboxy-4-(4-(4-aminobutyl)phenyl)methylcarbonylamino-5,7-dichloro-1,2,3,4-tetrahydroquinoline hydrochloride in place of trans-2-carboxy-4-(3-aminomethylphenyl)carbonylamino-5,7-dichloro-1,2,3,4-tetrahydroquinoline hydrochloride to give the title compound as colourless crystals, m.p. 138-140°C.

### EXAMPLE 90

### Trans-4-(3-aminomethylphenyl)methylcarbonylamino-2-carboxy-5,7-dichloro-1,2,3,4-tetrahydroquinoline hydrochloride

### Step a) 3-Bromomethylphenyl acetic acid methyl ester

A solution of 3-tolylacetic acid (5g, 33mmol) and N-bromosuccinimide (5.9g, 33mmol) in carbon tetrachloride (100ml) was refluxed for 3 hours then cooled and filtered. The solvent was removed under vacuum to yield a crude product to which was added methanol (250ml) which had been saturated with hydrogen chloride (250ml) and this mixture was stirred at room temperature for 4 hours. The solvent was removed under vacuum and the crude residue was purified using flash chromatography (using ethyl acetate in hexane as eluent) to yield the title compound (7.3g) as a yellow oil.

### Step b) 3-Azidomethylphenyl acetic acid methyl ester

To a solution of 3-bromomethylphenyl acetic acid methyl ester (7.3g, 30mmol) in N,N-dimethylformamide (100ml) was added sodium azide (2.53, 33mmol) and the solution was stirred at room temperature for 18 hours. The reaction mixture was poured into water (600ml), extracted into ethyl acetate (3 x 100ml) and the combined extracts were washed with water (2 x 100ml) and brine solution (2 x 100ml), dried (MgSO₄) and evaporated to give a crude product, which was purified by flash chromatography (using ethyl acetate in hexane as eluent) to give the title compound as an oil (4.2g).

### Step c) 3-(Tertiarybutyloxycarbonylaminomethyl)phenyl acid methyl ester

This material was prepared using the method given for Example 84 step a) using 3-azidomethylphenyl acetic acid methyl ester in place of 4-(3-azidoprop-2-ynyl)phenylacetic acid methyl ester to give the title compound as an oil.

### Step d) 3-(Tertiarybutyloxycarbonylaminomethyl)phenyl acetic acid

This material was prepared using the method given for Example 76 step b) using methyl 3-(tertiarybutyloxycarbonylaminomethyl)phenylacetate in place of methyl 4-(tertiarybutyloxocarbonylaminoethyl)phenylacetate to give the title compound as a colourless oil.

### Step e) Trans-4-(3-tertiary-butyloxycarbonylaminomethyl)phenylmethylcarbonylamino-5,7-dichloro-2-methoxycarbonyl-1,2,3,4-tetrahydroquinoline

This material was prepared using the method given for Example 7 step a) using 3-(tertiarybutyloxycarbonyl-aminomethyl)phenyl acetic acid in place of phenylacetic acid to give the title compound as colourless crystals, m.p.162-163.4°C.

### Step f) Trans-4-(3-aminomethylphenyl)methyl-carbonylamino-2-carboxy-5,7-dichloro-1,2,3,4-tetrahydroquinoline hydrochloride

This material was prepared using the method given in Example 76 step d) using trans-4-(3-tertiary-butyloxy-carbonylaminomethyl) phenylmethylcarbonylamino-5,7-dichloro-2-methoxycarbonyl-1,2,3,4-tetrahydroquinoline in place of trans-4-(4-tertiary-butyloxycarbonylaminoethylphenyl)-methylcarbonylamino-5,7-dichloro-2-methoxycarbonyl-1,2,3,4-tetrahydroquinoline to give the title compound as colourless crystals, m.p. 163.7-165°C dec.

### EXAMPLE 91

### Trans-4-(3-aminomethylphenyl)methylcarbonylamino-5,7-dichloro-2-methoxycarbonyl-1,2,3,4-tetrahydroquinoline hydrochloride

This compound was prepared by the method given in Example 77 using trans-4-(3-tertiary-butyloxycarbonylaminomethyl)phenylmethylcarbonylamino-5,7-dichloro-2-methoxycarbonyl-1,2,3,4-tetrahydroquinoline (Example 90e) in place of trans-4(4-tertiary-butyloxycarbonylamino-ethylphenyl) methylcarbonylamino-5,7-dichloro-2-methoxycarbonyl-1,2,3,4-tetrahydroquinoline to give the title compound as a colourless solid; m.p. 161-162°C.

### EXAMPLE 92

### Trans-4-(2-aminomethylphenyl)methylcarbonylamino-2-carboxy-5,7-dichloro-1,2,3,4-tetrahydroquinoline hydrochloride

### Step a) 2-(Azidomethyl)phenyl acetic acid

This material was prepared using the method given for Example 90 steps a), b) and d) using 2-tolyacetic acid in place of 3-tolyacetic acid to give the title compound as a colourless oil.

### Step b) Trans-4-(2-azidomethylphenyl)methyl-carbonylamino-5,7-dichloro-2-methoxycarbonyl-1,2,3,4-tetrahydroquinoline

This material was prepared using the method given for Example 7 step a) using 2-(azidomethyl)phenylacetic acid in place of phenylacetic acid to give the title compound as colourless crystals, m.p. 216-217°C.

### Step c) Trans-4-(2-tertiary-butyloxycarbonylamino-methyl) phenylmethylcarbonylamino-5,7-dichloro-2-methoxycarbonyl-1,2,3,4-tetrahydroquinoline

To a solution of trans-4-(2-azidomethylphenyl)methylcarbonylamino-5,7-dichloro-2-methoxycarbonyl-1,2,3,4-tetrahydroquinoline (0.3g, 0.67mmol) in ethyl acetate (30ml) was added ditertiary-butyldicarbonate (0.22g, 1.0mmol) and 10% palladium on carbon and this mixture was hydrogenated under 50 psi pressure for 18 hours. The mixture was filtered and the solvent was removed under vacuum to yield the crude product, which was purified by flash chromatography (using ethyl acetate in hexane as eluent) followed by recrystallisation from ethyl acetate/hexane to give the title compound, m.p. 224-225°C.

### Step d) Trans-4-(2-aminomethylphenyl)methyl-carbonylamino-2-carboxy-5,7-dichloro-1,2,3,4-tetrahydroquinoline hydrochloride

This material was prepared using the method given for Example 76 step d) using trans-4-(2-tertiarybutyloxy-carbonylaminomethyl) phenylmethylcarbonylamino-5,7-dichloro-2-methoxycarbonyl-1,2,3,4-tetrahydroquinoline in place of trans-4-(4-tertiary-butyoxycarbonylaminoethylphenyl)- methylcarbonylamino-5,7-dichloro-2-methoxycarbonyl-1,2,3,4-tetrahydroquinoline to give the title compound as colourless crystals, m.p. 182-185°C dec.

### EXAMPLE 93

### Trans-4-(2-aminomethylphenyl)methylcarbonylamino-5,7-dichloro-2-methoxycarbonyl-1,2,3,4-tetrahydroquinoline hydrochloride

This material was prepared by the method given in Example 77 using trans-4-(2-tertiary-butyloxycarbonylaminomethyl)phenylmethylcarbonylamino-5,7-dichloro-2-methoxycarbonyl-1,2,3,4-tetrahydroquinoline in place of trans-4-(4-tertiary-butyloxycarbonylamino-ethylphenyl)methylcarbonylamino-5,7-dichloro-2-methoxycarbonyl-1,2,3,4-tetrahydroquinoline to give the title compound as a colourless solid, m.p. 169-172°C dec.

### EXAMPLE 94

### Trans-4-(2-(4-aminomethylphenyl)ethyl)carbonylamino-2-carboxy-5,7-dichloro-1,2,3,4-tetrahydroquinoline hydrochloride

### Step a) 3-(4-Cyanophenyl)propenoic acid methyl ester

To a solution of 4-cyanobenzaldehyde (10g) in toluene (100ml) was added methyl(triphenylphosphoranylidine)-acetate (30.6g) and the solution was heated to reflux for four hours, then cooled to room temperature. The solvent was removed under vacuum and the crude product was purified by flash chromatography (using ethyl acetate in hexane as eluent) to yield the title compound (11.5g).

### Step b) Methyl-4-tertiarybutyloxycarbonylaminomethylphenylproponate

To a solution of 3-(4-cyanophenyl)propenoic acid methyl ester (10g) in ethanol (300ml) was added 1M hydrochloric acid (30ml) followed by 10% palladium on carbon (2g) and the resulting mixture was hydrogenated at room temperature under 50 psi for 18hr. The mixture was filtered and the solvent was removed under vacuum. To the crude residue was added dichloromethane (300ml) and ditertiarybutyldicarbonate (17.3g, 80.5mmol), followed by the dropwise addition of triethylamine (15ml, 204mmol) and the mixture was stirred at room temperature for 4 hours. The reaction mixture was washed with water (2 x 100ml), aqueous citric acid (2 x 100ml, 1M), saturated sodium bicarbonate solution (3 x 100ml) and brine solution (1 x 100ml), dried (MgSO₄) and evaporated. The crude residue was purified by flash chromatography (using ethyl acetate in hexane as eluent to give the title compound (2.5g).

### Step c) 4-Tertiarybutyloxycarbonylaminomethylphenyl propionic acid

This material was prepared using the method given for Example 76 step b) using methyl-4-tertiary-butyloxocarbonylaminomethylphenylpropionate in place of methyl 4-(tertiarybutyloxycarbonylaminoethyl)-phenylacetate to give the title compound as colourless crystals, m.p. 140-141.5°C.

### Step d) Trans-4-(2-(4-tertiarybutyloxycarbonylaminomethylphenyl)ethyl)carbonylamino-5,7-dichloro-2-methoxycarbonyl- 1,2,3,4-tetrahydroquinoline

This material was prepared using the method given for Example 7 step a) using 4-tertiarybutyloxycarbonyl-aminomethylphenylpropionic acid in place of phenylacetic acid to give the title compound as colourless crystals, m.p. 193-194°C.

### Step e) Trans-4-(2-(4-aminomethylphenvl)ethyl)-carbonylamino-2-carboxy-5,7-dichloro-1,2,3,4-tetrahydroquinoline hydrochloride

This compound was prepared by the method given in Example 76 step d) using trans-4-(2-(4-tertiary-butyloxycarbonylaminomethylphenyl) ethyl)carbonylamino-5,7-dichloro-2-methoxycarbonyl-1,2,3,4-tetrahydroquinoline in place of trans-4-(4-tertiarybutyloxycarbonylaminoethylphenyl)methylcarbonylamino-5,7-dichloro-2- methoxycarbonyl-1,2,3,4-tetrahydroquinoline to give the title compound as colourless crystals, m.p. 156-159°C dec.

### EXAMPLE 95

### Trans-4-(2-(4-aminomethylphenyl)ethyl)carbonylamino-5,7-dichloro-2-methoxycarbonyl-1,2,3,4-tetrahydroquinoline hydrochloride

This compound was prepared by the method given in Example 52 using trans-4-(2-(4-aminomethylphenyl)-ethylcarbonylamino-2-carboxy-5,7-dichloro-1,2,3,4-tetrahydroquinoline hydrochloride in place of trans-2-carboxy-5,7-dichloro-4-(3-aminomethylphenyl)carbonylamino-1,2,3,4-tetrahydroquinoline hydrochloride to give the title compound as colourless crystals, m.p. 172-173.2°C.

### EXAMPLE 96

### Trans-4-(4-aminophenyl)methylcarbonylamino-5,7-dichloro-2-carboxy-1,2,3,4-tetrahydroquinoline hydrochloride

### Step a) Trans-4-(4-tertiarybutyloxycarbonylaminophenyl)methylcarbonylamino-5,7-dichloro-2-methoxycarbonyl-1,2,3,4-tetrahydroquinoline

This material was prepared using the method given for Example 7 step a) using 4-tertiarybutyloxycarbonyl-aminophenylacetic acid in place of phenylacetic acid to give title compound as colourless crystals, m.p. 236-239°C.

### Step b) Trans-4-(4-aminophenyl)methylcarbonylamino-5,7-dichloro-2-carboxy-1,2,3,4-tetrahydroquinoline hydrochloride

This compound was prepared by the method given in Example 76 step d) using trans-4-(4-tertiarybutyloxy-carbonylaminophenyl) methylcarbonylamino-5,7-dichloro-2-methoxycarbonyl-1,2,3,4-tetrahydroquinoline in place of trans-4-(4-tertiarybutyloxycarbonylaminoethylphenyl)- methylcarbonylamino-5,7-dichloro-2-methoxycarbonyl-1,2,3,4-tetrahydroquinoline to give the title compound as colourless crystals, m.p. 165-167°C.

### EXAMPLE 97

### Trans-2-carboxy-5,7-dichloro-4-(1,2,3,4-tetrahydroisoquinol-3-yl)carbonylamino-1,2,3,4-tetrahydroquinoline hydrochloride

### Step a) Trans-5,7-dichloro-2-methoxycarbonyl-4-(3-(N-tertiarybutyloxycarbonyl)-1,2,3,4-tetrahydroisoquinoline) carbonylamino-1,2,3,4-tetrahydroquinoline

This material was prepared using the method given for Example 7 step a) using 3-(N-tertiarybutyloxycarbonyl)-1,2,3,4-tetrahydroisoquinoline carboxylic acid in place of phenylacetic acid to give the title compound as colourless crystals; m.p. 244-246°C.

### Step b) Trans-2-carboxy-5,7-dichloro-4-(3-(N-tertiarybutyloxycarbonyl)1,2,3,4-tetrahydroisoquinoline)carbonylamino-1,2,3,4-tetrahydroquinoline

This material was prepared using the method given for Example 76 step d) using trans-5,7-dichloro-2-methoxy-carbonyl-4-(3-N-tertiarybutyloxycarbonyl)-1,2,3,4-tetrahydroisoquinoline)carbonyamino-1,2,3,4-tetrahydro-quinoline in place of trans-4-(4-tertiarybutyloxycarbonyl-aminoethylphenyl)methylcarbonylamino-5,7-dichloro-2-methoxycarbonyl-1,2,3,4-tetrahydroquinoline to give the title compound as colourless crystals; m.p. 212-214°C dec.

### EXAMPLE 98

### Trans-4-(2-carboxyphenyl)carbonylamino-2-carboxy-5,7-dichloro-1,2,3,4-tetrahydroquinoline

### Step a) Trans-4-(2-carboxyphenyl)carbonylamino-5,7-dichloro-2-methoxycarbonyl-1,2,3,4-tetrahydroquinoline

To a suspension of trans-4-amino-5,7-dichloro-2-methoxycarbonyl-1,2,3,4-tetrahydroquinoline (0.5g, 1.6mmol) in anhydrous tetrahydrofuran (50ml) under an atmosphere of nitrogen was added dry triethylamine (0.49ml, 3.5mmol) and the mixture stirred until dissolution was complete. To this solution was added phthalic anhydride (0.28g, 1.9mmol) and 4-dimethylaminopyridine (5mg) and the reaction mixture was stirred at room temperature for 3 hours. The mixture was then evaporated to dryness in vacuo and the residue was partitioned between ethyl acetate (100ml) and water (200ml). The organic layer was separated and washed successively with dilute hydrochloric acid (2 x 50ml, 1M), water (1 x 50ml) and brine (1 x 50ml), then dried (MgSO₄) and evaporated to give a solid which was recrystallised from ethyl acetate/ hexane to give the title compound as colourless crystals (0.45g), m.p. 241-243°C.

### Step b) Trans-4-(2-carboxyphenyl)carbonylamino-2-carboxy-5,7-dichloro-1,2,3,4-tetrahydroquinoline

This material was prepared by the method given for Example 7 step b) using trans-4-(2-carboxyphenyl)-carbonylamino-5,7-dichloro-2-methoxycarbonyl-1,2,3,4-tetrahydroquinoline in place of trans-5,7-dichloro-2- methoxycarbonyl-4-phenylmethylcarbonylamino-1,2,3,4-tetrahydroquinoline to give the title compound as colourless crystals, m.p. 195-197°C dec.

### EXAMPLE 99

### Trans-2-carboxy-5,7-dichloro-4-(4-chloro-3-nitro-phenyl) methylcarbonylamino-1,2,3,4-tetrahydroquinoline

This compound was prepared by the method given in Example 7 using 4-chloro-3-nitrophenylacetic acid in place of phenylacetic acid to give the title compound as colourless crystals, m.p. 233-235°C.

### EXAMPLE 100

### Trans-2-carboxy-5,7-dichloro-4-(4-hydroxy-3-nitro-phenyl) methylcarbonylamino-1,2,3,4-tetrahydroquinoline

This compound was prepared by the method given in Example 7 using 4-hydroxy-3-nitrophenylacetic acid in place of phenylacetic acid to give the title compound as yellow crystals, m.p. 245-247°C.

### EXAMPLE 101

### Trans-2-carboxy-5,7-dichloro-4-(diphenylmethyl-carbonyl)amino-1,2,3,4-tetrahydroquinoline

This compound was prepared by the method given in Example 7 using diphenylacetic acid in place of phenylacetic acid to give the title compound as colourless crystals, m.p. 238-239°C.

### EXAMPLE 102

### Trans-2-carboxy-5,7-dichloro-4-(1-phenylethyl-carbonyl)amino-1,2,3,4-tetrahydroquinoline (isomer A)

This compound was prepared by the method given in Example 7 using (±) 2-phenylpropionic acid in place of phenylacetic acid. The two diastereoisomers were separated by flash chromatography followed by recrystallisation as their methyl esters. The less polar isomer gave on treatment with lithium hydroxide, the title compound (isomer A) as colourless crystals, m.p. 210-212°C.

### EXAMPLE 103

### Trans-2-carboxy-5,7-dichloro-4-(1-phenylethyl-carbonyl)amino-1,2,3,4-tetrahydroquinoline (isomer B)

The more polar isomer from Example 102 on treatment with lithium hydroxide gave the title compound as colourless crystals, m.p. 248-249°C.

### EXAMPLE 104

### Trans-2-carboxy-5,7-dichloro-4-(4-acetylphenyl-methylcarbonyl) amino-1,2,3,4-tetrahydroquinoline

This compound was prepared by the method given in Example 7 using 4-acetylphenylacetic acid in place of phenylacetic acid to give the title compound as colourless crystals, m.p. 292-294°C dec.

### EXAMPLE 105

### Trans-2-carboxy-5,7-dichloro-4-(phenyloxymethyl-carbonyl)amino-1,2,3,4-tetrahydroquinoline

This compound was prepared by the method given in Example 7 using phenoxyacetic acid in place of phenylacetic acid to give the title compound as colourless crystals, m.p. 254-256°C.

### EXAMPLE 106

### Trans-2-carboxy-5,7-dichloro-4-(4-ethylphenylmethylcarbonyl)amino-1,2,3,4-tetrahydroquinoline

This compound was prepared by the method given in Example 7 using 4-ethylphenylacetic acid in place of phenylacetic acid to give the title compound as colourless crystals, m.p. 218-220°C.

### EXAMPLE 107

### Trans-2-carboxy-5,7-dichloro-4-(4-hydroxyphenylmethyl-carbonyl) amino-1,2,3,4-tetrahydroquinoline

This compound was prepared by the method given in Example 7 using 4-hydroxyphenylacetic acid in place of phenylacetic acid to give the title compound as colourless crystals, m.p. 259-261°C dec.

### EXAMPLE 108

### Trans-2-carboxy-5,7-dichloro-4-(4-acetamidophenylmethyl carbonyl) amino-1,2,3,4-tetrahydroquinoline

This compound was prepared by the method given in Example 7 using 4-acetylaminophenylacetic acid in place of phenylacetic acid to give the title compound as colourless crystals, m.p. 279-281°C.

### EXAMPLE 109

### Trans-2-carboxy-5,7-dichloro-4-(1-naphthylamino) carbonylamino-1,2,3,4-tetrahydroquinoline

This compound was prepared by the method given in Example 8 using 1-naphthyl isocyanate in place of phenyl isocyanate to give the title compound as colourless crystals, m.p. 241-242°C.

### EXAMPLE 110

### Trans-2-carboxy-5,7-dichloro-4-(cyclohexyl)-aminocarbonylamino-1,2,3,4-tetrahydroquinoline

This compound was prepared by the method given in Example 8 using cyclohexylisocyanate in place of phenyl isocyanate to give the title compound as colourless crystals, m.p. 217-219°C.

### EXAMPLE 111

### Trans-2-carboxy-5,7-dichloro-4-((N-methyl-N-4-methylphenyl)amino)carbonylamino-1,2,3,4-tetrahydroquinoline

The material was prepared using the method given for Example 48 step b) and c) using N-methyl-N-4-methylphenyl aniline in place of N-methylaniline to give the title compound as colourless crystals, m.p. 210-212°C.

### EXAMPLE 112

### Trans-2-carboxy-5,7-dichloro-4-(N,N-diphenylamino)carbonylamino-1,2,3,4-tetrahydroquinoline

### Step a) Trans-2-methoxycarboxyl-5,7-dichloro-4-(N,N-diphenylamino)carbonylamino-1,2,3,4-tetrahydroquinoline

This compound was prepared by the method given in Example 48 step b using N,N-diphenylamine in place of N-methylaniline to give the title compound as colourless crystals.

### Step b) Trans-2-carboxy-5,7-dichloro-4-(N,N-diphenylamino)carbonylamino-1,2,3,4-tetrahydroquinoline

This compound was prepared by the method given in Example 7 step b) using trans-2-methoxycarbonyl-5,7-dichloro-4-(N,N-diphenylamino)carbonylamino-1,2,3,4-tetrahydroquinoline in place of trans-5,7-dichloro-2-methoxycarbonyl-4-phenylmethylcarbonylamino-1,2,3,4-tetrahydroquinoline to give the title compound as colourless crystals, m.p. 214-216°C.

### EXAMPLE 113

### Trans-2-carboxy-5,7-dichloro-4-(4-ethylphenyl)amino-carbonylamino-1,2,3,4-tetrahydroquinoline

This compound was prepared by the methods given in Example 48 steps a-c) using 4-ethylaniline in place of 4-iodoaniline to give the title compound as colourless crystals, m.p. 195-197°C.

### EXAMPLE 114

### Trans-2-carboxy-5,7-dichloro-4-((N-ethyl-N-phenyl)amino) carbonylamino-1,2,3,4-tetrahydroquinoline

This material was prepared using the method given in Example 48 step b and c) using N-ethylaniline in place of N-methylaniline to give the title compound as colourless crystals, m.p. 227-229°C.

### EXAMPLE 115

### Trans-2-[(tertiary-butylcarbonyloxy)methyloxycarbonyl]-5,7-dichloro-4-phenylmethylcarbonylamino-1,2,3,4-tetrahydroquinoline

To a solution of trans-2-carboxy-5,7-dichloro-4-phenylmethylcarbonylamino-1,2,3,4-tetrahydroquinoline (0.06g, 0.16mmol, Example 4) in tetrahydrofuran (5ml) was added triethylamine (0.027ml, 0.19mmol) followed by a solution of iodomethylpivalate (0.06g, 0.24mmol) in tetrahydrofuran (2ml). This mixture was stirred at room temperature for 4 hours. The solvent was removed under vacuum and the residue obtained was purified by flash chromatography (using ethyl acetate in hexane as eluent) to give the title compound (0.044g), m.p. 162.4-163°C.

### EXAMPLE 116

### Trans-5,7-dichloro-2-[(N-methylaminocarbonylmethyl)oxycarbonyl]-4-phenylmethylcarbonylamino-1,2,3,4-tetrahydroquinoline

To a solution of trans-2-carboxy-5,7-dichloro-4-phenylmethylcarbonylamino-1,2,3,4-tetrahydroquinoline (0.05g, 0.13mmol, Example 4) in tetrahydroquinoline (5ml) was added triethylamine (0.055ml, 0.4mmol), 1-hydroxy-N-methylacetamide (0.018g, 0.2mmol), 4-dimethylaminopyridine (0.019g, 0.16mmol) and 1-(3-dimethylaminopropyl)-3-ethyl-carbodiimide hydrochloride (0.03g, 0.16mmol). This mixture was stirred at room temperature for 18 hours. The solvent was removed under vacuum and the residue was partitioned between ethyl acetate (50ml) and citric acid solution (1M, 100ml). The organic layer was separated and washed successively with citric acid solution (1M, 1 x 75ml), saturated sodium bicarbonate solution (2 x 75ml), saturated brine (1 x 75ml) then dried (MgSO₄) and evaporated and the residue purified by flash chromatography (using ethyl acetate in hexane as eluent) to give the title compound (0.037g), m.p. 229-231°C.

### EXAMPLE 117

### Trans-5,7-dichloro-2-[(N,N-dimethylamino)ethylaminocarbonylmethyl)oxycarbonyl]-4-phenylmethylcarbonylamino-1,2,3,4-tetrahydroquinoline hydrochloride

To a solution of trans-2-carboxy-5,7-dichloro-4 phenylmethylcarbonylamino-1,2,3,4-tetrahydroquinoline (0.1g, 0.26mmol, Example 4) in tetrahydrofuran (20ml) was added 1,1-carbonyldiimidazole (0.064g, 0.4mmol) and this mixture heated to reflux under an atmosphere of nitrogen for 3 hours. The reaction mixture was cooled to -78°C and a preformed solution of the lithium salt of 1-hydroxy-N,N-dimethylaminoethylacetamide [formed from addition of n-butylithium (0.63ml of 1.6M solution in hexane) to a solution of 1-hydroxy-N,N-dimethylaminoethylacetamide (0.170g, 1.1mmol) in tetrahydrofuran (10ml) at -78°C and warmed to room temperature then recooled back to -78°C], was added and after stirring for 3 minutes acetic acid (0.3ml, 5mmol) was added and the mixture left to warm to room temperature. The solvent was removed under vacuum and the residue was partitioned between ethyl acetate (50ml) and saturated sodium bicarbonate (100ml). The organic layer was separated and washed successively with saturated sodium bicarbonate (1 x 75ml), saturated brine (1 x 75ml), then dried (MgSO₄) and evaporated. To the residue was added hydrogen chloride in ethyl acetate (1ml, 5M), this solution was extracted with water (2 x 50ml) and the aqueous layer freeze dried to yield the title compound as a white solid (0.036g).

### EXAMPLE 118

### Trans-5,7-dichloro-2(2-N,N-diethylaminoethyl)oxycarbonyl-4-phenylmethyl-carbonylamino-1,2,3,4-tetrahydroquinoline hydrochloride

### Step a) N,N-diethylethanolamine lithium salt

To a solution of N,N-diethylethanolamine (0.265ml, 2.0mmol) in anhydrous tetrahydrofuran (10ml) cooled to -78°C under an atmosphere of nitrogen was slowly added a 1.6M solution of n-butyllithium in hexane (1.25ml, 2.0mmol). The mixture was then allowed to warm to room temperature and was used as such for step b).

### Step b) Trans-5,7-dichloro-2(2-N,N-diethylaminoethyl)oxycarbonyl-4-phenylmethylcarbonylamino-1,2,3,4-tetrahydroquinoline hydrochloride

To a solution of trans-2-carboxy-5,7-dichloro-4-phenylmethylcarbonylamino-1,2,3,4-tetrahydroquinoline (0.100g, 0.264mmol) in anhydrous tetrahydrofuran (20ml) was added carbonyldlimidazole (0.065g, 0.40mmol) and the resulting mixture was heated at reflux under an atmosphere of nitrogen for 3h. The reaction was then cooled to -78°C and a solution of N,N-diethylethanolamine lithium salt in tetrahydrofuran (from step a), 4.60ml, 0.80mmol) was added slowly. The resulting mixture was stirred at -78°C for 5 mins then quenched with glacial acetic acid (0.1ml), allowed to warm to room temperature and concentrated in vacuo. The residue was suspended in ethyl acetate (50ml), washed with saturated sodium bicarbonate (2 x 20ml), saturated brine (1 x 20ml), dried (MgSO₄) and evaporated under vacuum to give a solid which was purified by flash chromatography on silica gel, using 5% methanol in dichloromethane as eluent. To a solution of the purified material in ethyl acetate (5ml) was added a saturated solution of hydrogen chloride in ethyl acetate (1ml) and the resulting mixture was stirred at room temperature for 5 min. The solvent was removed under vacuum to give the crude hydrochloride salt as an oil which was dissolved in water (10ml), filtered and freeze dried to give the title compound as a colourless foam (0.072g).

### EXAMPLE 119

### Trans-5,7-dichloro-2-(3-N,N-dimethylaminopropyl) oxycarbonyl-4-phenylmethylcarbonylamino-1,2,3,4-tetrahydroquinoline hydrochloride

This material was prepared in the same way as Example 118 but using 3-dimethylamino-1-propanol in place of N,N-diethylethanolamine (step a) to give the title compound as a colourless foam.

### EXAMPLE 120

### Trans 2-(2-(N,N-dimethylamino)ethyl)aminocarbonyl-4-phenylmethylcarbonylamino-5,7-dichloro-1,2,3,4-tetrahydroquinoline

Trans 5,7-dichloro-2-methoxycarbonyl-4-phenylmethylcarbonylamino-1,2,3,4-tetrahydroquinoline (Example 7a) (0.3g) was dissolved in N,N-dimethylethylene-diamine (30ml) and allowed to stand at room temperature for 6h. After this time the reaction mixture was filtered and the solvent was removed under vacuum. The residue was dissolved in ethyl acetate (100ml) and washed with water (100ml) then brine (100ml). The organic solution was dried (MgSO₄), filtered and concentrated in vacuo to give a solid which was triturated with diethyl ether and collected by filtration to give the title compound (0.19g) as a colourless solid, m.p. 158-159°C.

### EXAMPLE 121

### Trans-2-(2-(N,N-dimethylamino)ethyl)aminocarbonyl-4-phenylaminocarbonylamino-5,7-dichloro-1,2,3,4-tetrahydroquinoline

This compound was prepared by the route outlined in Example 120 using trans 5,7-dichloro-2-methoxycarbonyl-4-phenylaminocarbonylamino-1,2,3,4-tetrahydroquinoline (example 8a) in place of trans-5,7-dichloro- 2-4-methoxycarbonylphenylmethylcarbonylamino-1,2,3,4-tetrahydroquinoline to give the title compound as colourless crystals m.p. 223-224°C.

### EXAMPLE 122

### Trans-4-(4-aminomethylphenyl)methylcarbonylamino-5,7-dichloro-2-(N-methylaminocarbonylmethyl)oxycarbonyl-1,2,3,4-tetrahydroquinoline hydrochloride

### Step a) Trans-4-(4-tertiary-butyloxycarbonylaminophenyl)methylcarbonylamino-5,7-dichloro-2-(N-methylaminocarbonylmethyl)oxycarbonyl-1,2,3,4-tetrahydroquinoline

This compound was prepared by the method given in Example 116 using trans-4-(4-tertiary-butyloxycarbonylaminophenyl)methylcarbonylamino-2-carboxy-5,7-dichloro-1,2,3,4-tetrahydroquinoline (Example 74) in place of trans-2-carboxy-5,7-dichloro-4-phenylmethylcarbonylamino-1,2,3,4-tetrahydroquinoline to give the title compound.

### Step b) Trans-4-(4-aminomethylphenyl)methylcarbonylamino-5,7-dichloro-2-(N-methylaminocarbonylmethyl)oxycarbonyl-1,2,3,4-tetrahydroquinoline hydrochloride

This compound was prepared by the method given in Example 77 using trans-4-(4-tertiary-butyloxycarbonylaminophenyl)methylcarbonylamino-5,7-dichloro-2-(N-methylaminocarbonylmethyl)oxycarbonyl-1,2,3,4-tetrahydroquinoline in place of trans-4-(4-tertiary-butyloxycarbonylaminoethylphenyl)methylcarbonylamino-5,7-dichloro-2-methoxycarbonyl-1,2,3,4-tetrahydroquinoline to give the title compound, m.p. 195-197°C dec.

### EXAMPLE 123

### Trans-4-(4-aminomethylphenyl)methylcarbonylamino-5,7-dichloro-2-hexyloxycarbonyl-1,2,3,4-tetrahydroquinoline hydrochloride

This compound was prepared by the methods given for Example 116 followed by Example 77 using n-hexanol in place of 1-hydroxy-N-methylacetamide to give the title compound as colourless solid, m.p. 249-251°C.

### EXAMPLE 124

### Tablet Preparation

Tablets containing 1.0, 2.0, 25.0, 26.0, 50.0 and 100.0mg, respectively, of:
Trans-2-carboxy-5,7-dichloro-4-phenylmethylcarbonylamino-1,2,3,4-tetrahydroquinoline
Trans-2-carboxy-5,7-dichloro-4-benzoylamino-1,2,3,4-tetrahydroquinoline
Trans-2-carboxy-5,7-dichloro-4-phenylaminocarbonylamino-1,2,3,4-tetrahydroquinoline
Trans-2-methoxycarbonyl-4-(4-aminomethylphenyl) methylcarbonylamino-5,7-dichloro-1,2,3,4-tetrahydroquinoline hydrochloride

| TABLE FOR DOSES CONTAINING FROM 1-25MG OF THE ACTIVE COMPOUND | | | |
|---|---|---|---|
| | Amount-mg. | | |
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

| TABLE FOR DOSES CONTAINING FROM 26-100MG OF THE ACTIVE COMPOUND | | | |
|---|---|---|---|
| | Amount-mg | | |
| Active Compound | 26.0 | 50.0 | 100.0 |
| Microcrystalline cellulose | 52.0 | 100.0 | 200.0 |
| Modified food corn starch | 2.21 | 4.25 | 8.5 |
| Magnesium stearate | 0.39 | 0.75 | 1.5 |

All of the active compound, cellulose, and a portion of the corn starch are mixed and granulated to 10% corn starch paste. The resulting granulation is sieved, dried and blended with the remainder of the corn starch and the magnesium stearate. The resulting granulation is then compressed into tablets containing 1.0mg, 2.0mg, 25.0mg, 26.0mg, 50.0mg, and 100mg of active ingredient per tablet.

## Claims

1. A compound of formula IIC or a salt thereof: 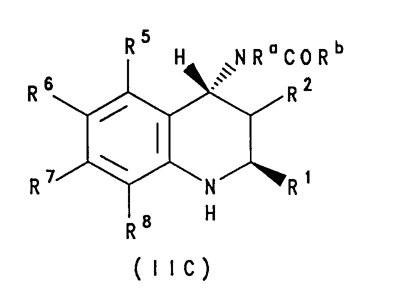 wherein
R¹ represents a carboxy group or a group which is convertible thereto in vivo;
R² represents hydrogen;
R⁵, R⁶, R⁷ and R⁸ independently represent hydrogen, halogen, cyano, trifluoromethyl, nitro, hydroxy, amino, carboxy, C₁₋₆ alkyl, C₁₋₆ alkoxy, C₁₋₆ alkylthio or C₁₋₆ alkoxycarbonyl; and
R^{a} and R^{b} independently represent hydrogen; or C₁₋₆ alkyl, C₂₋₆ alkenyl, C₃₋₇ cycloalkyl, C₃₋₇ cycloalkyl(C₁₋₆)alkyl, aryl selected from phenyl, naphthyl and fluorenyl, aryl(C₁₋₆)alkyl, C₃₋₇ heterocycloalkyl, C₃₋₇ heterocycloalkyl(C₁₋₆)alkyl, heteroaryl selected from pyridyl, quinolyl, isoquinolyl, pyridazinyl, pyrimidinyl, pyrazinyl, pyranyl, furyl, benzofuryl, thienyl, benzthienyl, indolinyl, imidazolyl, oxadiazolyl, thiadiazolyl and tetrazolyl, or heteroaryl(C₁₋₆)alkyl, any of which groups may be optionally substituted by one or more groups selected from methyl, ethyl, phenyl, chloro, aminomethyl, aminoethyl, aminopropyl, aminobutyl, methylaminomethyl, dimethylaminomethyl, aminopropynyl, aminobutynyl, hydroxy, methoxy, phenoxy, nitro, cyano, carboxy, acetyl, amino and acetylamino.

2. A compound as claimed in claim 1 wherein R^{a} is hydrogen and R^{b} represents phenyl or benzyl, optionally substituted by an aminomethyl or aminoethyl group.

3. A compound of formula IID or a salt thereof: 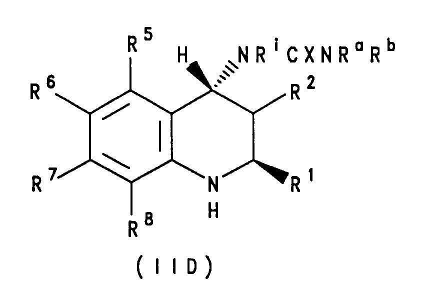 wherein R¹, R², R⁵, R⁶, R⁷ and R⁸ are as defined in claim 1; R^{a} and Rⁱ independently represent hydrogen, C₁₋₆ alkyl or aryl selected from phenyl, naphthyl and fluorenyl; R^{b} represents C₃₋₇ cycloalkyl, aryl selected from phenyl naphthyl and fluoroenyl, or aryl(C₁₋₆)alkyl, any of which groups may be optionally substituted by one or more groups selected from methyl, ethyl, chloro, iodo, methoxy and nitro; and X represents oxygen or sulphur.

4. A compound as claimed in claim 3 wherein R^{a} and Rⁱ independently represent hydrogen, methyl, ethyl or phenyl; and R^{b} represents cyclohexyl, phenyl, naphthyl or benzyl.

5. A compound as claimed in claim 4 wherein R^{a} and Rⁱ both represent hydrogen; and X represents oxygen.

6. A compound as claimed in any one of the preceding claims wherein R⁸ represents hydrogen, and R⁵, R⁶ and R⁷ independently represent hydrogen, halogen or C₁₋₆ alkyl.

7. A compound as claimed in claim 1 selected from:
4-benzoylamino-2-carboxy-5,7-dichloro-1,2,3,4-tetrahydroquinoline;
4-acetylamino-2-carboxy-5,7-dichloro-1,2,3,4-tetrahydroquinoline;
2-carboxy-5,7-dichloro-4-cyclohexylcarbonylamino-1,2,3,4-tetrahydroquinoline;
4-benzylcarbonylamino-2-carboxy-5,7-dichloro-1,2,3,4-tetrahydroquinoline;
2-carboxy-4-(4-chlorophenylcarbonylamino)-5,7-dichloro-1,2,3,4-tetrahydroquinoline;
2-carboxy-5,7-dichloro-4-(4-pyridylcarbonylamino)-1,2,3,4-tetrahydroquinoline;
2-carboxy-5,7-dichloro-4-[2-(2-aminophenethyl)]carbonylamino-1,2,3,4-tetrahydroquinoline;
2-carboxy-5,7-dichloro-4-n-propylcarbonylamino-1,2,3,4-tetrahydroquinoline;
2-carboxy-5,7-dichloro-4-(4-methoxyphenylcarbonylamino)-1,2,3,4-tetrahydroquinoline;
2-carboxy-5,7-dichloro-4-(2-methoxybenzylcarbonylamino)-1,2,3,4-tetrahydroquinoline;
2-carboxy-5,7-dichloro-4-(2-methylbenzylcarbonylamino)-1,2,3,4-tetrahydroquinoline;
2-carboxy-5,7-dichloro-4-(α-methoxybenzylcarbonylamino)-1,2,3,4-tetrahydroquinoline;
2-carboxy-5,7-dichloro-4-(2-nitrobenzylcarbonylamino)-1,2,3,4-tetrahydroquinoline;
4-(4'-biphenylcarbonylamino)-2-carboxy-5,7-dichloro-1,2,3,4-tetrahydroquinoline;
2-carboxy-5,7-dichloro-4-isopropylcarbonylamino-1,2,3,4-tetrahydroquinoline;
2-carboxy-4-(2-chlorophenylcarbonylamino)-5,7-dichloro-1,2,3,4-tetrahydroquinoline;
2-carboxy-5,7-dichloro-4-(1-naphthylcarbonylamino)-1,2,3,4-tetrahydroquinoline;
2-carboxy-5,7-dichloro-4-(2-naphthylcarbonylamino)-1,2,3,4-tetrahydroquinoline;
2-carboxy-5,7-dichloro-4-(2-furylcarbonylamino)-1,2,3,4-tetrahydroquinoline;
2-carboxy-5,7-dichloro-4-(2-methylphenylcarbonylamino)-1,2,3,4-tetrahydroquinoline;
2-carboxy-5,7-dichloro-4-(2-phenethylcarbonylamino)-1,2,3,4-tetrahydroquinoline;
2-carboxy-5,7-dichloro-4-(2-phenylethenylcarbonylamino)-1,2,3,4-tetrahydroquinoline;
2-carboxy-5,7-dichloro-4-(2-thienylmethylcarbonylamino)-1,2,3,4-tetrahydroquinoline;
2-carboxy-4-(3-chlorophenylcarbonylamino)-5,7-dichloro-1,2,3,4-tetrahydroquinoline;
2-carboxy-5,7-dichloro-4-(3-phenylpropylcarbonylamino)-1,2,3,4-tetrahydroquinoline;
2-carboxy-5,7-dichloro-4-(9-fluorenylcarbonylamino)-1,2,3,4-tetrahydroquinoline;
2-carboxy-4-cyclohexylmethylcarbonylamino-5,7-dichloro-1,2,3,4-tetrahydroquinoline;
2-carboxy-4-(2-chlorobenzylcarbonylamino)-5,7-dichloro-1,2,3,4-tetrahydroquinoline;
2-carboxy-4-(3-chlorobenzylcarbonylamino)-5,7-dichloro-1,2,3,4-tetrahydroquinoline;
2-carboxy-4-(4-chlorobenzylcarbonylamino)-5,7-dichloro-1,2,3,4-tetrahydroquinoline;
2-carboxy-5,7-dichloro-4-(4-methylbenzylcarbonylamino)-1,2,3,4-tetrahydroquinoline;
2-carboxy-5,7-dichloro-4-(4-methoxybenzylcarbonylamino)-1,2,3,4-tetrahydroquinoline;
2-carboxy-5,7-dichloro-4-(4-nitrobenzylcarbonylamino)-1,2,3,4-tetrahydroquinoline;
2-carboxy-4-(3-cyanophenylcarbonylamino)-5,7-dichloro-1,2,3,4-tetrahydroquinoline;
2-carboxy-5,7-dichloro-4-(2,3-dihydroindol-1-ylcarbonylamino)-1,2,3,4-tetrahydroquinoline;
2-carboxy-4-[2-(carboxyethyl)carbonylamino]-5,7-dichloro-1,2,3,4-tetrahydroquinoline;
4-[3-(aminomethyl)phenylcarbonylamino]-2-carboxy-5,7-dichloro-1,2,3,4-tetrahydroquinoline;
4-[3-(aminomethyl)phenylcarbonylamino]-5,7-dichloro-2-methoxycarbonyl-1,2,3,4-tetrahydroquinoline;
4-[4-(aminomethyl)phenylcarbonylamino]-2-carboxy-5,7-dichloro-1,2,3,4-tetrahydroquinoline;
4-[4-(aminomethyl)phenylcarbonylamino]-5,7-dichloro-2-methoxycarbonyl-1,2,3,4-tetrahydroquinoline;
4-[4-(2-aminoethyl)phenylcarbonylamino]-2-carboxy-5,7-dichloro-1,2,3,4-tetrahydroquinoline;
4-[4-(2-aminoethyl)phenylcarbonylamino]-5,7-dichloro-2-methoxycarbonyl-1,2,3,4-tetrahydroquinoline;
2-carboxy-5,7-dichloro-4-(3-methylbenzylcarbonylamino)-1,2,3,4-tetrahydroquinoline;
2-carboxy-5,7-dichloro-4-(3-nitrobenzylcarbonylamino)-1,2,3,4-tetrahydroquinoline;
2-carboxy-5,7-dichloro-4-(3-methoxybenzylcarbonylamino)-1,2,3,4-tetrahydroquinoline;
2-carboxy-5,7-dichloro-4-(1-naphthylmethylcarbonylamino)-1,2,3,4-tetrahydroquinoline;
2-carboxy-5,7-dichloro-4-(2-naphthylmethylcarbonylamino)-1,2,3,4-tetrahydroquinoline;
2-carboxy-5,7-dichloro-4-(3-thienylmethylcarbonylamino)-1,2,3,4-tetrahydroquinoline;
2-carboxy-5,7-dichloro-4-(2,6-dichlorobenzylcarbonylamino)-1,2,3,4-tetrahydroquinoline;
4-(3-aminopropyl)carbonylamino-2-carboxy-5,7-dichloro-1,2,3,4-tetrahydroquinoline;
4-(2-aminoethyl)carbonylamino-2-carboxy-5,7-dichloro-1,2,3,4-tetrahydroquinoline;
4-(4-aminobutyl)carbonylamino-2-carboxy-5,7-dichloro-1,2,3,4-tetrahydroquinoline;
2-carboxy-5,7-dichloro-4-(4-piperidylmethylcarbonylamino)-1,2,3,4-tetrahydroquinoline;
4-[4-(aminomethyl)benzylcarbonylamino]-2-carboxy-5,7-dichloro-1,2,3,4-tetrahydroquinoline;
4-[4-(aminomethyl)benzylcarbonylamino]-5,7-dichloro-2-methoxycarbonyl-1,2,3,4-tetrahydroquinoline;
4-[4-(2-aminoethyl)benzylcarbonylamino]-2-carboxy-5,7-dichloro-1,2,3,4-tetrahydroquinoline;
4-[4-(2-aminoethyl)benzylcarbonylamino]-5,7-dichloro-2-methoxycarbonyl-1,2,3,4-tetrahydroquinoline;
2-carboxy-5,7-dichloro-4-[4-(N-methylaminomethyl)-benzylcarbonylamino]-1,2,3,4-tetrahydroquinoline;
5,7-dichloro-2-methoxycarbonyl-4-[4-(N-methylaminomethyl)benzylcarbonylamino]-1,2,3,4-tetrahydroquinoline;
5,7-dichloro-4-[4-(N,N-dimethylaminomethyl)benzylcarbonylamino]-2-methoxycarbonyl-1,2,3,4-tetrahydroquinoline;
2-carboxy-5,7-dichloro-4-[4-(N,N-dimethylaminomethyl)benzylcarbonylamino]-1,2,3,4-tetrahydroquinoline;
4-[4-(3-aminoprop-2-ynyl)benzylcarbonylamino]-2-carboxy-5,7-dichloro-1,2,3,4-tetrahydroquinoline;
4-[4-(3-aminoprop-2-ynyl)benzylcarbonylamino]-5,7-dichloro-2-methoxycarbonyl-1,2,3,4-tetrahydroquinoline;
4-[4-(3-aminopropyl)benzylcarbonylamino]-2-carboxy-5,7-dichloro-1,2,3,4-tetrahydroquinoline;
4-[4-(3-aminopropyl)benzylcarbonylamino]-5,7-dichloro-2-methoxycarbonyl-1,2,3,4-tetrahydroquinoline;
4-[4-(4-aminobut-2-ynyl)benzylcarbonylamino]-2-carboxy-5,7-dichloro-1,2,3,4-tetrahydroquinoline;
4-[4-(4-aminobut-2-ynyl)benzylcarbonylamino]-5,7-dichloro-2-methoxycarbonyl-1,2,3,4-tetrahydroquinoline;
4-[4-(4-aminobutyl)benzylcarbonylamino]-2-carboxy-5,7-dichloro-1,2,3,4-tetrahydroquinoline;
4-[4-(4-aminobutyl)benzylcarbonylamino]-5,7-dichloro-2-methoxycarbonyl-1,2,3,4-tetrahydroquinoline;
4-[3-(aminomethyl)benzylcarbonylamino]-2-carboxy-5,7-dichloro-1,2,3,4-tetrahydroquinoline;
4-[3-(aminomethyl)benzylcarbonylamino]-5,7-dichloro-2-methoxycarbonyl-1,2,3,4-tetrahydroquinoline;
4-[2-(aminomethyl)benzylcarbonylamino]-2-carboxy-5,7-dichloro-1,2,3,4-tetrahydroquinoline;
4-[2-(aminomethyl)benzylcarbonylamino]-5,7-dichloro-2-methoxycarbonyl-1,2,3,4-tetrahydroquinoline;
4-[2-(4-(aminomethyl)phenyl)ethylcarbonylamino]-2-carboxy-5,7-dichloro-1,2,3,4-tetrahydroquinoline;
4-[2-(4-(aminomethyl)phenyl)ethylcarbonylamino]-5,7-dichloro-2-methoxycarbonyl-1,2,3,4-tetrahydroquinoline;
4-(4-aminobenzylcarbonylamino)-2-carboxy-5,7-dichloro-1,2,3,4-tetrahydroquinoline;
2-carboxy-4-(2-carboxyphenylcarbonylamino)-5,7-dichloro-1,2,3,4-tetrahydroquinoline;
2-carboxy-4-(4-chloro-3-nitrobenzylcarbonylamino)-5,7-dichloro-1,2,3,4-tetrahydroquinoline;
2-carboxy-5,7-dichloro-4-(4-hydroxy-3-nitrobenzylcarbonylamino)-1,2,3,4-tetrahydroquinoline;
2-carboxy-5,7-dichloro-4-(diphenylmethylcarbonylamino)-1,2,3,4-tetrahydroquinoline;
2-carboxy-5,7-dichloro-4-(1-phenylethyl)carbonylamino-1,2,3,4-tetrahydroquinoline;
4-(4-acetylbenzylcarbonylamino)-2-carboxy-5,7-dichloro-1,2,3,4-tetrahydroquinoline;
2-carboxy-5,7-dichloro-4-phenoxymethylcarbonylamino-1,2,3,4-tetrahydroquinoline;
2-carboxy-5,7-dichloro-4-(4-ethylbenzylcarbonylamino)-1,2,3,4-tetrahydroquinoline;
2-carboxy-5,7-dichloro-4-(4-hydroxybenzylcarbonylamino)-1,2,3,4-tetrahydroquinoline;
4-(4-acetamidobenzylcarbonylamino)-2-carboxy-5,7-dichloro-1,2,3,4-tetrahydroquinoline;
4-benzylcarbonylamino-2-(t-butylcarbonyloxy)methoxycarbonyl-5,7-dichloro-1,2,3,4-tetrahydroquinoline;
4-benzylcarbonylamino-5,7-dichloro-2-(methylaminocarbonyl)methoxycarbonyl-1,2,3,4-tetrahydroquinoline;
4-benzylcarbonylamino-5,7-dichloro-2-[2-(N,N-dimethylamino)ethylaminocarbonyl]methoxycarbonyl-1,2,3,4-tetrahydroquinoline;
4-benzylcarbonylamino-5,7-dichloro-2-[2-(N,N-dimethylamino)ethoxycarbonyl]-1,2,3,4-tetrahydroquinoline;
4-benzylcarbonylamino-5,7-dichloro-2-[3-(N,N-dimethylamino)propoxycarbonyl]-1,2,3,4-tetrahydroquinoline;
4-benzylcarbonylamino-5,7-dichloro-2-[2-(N,N-dimethylamino)ethylaminocarbonyl]-1,2,3,4-tetrahydroquinoline;
4-[4-(aminomethyl)benzylcarbonylamino]-5,7-dichloro-2-(methylaminocarbonyl)methoxycarbonyl-1,2,3,4-tetrahydroquinoline;
4-[4-(aminomethyl)benzylcarbonylamino]-5,7-dichloro-2-hexyloxycarbonyl-1,2,3,4-tetrahydroquinoline;
and salts thereof; wherein the relative orientation of the substituents at the 2- and 4-positions of the tetrahydroquinoline ring system is trans.

8. A compound as claimed in claim 3 selected from:
2-carboxy-5,7-dichloro-4-phenylaminocarbonylamino-1,2,3,4-tetrahydroquinoline;
4-benzylaminocarbonylamino-2-carboxy-5,7-dichloro-1,2,3,4-tetrahydroquinoline;
2-carboxy-5,7-dichloro-4-(2-nitrophenylaminocarbonylamino)-1,2,3,4-tetrahydroquinoline;
2-carboxy-5,7-dichloro-4-(2-methoxyphenylaminocarbonylamino)-1,2,3,4-tetrahydroquinoline;
2-carboxy-5,7-dichloro-4-(2-methylphenylaminocarbonylamino)-1,2,3,4-tetrahydroquinoline;
2-carboxy-4-(2-chlorophenylaminocarbonylamino)-5,7-dichloro-1,2,3,4-tetrahydroquinoline;
2-carboxy-4-(4-chlorophenylaminocarbonylamino)-5,7-dichloro-1,2,3,4-tetrahydroquinoline;
2-carboxy-5,7-dichloro-4-(4-methylphenylaminocarbonylamino)-1,2,3,4-tetrahydroquinoline;
2-carboxy-5,7-dichloro-4-(4-methoxyphenylaminocarbonylamino)-1,2,3,4-tetrahydroquinoline;
2-carboxy-5,7-dichloro-4-(4-nitrophenylaminocarbonylamino)-1,2,3,4-tetrahydroquinoline;
2-carboxy-5,7-dichloro-4-(4-iodophenylaminocarbonylamino)-1,2,3,4-tetrahydroquinoline;
2-carboxy-5,7-dichloro-4-[phenylaminocarbonyl(N-methyl)amino]-1,2,3,4-tetrahydroquinoline;
2-carboxy-5,7-dichloro-4-[(N-methyl-N-phenyl)aminocarbonylamino]-1,2,3,4-tetrahydroquinoline;
2-carboxy-5,7-dichloro-4-phenylaminothiocarbonylamino-1,2,3,4-tetrahydroquinoline;
2-carboxy-5,7-dichloro-4-(3-methoxyphenylaminocarbonylamino)-1,2,3,4-tetrahydroquinoline;
2-carboxy-5,7-dichloro-4-(3-methylphenylaminocarbonylamino)-1,2,3,4-tetrahydroquinoline;
2-carboxy-4-(3-chlorophenylaminocarbonylamino)-5,7-dichloro-1,2,3,4-tetrahydroquinoline;
2-carboxy-5,7-dichloro-4-(3-nitrophenylaminocarbonylamino)-1,2,3,4-tetrahydroquinoline;
2-carboxy-5,7-dichloro-4-(1-naphthylaminocarbonylamino)-1,2,3,4-tetrahydroquinoline;
2-carboxy-4-cyclohexylaminocarbonylamino-5,7-dichloro-1,2,3,4-tetrahydroquinoline;
2-carboxy-5,7-dichloro-4-[N-methyl-N-(4-methylphenyl)amino]carbonylamino-1,2,3,4-tetrahydroquinoline;
2-carboxy-5,7-dichloro-4-(N,N-diphenylamino)carbonylamino-1,2,3,4-tetrahydroquinoline;
2-carboxy-5,7-dichloro-4-(4-ethylphenyl)aminocarbonylamino-1,2,3,4-tetrahydroquinoline;
2-carboxy-5,7-dichloro-4-(N-ethyl-N-phenyl)aminocarbonylamino-1,2,3,4-tetrahydroquinoline;
5,7-dichloro-2-[2-(N,N-dimethylamino)ethylaminocarbonyl]-4-phenylaminocarbonylamino-1,2,3,4-tetrahydroquinoline;
and salts thereof; wherein the relative orientation of the substituents at the 2- and 4-positions of the tetrahydroquinoline ring system is trans.

9. Trans-2-carboxy-5,7-dichloro-4-phenylaminocarbonylamino-1,2,3,4-tetrahydroquinoline, or a pharmaceutically acceptable salt thereof.

10. A pharmaceutical composition comprising a compound as claimed in any one of the preceding claims in association with a pharmaceutically acceptable carrier.

11. The use of a compound as claimed in any one of claims 1 to 9 for the manufacture of a medicament for the treatment and/or prevention of neurodegenerative disorders.

12. A process for the preparation of a compound as claimed in any one of claims 1 to 9, which process comprises:
(A) reduction of a quinoline derivative of formula IV: 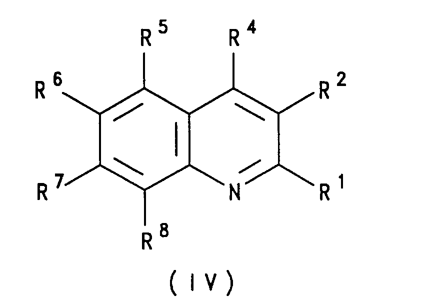 wherein R¹, R², R⁵, R⁶, R⁷ and R⁸ are as defined in claim 1, and R⁴ represents -NR^{a}COR^{b} or -NRⁱCXNR^{a}R^{b} as defined in claims 1 and 3 respectively; or
(B) reaction of a compound of formula A-COR^{b} with a compound of formula V: 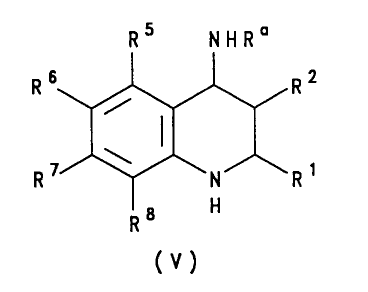 wherein the relative orientation of the R¹ and -NHR^{a} substituents is trans; R¹, R², R⁵, R⁶, R⁷, R⁸, R^{a} and R^{b} are as defined in claim 1; and A represents a leaving group; or
(C) reaction of a compound of formula X, or a protected derivative thereof, with a compound of formula XI, or a protected derivative thereof: 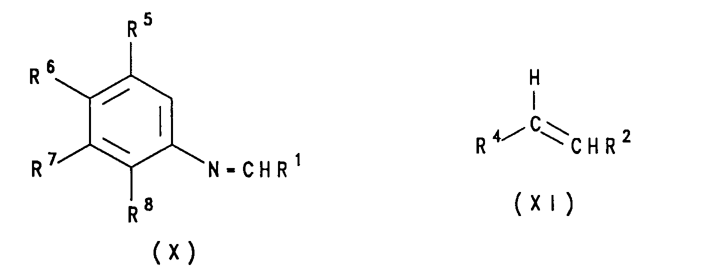 wherein R¹, R² and R⁵ to R⁸ are as defined in claim 1, and R⁴ is as defined above; followed, where necessary, by removal of the protecting groups.

## Patentansprüche

1. Eine Verbindung der Formel IIC oder ein Salz davon: 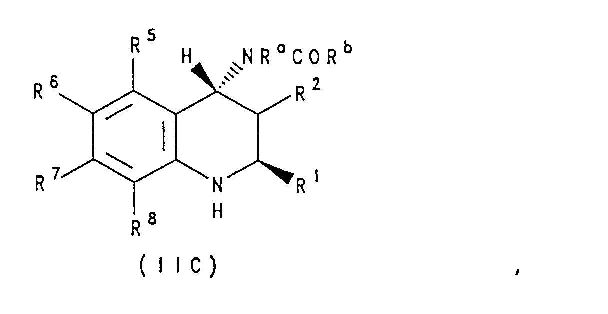 worin
R¹ eine Carboxygruppe oder eine Gruppe, die in vivo in diese umwandelbar ist, bedeutet,
R² Wasserstoff bedeutet,
R⁵, R⁶, R⁷ und R⁸ unabhängig voneinander Wasserstoff, Halogen, Cyano, Trifluormethyl, Nitro, Hydroxy, Amino, Carboxy, C₁₋₆-Alkyl, C₁₋₆-Alkoxy, C₁₋₆-Alkylthio oder C₁₋₆-Alkoxycarbonyl bedeuten, und
R^{a} und R^{b} unabhängig voneinander Wasserstoff bedeuten oder C₁₋₆-Alkyl, C₂₋₆-Alkenyl, C₃₋₇-Cycloalkyl, C₃₋₇-Cycloalkyl-(C₁₋₆)-alkyl, Aryl, ausgewählt aus Phenyl, Naphthyl und Fluorenyl, Aryl-(C₁₋₆)-alkyl, C₃₋₇-Heterocycloalkyl, C₃₋₇-Heterocycloalkyl-(C₁₋₆)alkyl, Heteroaryl, ausgewählt aus Pyridyl, Chinolyl, Isochinolyl, Pyridazinyl, Pyrimidinyl, Pyrazinyl, Pyranyl, Furyl, Benzofuryl, Thienyl, Benzthienyl, Indolinyl, Imidazolyl, Oxadiazolyl, Thiadiazolyl und Tetrazolyl, oder Heteroaryl-(C₁₋₆)-alkyl, wobei irgendwelche dieser Gruppen gegebenenfalls substituiert sein können durch eine oder mehrere Gruppen, ausgewählt aus Methyl, Ethyl, Phenyl, Chlor, Aminomethyl, Aminoethyl, Aminopropyl, Aminobutyl, Methylaminomethyl, Dimethylaminomethyl, Aminopropinyl, Aminobutinyl, Hydroxy, Methoxy, Phenoxy, Nitro, Cyano, Carboxy, Acetyl, Amino und Acetylamino.

2. Eine Verbindung wie in Anspruch 1 beansprucht, worin R^{a} Wasserstoff ist und R^{b} Phenyl oder Benzyl, gegebenenfalls durch eine Aminomethyl- oder Aminoethylgruppe substituiert, bedeutet.

3. Eine Verbindung der Formel IID oder ein Salz davon: 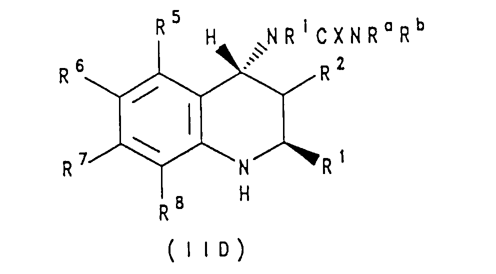 worin R¹, R², R⁵, R⁶, R⁷ und R⁸ wie in Anspruch 1 definiert sind; R^{a} und Rⁱ unabhängig voneinander Wasserstoff, C₁₋₆-Alkyl oder Aryl, ausgewählt aus Phenyl, Naphthyl und Fluorenyl, bedeuten; R^{b} C₃₋₇-Cycloalkyl, Aryl, ausgewählt aus Phenyl, Naphthyl und Fluorenyl, oder Aryl-(C₁₋₆)-alkyl bedeutet, wobei irgendwelche dieser Gruppen gegebenenfalls substituiert sein können durch eine oder mehrere Gruppen, ausgewählt aus Methyl, Ethyl, Chlor, Iod, Methoxy und Nitro; und X Sauerstoff oder Schwefel bedeutet.

4. Eine Verbindung wie in Anspruch 3 beansprucht, worin R^{a} und Rⁱ unabhängig voneinander Wasserstoff, Methyl, Ethyl oder Phenyl bedeuten, und R^{b} Cyclohexyl, Phenyl, Naphthyl oder Benzyl bedeutet.

5. Eine Verbindung wie in Anspruch 4 beansprucht, worin R^{a} und Rⁱ beide Wasserstoff bedeuten, und X Sauerstoff bedeutet.

6. Eine Verbindung wie in irgendeinem der vorhergehenden Ansprüche beansprucht, worin R⁸ Wasserstoff bedeutet, und R⁵, R⁶ und R⁷ unabhängig voneinander Wasserstoff, Halogen oder C₁₋₆-Alkyl bedeuten.

7. Eine Verbindung wie in Anspruch 1 beansprucht, ausgewählt aus:
4-Benzoylamino-2-carboxy-5,7-dichlor-1,2,3,4-tetrahydrochinolin,
4-Acetylamino-2-carboxy-5,7-dichlor-1,2,3,4-tetrahydrochinolin,
2-Carboxy-5,7-dichlor-4-cyclohexylcarbonylamino-1,2,3,4-tetrahydrochinolin,
4-Benzylcarbonylamino-2-carboxy-5,7-dichlor-1,2,3,4-tetrahydrochinolin,
2-Carboxy-4-(4-chlorphenylcarbonylamino)-5,7-dichlor-1,2,3,4-tetrahydrochinolin,
2-Carboxy-5,7-dichlor-4-(4-pyridylcarbonylamino)-1,2,3,4-tetrahydrochinolin,
2-Carboxy-5,7-dichlor-4-[2-(2-aminophenethyl)]carbonylamino-1,2,3,4-tetrahydrochinolin,
2-Carboxy-5,7-dichlor-4-n-propylcarbonylamino-1,2,3,4-tetrahydrochinolin,
2-Carboxy-5,7-dichlor-4-(4-methoxyphenylcarbonylamino)-1,2,3,4-tetrahydrochinolin,
2-Carboxy-5,7-dichlor-4-(2-methoxybenzylcarbonylamino)-1,2,3,4-tetrahydrochinolin,
2-Carboxy-5,7-dichlor-4-(2-methylbenzylcarbonylamino)-1,2,3,4-tetrahydrochinolin,
2-Carboxy-5,7-dichlor-4-(α-methoxybenzylcarbonylamino)-1,2,3,4-tetrahydrochinolin,
2-Carboxy-5,7-dichlor-4-(2-nitrobenzylcarbonylamino)-1,2,3,4-tetrahydrochinolin,
4-(4'-Biphenylcarbonylamino)-2-carboxy-5,7-dichlor-1,2,3,4-tetrahydrochinolin,
2-Carboxy-5,7-dichlor-4-isopropylcarbonylamino-1,2,3,4-tetrahydrochinolin,
2-Carboxy-4-(2-chlorphenylcarbonylamino)-5,7-dichlor-1,2,3,4-tetrahydrochinolin,
2-Carboxy-5,7-dichlor-4-(1-naphthylcarbonylamino)-1,2,3,4-tetrahydrochinolin,
2-Carboxy-5,7-dichlor-4-(2-naphthylcarbonylamino)-1,2,3,4-tetrahydrochinolin,
2-Carboxy-5,7-dichlor-4-(2-furylcarbonylamino)-1,2,3,4-tetrahydrochinolin,
2-Carboxy-5,7-dichlor-4-(2-methylphenylcarbonylamino)-1,2,3,4-tetrahydrochinolin,
2-Carboxy-5,7-dichlor-4-(2-phenethylcarbonylamino)-1,2,3,4-tetrahydrochinolin,
2-Carboxy-5,7-dichlor-4-(2-phenylethenylcarbonylamino)-1,2,3,4-tetrahydrochinolin,
2-Carboxy-5,7-dichlor-4-(2-thienylmethylcarbonylamino)-1,2,3,4-tetrahydrochinolin,
2-Carboxy-4-(3-chlorphenylcarbonylamino)-5,7-dichlor-1,2,3,4-tetrahydrochinolin,
2-Carboxy-5,7-dichlor-4-(3-phenylpropylcarbonylamino)-1,2,3,4-tetrahydrochinolin,
2-Carboxy-5,7-dichlor-4-(9-fluorenylcarbonylamino)-1,2,3,4-tetrahydrochinolin,
2-Carboxy-4-cyclohexylmethylcarbonylamino-5,7-dichlor-1,2,3,4-tetrahydrochinolin,
2-Carboxy-4-(2-chlorbenzylcarbonylamino)-5,7-dichlor-1,2,3,4-tetrahydrochinolin,
2-Carboxy-4-(3-chlorbenzylcarbonylamino)-5,7-dichlor-1,2,3,4-tetrahydrochinolin,
2-Carboxy-4-(4-chlorbenzylcarbonylamino)-5,7-dichlor-1,2,3,4-tetrahydrochinolin,
2-Carboxy-5,7-dichlor-4-(4-methylbenzylcarbonylamino)-1,2,3,4-tetrahydrochinolin,
2-Carboxy-5,7-dichlor-4-(4-methoxybenzylcarbonylamino)-1,2,3,4-tetrahydrochinolin,
2-Carboxy-5,7-dichlor-4-(4-nitrobenzylcarbonylamino)-1,2,3,4-tetrahydrochinolin,
2-Carboxy-4-(3-cyanophenylcarbonylamino)-5,7-dichlor-1,2,3,4-tetrahydrochinolin,
2-Carboxy-5,7-dichlor-4-(2,3-dihydroindol-1-ylcarbonylamino)-1,2,3,4-tetrahydrochinolin,
2-Carboxy-4-[2-(carboxyethyl)carbonylamino]-5,7-dichlor-1,2,3,4-tetrahydrochinolin,
4-[3-(Aminomethyl)phenylcarbonylamino]-2-carboxy-5,7-dichlor-1,2,3,4-tetrahydrochinolin,
4-[3-(Aminomethyl)phenylcarbonylamino]-5,7-dichlor-2-methoxycarbonyl-1,2,3,4-tetrahydrochinolin,
4-[4-(Aminomethyl)phenylcarbonylamino]-2-carboxy-5,7-dichlor-1,2,3,4-tetrahydrochinolin,
4-[4-(Aminomethyl)phenylcarbonylamino]-5,7-dichlor-2-methoxycarbonyl-1,2,3,4-tetrahydrochinolin,
4-[4-(2-Aminoethyl)phenylcarbonylamino]-2-carboxy-5,7-dichlor-1,2,3,4-tetrahydrochinolin,
4-[4-(2-Aminoethyl)phenylcarbonylamino]-5,7-dichlor-2-methoxycarbonyl-1,2,3,4-tetrahydrochinolin,
2-Carboxy-5,7-dichlor-4-(3-methylbenzylcarbonylamino)-1,2,3,4-tetrahydrochinolin,
2-Carboxy-5,7-dichlor-4-(3-nitrobenzylcarbonylamino)-1,2,3,4-tetrahydrochinolin,
2-Carboxy-5,7-dichlor-4-(3-methoxybenzylcarbonylamino)-1,2,3,4-tetrahydrochinolin,
2-Carboxy-5,7-dichlor-4-(1-naphthylmethylcarbonylamino)-1,2,3,4-tetrahydrochinolin,
2-Carboxy-5,7-dichlor-4-(2-naphthylmethylcarbonylamino)-1,2,3,4-tetrahydrochinolin,
2-Carboxy-5,7-dichlor-4-(3-thienylmethylcarbonylamino)-1,2,3,4-tetrahydrochinolin,
2-Carboxy-5,7-dichlor-4-(2,6-dichlorbenzylcarbonylamino)-1,2,3,4-tetrahydrochinolin,
4-(3-Aminopropyl)carbonylamino-2-carboxy-5,7-dichlor-1,2,3,4-tetrahydrochinolin,
4-(2-Aminoethyl)carbonylamino-2-carboxy-5,7-dichlor-1,2,3,4-tetrahydrochinolin,
4-(4-Aminobutyl)carbonylamino-2-carboxy-5,7-dichlor-1,2,3,4-tetrahydrochinolin,
2-Carboxy-5,7-dichlor-4-(4-piperidylmethylcarbonylamino)-1,2,3,4-tetrahydrochinolin,
4-[4-(Aminomethyl)benzylcarbonylaminol-2-carboxy-5,7-dichlor-1,2,3,4-tetrahydrochinolin,
4-[4-(Aminomethyl)benzylcarbonylamino]-5,7-dichlor-2-methoxycarbonyl-1,2,3,4-tetrahydrochinolin,
4-[4-(2-Aminoethyl)benzylcarbonylamino]-2-carboxy-5,7-dichlor-1,2,3,4-tetrahydrochinolin,
4-[4-(2-Aminoethyl)benzylcarbonylamino]-5,7-dichlor-2-methoxycarbonyl-1,2,3,4-tetrahydrochinolin,
2-Carboxy-5,7-dichlor-4-[4-(N-methylaminomethyl)benzylcarbonylamino]-1,2,3,4-tetrahydrochinolin,
5,7-Dichlor-2-methoxycarbonyl-4-[4-(N-methylaminomethyl)benzylcarbonylamino]-1,2,3,4-tetrahydrochinolin,
5,7-Dichlor-4-[4-(N,N-dimethylaminomethyl)benzylcarbonylamino]-2-methoxycarbonyl-1,2,3,4-tetrahydrochinolin,
2-Carboxy-5,7-dichlor-4-[4-(N,N-dimethylaminomethyl)benzylcarbonylamino]-1,2,3,4-tetrahydrochinolin,
4-[4-(3-Aminoprop-2-inyl)benzylcarbonylamino]-2-carboxy-5,7-dichlor-1,2,3,4-tetrahydrochinolin,
4-[4-(3-Aminoprop-2-inyl)benzylcarbonylaminol-5,7-dichlor-2-methoxycarbonyl-1,2,3,4-tetrahydrochinolin,
4-[4-(3-Aminopropyl)benzylcarbonylamino]-2-carboxy-5,7-dichlor-1,2,3,4-tetrahydrochinolin,
4-[4-(3-Aminopropyl)benzylcarbonylaminol-5,7-dichlor-2-methoxycarbonyl-1,2,3,4-tetrahydrochinolin,
4-[4-(4-Aminobut-2-inyl)benzylcarbonylamino]-2-carboxy-5,7-dichlor-1,2,3,4-tetrahydrochinolin,
4-[4-(4-Aminobut-2-inyl)benzylcarbonylamino]-5,7-dichlor-2-methoxycarbonyl-1,2,3,4-tetrahydrochinolin,
4-[4-(4-Aminobutyl)benzylcarbonylamino]-2-carboxy-5,7-dichlor-1,2,3,4-tetrahydrochinolin,
4-[4-(4-Aminobutyl)benzylcarbonylamino]-5,7-dichlor-2-methoxycarbonyl-1,2,3,4-tetrahydrochinolin,
4-[3-(Aminomethyl)benzylcarbonylamino]-2-carboxy-5,7-dichlor-1,2,3,4-tetrahydrochinolin,
4-[3-(Aminomethyl)benzylcarbonylamino]-5,7-dichlor-2-methoxycarbonyl-1,2,3,4-tetrahydrochinolin,
4-[2-(Aminomethyl)benzylcarbonylamino]-2-carboxy-5,7-dichlor-1,2,3,4-tetrahydrochinolin,
4-[2-(Aminomethyl)benzylcarbonylamino]-5,7-dichlor-2-methoxycarbonyl-1,2,3,4-tetrahydrochinolin,
4-[2-(4-(Aminomethyl)phenyl)ethylcarbonylamino]-2-carboxy-5,7-dichlor-1,2,3,4-tetrahydrochinolin,
4-[2-(4-(Aminomethyl)phenyl)ethylcarbonylamino]-5,7-dichlor-2-methoxycarbonyl-1,2,3,4-tetrahydrochinolin,
4-(4-Aminobenzylcarbonylamino)-2-carboxy-5,7-dichlor-1,2,3,4-tetrahydrochinolin,
2-Carboxy-4-(2-carboxyphenylcarbonylamino)-5,7-dichlor-1,2,3,4-tetrahydrochinolin,
2-Carboxy-4-(4-chlor-3-nitrobenzylcarbonylamino)-5,7-dichlor-1,2,3,4-tetrahydrochinolin,
2-Carboxy-5,7-dichlor-4-(4-hydroxy-3-nitrobenzylcarbonylamino)-1,2,3,4-tetrahydrochinolin,
2-Carboxy-5,7-dichlor-4-(diphenylmethylcarbonylamino)-1,2,3,4-tetrahydrochinolin,
2-Carboxy-5,7-dichlor-4-(1-phenylethyl)carbonylamino-1,2,3,4-tetrahydrochinolin,
4-(4-Acetylbenzylcarbonylamino)-2-carboxy-5,7-dichlor-1,2,3,4-tetrahydrochinolin,
2-Carboxy-5,7-dichlor-4-phenoxymethylcarbonylamino-1,2,3,4-tetrahydrochinolin,
2-Carboxy-5,7-dichlor-4-(4-ethylbenzylcarbonylamino)-1,2,3,4-tetrahydrochinolin,
2-Carboxy-5,7-dichlor-4-(4-hydroxybenzylcarbonylamino)-1,2,3,4-tetrahydrochinolin,
4-(4-Acetamidobenzylcarbonylamino)-2-carboxy-5,7-dichlor-1,2,3,4-tetrahydrochinolin,
4-Benzylcarbonylamino-2-(t-butylcarbonyloxy)methoxycarbonyl-5,7-dichlor-1,2,3,4-tetrahydrochinolin,
4-Benzylcarbonylamino-5,7-dichlor-2-(methylaminocarbonyl)methoxycarbonyl-1,2,3,4-tetrahydrochinolin,
4-Benzylcarbonylamino-5,7-dichlor-2-[2-(N,N-dimethylamino)ethylaminocarbonyl]methoxycarbonyl-1,2,3,4-tetrahydrochinolin,
4-Benzylcarbonylamino-5,7-dichlor-2-[2-(N,N-dimethylamino)ethoxycarbonyl]-1,2,3,4-tetrahydrochinolin,
4-Benzylcarbonylamino-5,7-dichlor-2-[3-(N,N-dimethylamino)propoxycarbonyl]-1,2,3,4-tetrahydrochinolin,
4-Benzylcarbonylamino-5,7-dichlor-2-[2-(N,N-dimethylamino)ethylaminocarbonyl]-1,2,3,4-tetrahydrochinolin,
4-[4-(Aminomethyl)benzylcarbonylamino]-5,7-dichlor-2-(methylaminocarbonyl)methoxycarbonyl-1,2,3,4-tetrahydrochinolin,
4-[4-(Aminomethyl)benzylcarbonylamino]-5,7-dichlor-2-hexyloxycarbonyl-1,2,3,4-tetrahydrochinolin
und Salzen davon, worin die relative Orientierung der Substituenten in der 2- und 4-Stellung des Tetrahydrochinolin-Ringsystems trans ist.

8. Eine Verbindung wie in Anspruch 3 beansprucht, ausgewählt aus:
2-Carboxy-5,7-dichlor-4-phenylaminocarbonylamino-1,2,3,4-tetrahydrochinolin,
4-Benzylaminocarbonylamino-2-carboxy-5,7-dichlor-1,2,3,4-tetrahydrochinolin,
2-Carboxy-5,7-dichlor-4-(2-nitrophenylaminocarbonylamino)-1,2,3,4-tetrahydrochinolin,
2-Carboxy-5,7-dichlor-4-(2-methoxyphenylaminocarbonylamino)-1,2,3,4-tetrahydrochinolin,
2-Carboxy-5,7-dichlor-4-(2-methylphenylaminocarbonylamino)-1,2,3,4-tetrahydrochinolin,
2-Carboxy-4-(2-chlorphenylaminocarbonylamino)-5,7-dichlor-1,2,3,4-tetrahydrochinolin,
2-Carboxy-4-(4-chlorphenylaminocarbonylamino)-5,7-dichlor-1,2,3,4-tetrahydrochinolin,
2-Carboxy-5,7-dichlor-4-(4-methylphenylaminocarbonylamino)-1,2,3,4-tetrahydrochinolin,
2-Carboxy-5,7-dichlor-4-(4-methoxyphenylaminocarbonylamino)-1,2,3,4-tetrahydrochinolin,
2-Carboxy-5,7-dichlor-4-(4-nitrophenylaminocarbonylamino)-1,2,3,4-tetrahydrochinolin,
2-Carboxy-5,7-dichlor-4-(4-iodphenylaminocarbonylamino)-1,2,3,4-tetrahydrochinolin,
2-Carboxy-5,7-dichlor-4-[phenylaminocarbonyl(N-methyl)amino]-1,2,3,4-tetrahydrochinolin,
2-Carboxy-5,7-dichlor-4-[(N-methyl-N-phenyl)aminocarbonylamino]-1,2,3,4-tetrahydrochinolin,
2-Carboxy-5,7-dichlor-4-phenylaminothiocarbonylamino-1,2,3,4-tetrahydrochinolin,
2-Carboxy-5,7-dichlor-4-(3-methoxyphenylaminocarbonylamino)-1,2,3,4-tetrahydrochinolin,
2-Carboxy-5,7-dichlor-4-(3-methylphenylaminocarbonylamino)-1,2,3,4-tetrahydrochinolin,
2-Carboxy-4-(3-chlorphenylaminocarbonylamino)-5,7-dichlor-1,2,3,4-tetrahydrochinolin,
2-Carboxy-5,7-dichlor-4-(3-nitrophenylaminocarbonylamino)-1,2,3,4-tetrahydrochinolin,
2-Carboxy-5,7-dichlor-4-(1-naphthylaminocarbonylamino)-1,2,3,4-tetrahydrochinolin,
2-Carboxy-4-cyclohexylaminocarbonylamino-5,7-dichlor-1,2,3,4-tetrahydrochinolin,
2-Carboxy-5,7-dichlor-4-[N-methyl-N-(4-methylphenyl)amino]carbonylamino-1,2,3,4-tetrahydrochinolin,
2-Carboxy-5,7-dichlor-4-(N,N-diphenylamino)carbonylamino-1,2,3,4-tetrahydrochinolin,
2-Carboxy-5,7-dichlor-4-(4-ethylphenyl)aminocarbonylamino-1,2,3,4-tetrahydrochinolin,
2-Carboxy-5,7-dichlor-4-(N-ethyl-N-phenyl)aminocarbonylamino-1,2,3,4-tetrahydrochinolin,
5,7-Dichlor-2-[2-(N,N-dimethylamino)ethylaminocarbonyl]-4-phenylaminocarbonylamino-1,2,3,4-tetrahydrochinolin
und Salzen davon, worin die relative Orientierung der Substituenten in der 2- und 4-Stellung des Tetrahydrochinolin-Ringsystems trans ist.

9. Trans-2-Carboxy-5,7-dichlor-4-phenylaminocarbonylamino-1,2,3,4 tetrahydrochinolin oder ein pharmazeutisch annehmbares Salz davon.

10. Eine pharmazeutische Zusammensetzung mit einer wie in irgendeinem der vorhergehenden Ansprüche beanspruchten Verbindung in Verbindung mit einem pharmazeutisch annehmbaren Träger.

11. Die Verwendung einer wie in irgendeinem der Ansprüche 1 bis 9 beanspruchten Verbindung zur Herstellung eines Medikaments zur Behandlung und/oder Verhütung von neurodegenerativen Störungen.

12. Ein Verfahren zur Herstellung einer wie in irgendeinem der Ansprüche 1 bis 9 beanspruchten Verbindung, wobei dieses Verfahren umfaßt:
(A) Reduktion eines Chinolinderivats der Formel IV: 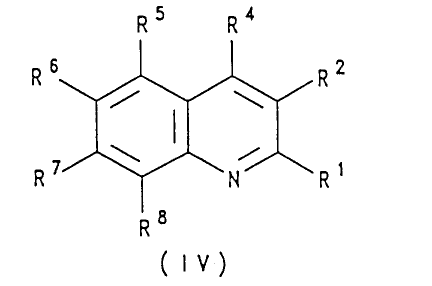 worin R¹, R², R⁵, R⁶, R⁷ und R⁸ wie in Anspruch 1 definiert sind, und R⁴ -NR^{a}COR^{b} oder -NRⁱCXNR^{a}R^{b} bedeutet, die wie in den Ansprüchen 1 bzw. 3 definiert sind, oder
(B) Umsetzung einer Verbindung der Formel A-COR^{b} mit einer Verbindung der Formel V: 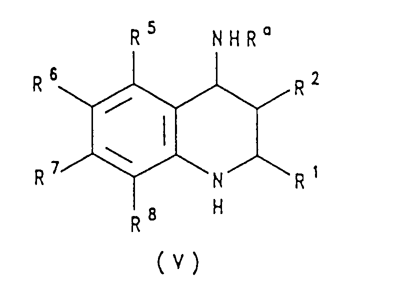 worin die relative Orientierung der R¹- und -NHR^{a}-Substituenten trans ist, R¹, R², R⁵, R⁶, R⁷, R⁸, R^{a} und R^{b} wie in Anspruch 1 definiert sind, und A eine Abgangsgruppe bedeutet, oder
(C) Umsetzung einer Verbindung der Formel X oder eines geschützten Derivats davon mit einer Verbindung der Formel XI oder einem geschützten Derivat davon: 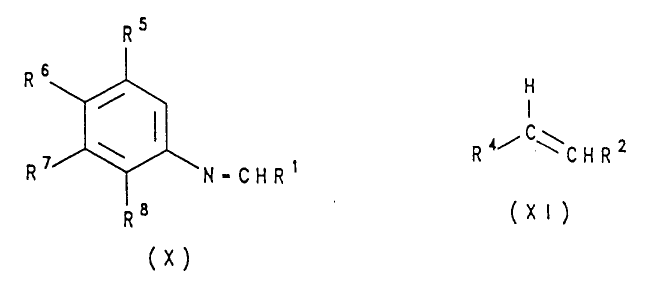 worin R¹, R² und R⁵ bis R⁸ wie in Anspruch 1 definiert sind und R⁴ wie oben definiert ist, gefolgt, wo notwendig, von Entfernen der Schutzgruppen.

## Revendications

1. Un composé de la formule IIC ou un sel de celui-ci: 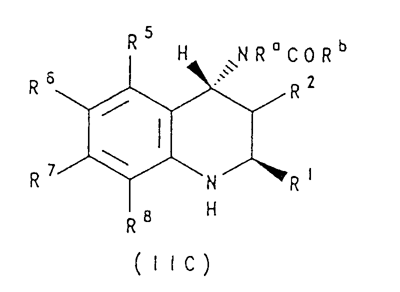 dans laquelle
R¹ représente un groupe carboxy ou un groupe qui est convertible in vivo en celui-ci;
R² représente de l'hydrogène;
R⁵, R⁶, R⁷ et R⁸ représentent, de façon indépendante, un groupe hydrogène, halogène, cyano, trifluorométhyle, nitro, hydroxy, amino, carboxy, alkyle en C₁ à C₆, alcoxy en C₁ à C₆, alkylthio en C₁ à C₆, ou alcoxy-carbonyle en C₁ à C₆; et
R^{a} et R^{b} représentent indépendamment de l'hydrogène ou un groupe alkyle en C₁ à C₆, alcényle en C₂ à C₆, cycloalkyle en C₃ à C₇, cycloalkyle en C₃ à C₇-alkyl (en C₁ à C₆), aryle choisi parmi le phényle, le naphtyle et le fluorényle, arylalkyl (en C₁ à C₆), hétérocycloalkyl en C₃ à C₇, hétérocycloalkyl (en C₃ à C₇)-alkyl (en C₁ à C₆), hétéroaryle choisi parmi les radicaux pirydyle, quinoléyle, isoquinoléyle, pyridazinyle, pyrimidinyle, pyrazinyle, pyranyle, furyle, benzofuryle, thiényle, benzthiényle, indolinyle, imidazolyle, oxadiazolyle, thiadiazolyle et tétrazolyle, ou bien un hétéroaryl-alkyl (en C₁ à C₆), l'un quelconque parmi ces groupes peut être substitué, le cas échéant, par un ou plus d'un groupe choisi parmi les groupes méthyle, éthyle, phényle, chloro, aminométhyle, aminoéthyle, aminopropyle, aminobutyle, méthylaminométhyle, diméthylaminométhyle, aminopropynyle, aminobutynyle, hydroxy, méthoxy, phénoxy, nitro, cyano, carboxy, acétyle, amino et acétylamino.

2. Un composé tel que revendiqué dans la revendication 1, dans lequel R^{a} est l'hydrogène et R^{b} représente du phényle ou du benzyle, facultativement substitués par un groupe aminométhyle ou un groupe aminoéthyle.

3. Un composé de la formule IID ainsi que ses sels: 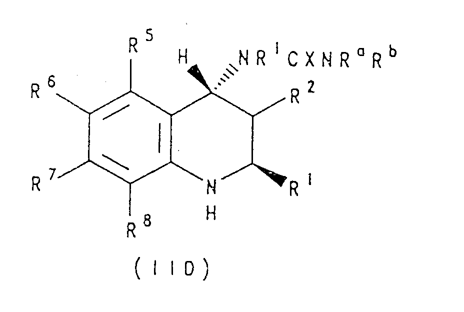 dans laquelle R¹, R², R⁵, R⁶, R⁷ et R⁸ sont tels que définis dans la revendication 1; R^{a} et Rⁱ représentent indépendamment de l'hydrogène, un alkyle en C₁ à C₆ ou un aryle choisi parmi le phényle, le naphtyle et le fluorényle; R^{b} représente un cycloalkyle en C₃ à C₇, un aryle choisi parmi le phényle, le naphtyle et le fluorényle, ou bien un arylalkyle (en C₁ à C₆), l'un quelconque parmi ces groupes pouvant être le cas échéant substitué par un ou plus d'un groupe choisi parmi les groupes méthyle, éthyle, chloro, iodo, méthoxy et nitro; et X représente de l'oxygène ou du soufre.

4. Un composé tel que revendiqué dans la revendication 3, dans lequel R^{a} et Rⁱ représentent indépendamment de l'hydrogène, du méthyle, de l'éthyle ou du phényle; et R^{b} représente du cyclohexyle, du phényle, du naphtyle ou du benzyle.

5. Un composé tel que revendiqué dans la revendication 4, dans lequel R^{a} et Rⁱ représentent chacun de l'hydrogène; et X représente de l'oxygène.

6. Un composé tel que revendiqué dans l'une quelconque des revendications précédentes, dans lequel R⁸ représente de l'hydrogène et R⁵, R⁶ et R⁷ représentent, indépendamment, de l'hydrogène, de l'halogène ou un alkyle en C₁ à C₆.

7. Un composé tel que revendiqué dans la revendication 1, choisi parmi les:
4-benzolylamino-2-carboxy-5,7-dichloro-1,2,3,4-tétrahydroquinoléine;
4-acétylamino-2-carboxy-5,7-dichloro-1,2,3,4-tétrahydroquinoléine;
2-carboxy-5,7-dichloro-4-cyclohexylcarbonylamino-1,2,3,4-tétrahydroquinoléine;
4-benzylcarbonylamino-2-carboxy-5,7-dichloro-1,2,3,4-tétrahydroquinoléine;
2-carboxy-4-(4-chlorophénylcarbonylamino)-5,7-dichloro-1,2,3,4-tétrahydroquinoléine;
2-carboxy-5,7-dichloro-4-(4-pyridylcarbonylamino)-1,2,3,4-tétrahydroquinoléine;
2-carboxy-5,7-dichloro-4-[2-(2-aminophénéthyl)]-carbonylamino)-1,2,3,4-tétrahydroquinoléine;
2-carboxy-5,7-dichloro-4-n-propylcarbonylamino-1,2,3,4-tétrahydroquinoléine;
2-carboxy-5,7-dichloro-4-(4-méthoxyphénylcarbonylamino)-1,2,3,4-tétrahydroquinoléine;
2-carboxy-5,7-dichloro-4-(2-méthoxybenzylcarbonylamino)-1,2,3,4-tétrahydroquinoléine;
2-carboxy-5,7-dichloro-4-(2-méthylbenzylcarbonylamino)-1,2,3,4-tétrahydroquinoléine;
2-carboxy-5,7-dichloro-4-(α-méthoxybenzylcarbonylamino)-1,2,3,4-tétrahydroquinoléine;
2-carboxy-5,7-dichloro-4-(2-nitrobenzylcarbonylamino)-1,2,3,4-tétrahydroquinoléine;
4-(4'-biphénylcarbonylamino)-2-carboxy-5,7-dichloro-1,2,3,4-tétrahydroquinoléine;
2-carboxy-5,7-dichloro-4-isopropylcarbonylamino)-1,2,3,4-tétrahydroquinoléine;
2-carboxy-4-(2-chlorophénylcarbonylamino)-5,7-dichloro-1,2,3,4-tétrahydroquinoléine;
2-carboxy-5,7-dichloro-4-(1-naphtylcarbonylamino)-1,2,3,4-tétrahydroquinoléine;
2-carboxy-5,7-dichloro-4-(2-naphtylcarbonylamino)-1,2,3,4-tétrahydroquinoléine;
2-carboxy-5,7-dichloro-4-(2-furylcarbonylamino)-1,2,3,4-tétrahydroquinoléine;
2-carboxy-5,7-dichloro-4-(2-méthylphénylcarbonylamino)-1,2,3,4-tétrahydroquinoléine;
2-carboxy-5,7-dichloro-4-(2-phénéthylcarbonylamino)-1,2,3,4-tétrahydroquinoléine;
2-carboxy-5,7-dichloro-4-(2-phényléthénylcarbonylamino)-1,2,3,4-tétrahydroquinoléine;
2-carboxy-5,7-dichloro-4-(2-thiénylméthylcarbonylamino)-1,2,3,4-tétrahydroquinoléine;
2-carboxy-4-(3-chlorophénylcarbonylamino)-5,7-dichloro-1,2,3,4-tétrahydroquinoléine;
2-carboxy-5,7-dichloro-4-(3-phénylpropylcarbonylamino)-1,2,3,4-tétrahydroquinoléine;
2-carboxy-5,7-dichloro-4-(9-fluorénylcarbonylamino)-1,2,3,4-tétrahydroquinoléine;
2-carboxy-4-cyclohexylméthylcarbonylamino)-5,7-dichloro-1,2,3,4-tétrahydroquinoléine;
2-carboxy-4-(2-chlorobenzylcarbonylamino)-5,7-dichloro-1,2,3,4-tétrahydroquinoléine;
2-carboxy-4-(3-chlorobenzylcarbonylamino)-5,7-dichloro-1,2,3,4-tétrahydroquinoléine;
2-carboxy-4-(4-chlorobenzylcarbonylamino)-5,7-dichloro-1,2,3,4-tétrahydroquinoléine;
2-carboxy-5,7-dichloro-4-(4-méthylbenzylcarbonylamino)-1,2,3,4-tétrahydroquinoléine;
2-carboxy-5,7-dichloro-4-(4-méthoxylbenzylcarbonylamino)-1,2,3,4-tétrahydroquinoléine;
2-carboxy-5,7-dichloro-4-(4-nitrobenzylcarbonylamino)-1,2,3,4-tétrahydroquinoléine;
2-carboxy-4-(3-cyanophénylcarbonylamino)-5,7-dichloro-1,2,3,4-tétrahydroquinoléine;
2-carboxy-5,7-dichloro-4-(2,3-dihydroindol-1-carbonylamino)-1,2,3,4-tétrahydroquinoléine;
2-carboxy-4-[2-(carboxyéthyl)carbonylamino]-5,7-dichloro-1,2,3,4-tétrahydroquinoléine;
4-[3-(aminométhyl)phénylcarbonylamino]-2-carboxy-5,7-dichloro-1,2,3,4-tétrahydroquinoléine;
4-[3-(aminométhyl)phénylcarbonylamino]-5,7-dichloro-2-méthoxycarbonyl-1,2,3,4-tétrahydroquinoléine;
4-[4-(aminométhyl)phénylcarbonylamino]-2-carboxy-5,7-dichloro-1,2,3,4-tétrahydroquinoléine;
4-[4-(aminométhyl)phénylcarbonylamino]-5,7-dichloro-2-méthoxycarbonyl-1,2,3,4-tétrahydroquinoléine;
4-[4-(2-aminoéthyl)phénylcarbonylamino]-2-carboxy-5,7-dichloro-1,2,3,4-tétrahydroquinoléine;
4-[4-(2-aminométhyl)phénylcarbonylamino]-5,7-dichloro-2-méthoxycarbonyl-1,2,3,4-tétrahydroquinoléine;
2-carboxy-5,7-dichloro-4-(3-méthylbenzylcarbonylamino)-1,2,3,4-tétrahydroquinoléine;
2-carboxy-5,7-dichloro-4-(3-nitrobenzylcarbonylamino)-1,2,3,4-tétrahydroquinoléine;
2-carboxy-5,7-dichloro-4-(3-méthoxybenzylcarbonylamino)-1,2,3,4-tétrahydroquinoléine;
2-carboxy-5,7-dichloro-4-(1-naphtylméthylcarbonylamino)-1,2,3,4-tétrahydroquinoléine;
2-carboxy-5,7-dichloro-4-(2-naphtylméthylcarbonylamino)-1,2,3,4-tétrahydroquinoléine;
2-carboxy-5,7-dichloro-4-(3-thiénylméthylcarbonylamino)-1,2,3,4-tétrahydroquinoléine;
2-carboxy-5,7-dichloro-4-(2,6-dichlorobenzylcarbonylamino)-1,2,3,4-tétrahydroquinoléine;
4-(3-aminopropyl)carbonylamino-2-carboxy-5,7-dichloro-1,2,3,4-tétrahydroquinoléine;
4-(4-aminobutyl)carbonylamino-2-carboxy-5,7-dichloro-1,2,3,4-tétrahydroquinoléine;
4-(3-aminobutyl)carbonylamino-2-carboxy-5,7-dichloro-1,2,3,4-tétrahydroquinoléine;
2-carboxy-5,7-dichloro-4-(4-pipéridylméthylcarbonylamino)-1,2,3,4-tétrahydroquinoléine;
4-[4-(aminométhyl)benzylcarbonylamino]-2-carboxy-5,7-dichloro-1,2,3,4-tétrahydroquinoléine;
4-[4-(aminométhyl)benzylcarbonylamino]-5,7-dichloro-1,2,3,4-2-méthoxycarbonyl-1,2,3,4-tétrahydroquinoléine;
4-[4-(2-aminoéthyl)benzylcarbonylamino]-2-carboxy-5,7-dichloro-1,2,3,4-tétrahydroquinoléine;
4-[4-(aminoéthyl)benzylcarbonylamino]-5,7-dichloro-2-méthoxycarbonyl-1,2,3,4-tétrahydroquinoléine;
2-carboxy-5,7-dichloro-4-[4-(N-méthylaminoéthyl)-benzylcarbonylamino]-1,2,3,4-tétrahydroquinoléine;
5,7-dichloro-2-méthoxycarbonyl-4-[4-(N-méthylaminométhyl)benzylcarbonylamino]-1,2,3,4-tétrahydroquinoléine;
5,7-dichloro-4-[4-(N,N-diméthylaminométhyl)-benzyl-carbonylamino]-2-méthoxycarbonyl-1,2,3,4-tétrahydroquinoléine;
2-carboxy-5,7-dichloro-4-[4-(N,N-diméthylaminométhyl)-benzylcarbonylamino]-1,2,3,4-tétrahydroquinoléine;
4-[4-(3-aminoprop-2-ynyl)benzylcarbonylamino]-2-carboxy-5,7-dichloro-1,2,3,4-tétrahydroquinoléine;
4-[4-(3-aminoprop-2-ynyl)benzylcarbonylamino]-5,7-dichloro-2-méthoxycarbonyl-1,2,3,4-tétrahydroquinoléine;
4-[4-(3-aminopropyl)benzylcarbonylamino]-2-carboxy-5,7-dichloro-1,2,3,4-tétrahydroquinoléine;
4-[4-(3-aminopropyl)benzylcarbonylamino]-5,7-dichloro-2-méthoxycarbonyl-1,2,3,4-tétrahydroquinoléine;
4-[4-(4-aminobut-2-ynyl)benzylcarbonylamino]-2-carboxy-5,7-dichloro-1,2,3,4-tétrahydroquinoléine;
4-[4-(4-aminobut-2-ynyl)benzylcarbonylamino]-5,7-dichloro-2-méthoxycarbonyl-1,2,3,4-tétrahydroquinoléine;
4-[4-(4-aminobutyl)benzylcarbonylamino]-2-carboxy-5,7-dichloro-1,2,3,4-tétrahydroquinoléine;
4-[4-(4-aminobutyl)benzylcarbonylamino]-5,7-dichloro-2-méthoxycarbonyl-1,2,3,4-tétrahydroquinoléine;
4-[3-(aminométhyl)benzylcarbonylamino]-2-carboxy-5,7-dichloro-1,2,3,4-tétrahydroquinoléine;
4-[3-(aminométhyl)benzylcarbonylamino]-5,7-dichloro-2-méthoxycarbonyl-1,2,3,4-tétrahydroquinoléine;
4-[2-(aminométhyl)benzylcarbonylamino]-2-carboxy-5,7-dichloro-1,2,3,4-tétrahydroquinoléine;
4-[2-(aminométhyl)benzylcarbonylamino]-5,7-dichloro-2-méthoxy carbonyl-1,2,3,4-tétrahydroquinoléine;
4-[2-(4-(aminométhyl)phényl) éthylcarbonylamino]-2-carboxy-5,7-dichloro-1,2,3,4-tétrahydroquinoléine;
4-[2-(4-(aminométhyl)phényl) éthylcarbonylamino]-5,7-dichloro-2-méthoxycarbonyl-1,2,3,4-tétrahydroquinoléine;
4-(4-(aminobenzylcarbonylamino]-2-carboxy-5,7-dichloro-1,2,3,4-tétrahydroquinoléine;
2-carboxy-4-(2-carboxyphénylcarbonylamino)-5,7-dichloro-1,2,3,4-tétrahydroquinoléine;
2-carboxy-4-(4-chloro-3-nitrobenzylcarbonylamino)-5,7-dichloro-1,2,3,4-tétrahydroquinoléine;
2-carboxy-5,7-dichloro-4-(4-hydroxy-3-nitrobenzylcarbonylamino)-1,2,3,4-tétrahydroquinoléine;
2-carboxy-5,7-dichloro-4-(diphénylméthylcarbonylamino)-1,2,3,4-tétrahydroquinoléine;
2-carboxy-5,7-dichloro-4-(1-phényléthyl)carbonylamino)-1,2,3,4-tétrahydroquinoléine;
4-(4-acétylbenzylcarbonylamino)-2-carboxy-5,7-dichloro-1,2,3,4-tétrahydroquinoléine;
2-carboxy-5,7-dichloro-4-(1-phénoxyméthylcarbonylamino)-1,2,3,4-tétrahydroquinoléine;
2-carboxy-5,7-dichloro-4-(4-éthylbenzylcarbonylamino)-1,2,3,4-tétrahydroquinoléine;
2-carboxy-5,7-dichloro-4-(4-hydroxybenzylcarbonylamino)-1,2,3,4-tétrahydroquinoléine;
4-(4-acétamidobenzylcarbonylamino)-2-carboxy-5,7-dichloro-1,2,3,4-tétrahydroquinoléine;
4-benzylcarbonylamino)-2-(t-butylcarbonyloxy)méthoxycarbonyl-5,7-dichloro-1,2,3,4-tétrahydroquinoléine;
4-benzylcarbonylamino)-5,7-dichloro-2-(méthylamino-butylcarbonyl)méthoxycarbonyl-1,2,3,4-tétrahydroquinoléine;
4-benzylcarbonylamino)-5,7-dichloro-2-[2-N,N-diméthylamino)éthylaminocarbonyl] méthoxycarbonyl-1,2,3,4-tétrahydroquinoléine;
4-benzylcarbonylamino)-5,7-dichloro-2-[2-N,N-diméthylamino)éthoxycarbonyl]-1,2,3,4-tétrahydroquinoléine;
4-benzylcarbonylamino-5,7-dichloro-2-[3-N,N-diméthylamino)propoxycarbonyl] 1,2,3,4-tétrahydroquinoléine;
4-benzylcarbonylamino-5,7-dichloro-2-[2-(N,N-diméthylamino)éthylaminocarbonyl]-1,2,3,4-tétrahydroquinoléine;
4-[4-(aminométhyl)benzylcarbonylamino]-5,7-dichloro-2-(méthylaminocarbonyl)méthoxycarbonyl-1,2,3,4-tétrahydroquinoléine;
4-[4-(aminométhyl)benzylcarbonylamino]-5,7-dichloro-2-(hexyloxycarbonyl-1,2,3,4-tétrahydroquinoléine; et les sels de ceux-ci, la relative orientation des substituants en position 2- et 4- du système cyclique de la tétrahydroquinoléine étant trans.

8. Un composé tel que revendiqué dans la revendication 3, choisi parmi les:
2-carboxy-5,7-dichloro-4-phénylaminocarbonylamino)-1,2,3,4-tétrahydroquinoléine;
4-benzylcarbonylamino-2-carboxy-5,7-dichloro-1,2,3,4-tétrahydroquinoléine;
2-carboxy-5,7-dichloro-4-(2-nitrophénylaminocarbonylamino)-1,2,3,4-tétrahydroquinoléine;
2-carboxy-5,7-dichloro-4-(2-méthoxyphénylaminocarbonylamino)-1,2,3,4-tétrahydroquinoléine;
2-carboxy-5,7-dichloro-4-(2-méthylphénylaminocarbonylamino)-1,2,3,4-tétrahydroquinoléine;
2-carboxy-4-(2-chlorophénylaminocarbonylamino)-5,7-dichloro-1,2,3,4-tétrahydroquinoléine;
2-carboxy-4-(4-chlorophénylaminocarbonylamino)-5,7-dichloro-1,2,3,4-tétrahydroquinoléine;
2-carboxy-5,7-dichloro-4-(4-méthylphénylaminocarbonylamino)-1,2,3,4-tétrahydroquinoléine;
2-carboxy-5,7-dichloro-4-(4-méthoxyphénylaminocarbonylamino)-1,2,3,4-tétrahydroquinoléine;
2-carboxy-5,7-dichloro-4-(4-nitrophénylaminocarbonylamino)-1,2,3,4-tétrahydroquinoléine;
2-carboxy-5,7-dichloro-4-(4-iodophénylaminocarbonylamino)-1,2,3,4-tétrahydroquinoléine;
2-carboxy-5,7-dichloro-4-[phénylaminocarbonyl)(N-méthyl)amino]-1,2,3,4-tétrahydroquinoléine;
2-carboxy-5,7-dichloro-4-[(N-méthyl-N-phényl)aminocarbonylamino]-1,2,3,4-tétrahydroquinoléine;
2-carboxy-5,7-dichloro-(4-phénylaminothiocarbonylamino)-1,2,3,4-tétrahydroquinoléine;
2-carboxy-5,7-dichloro-4-(3-méthoxyphénylaminocarbonylamino)-1,2,3,4-tétrahydroquinoléine;
2-carboxy-5,7-dichloro-4-(3-méthylphénylaminocarbonylamino)-1,2,3,4-tétrahydroquinoléine;
2-carboxy-4-(3-chlorophénylamiocarbonylamino)-5,7-dichloro-1,2,3,4-tétrahydroquinolénine;
2-carboxy-5,7-dichloro-4-(3-nitrophénylaminocarbonylamino)-1,2,3,4-tétrahydroquinoléine;
2-carboxy-5,7-dichloro-4-(1-naphtyphénylaminocarbonylamino)-1,2,3,4-tétrahydroquinoléine;
2-carboxy-4-cyclohexylaminocarbonylamino)-5,7-dichloro-1,2,3,4-tétrahydroquinoléine;
2-carboxy-5,7-dichloro-4-[(N-méthyl-N-(4-méthylphényl)amino]carbonylamino]-1,2,3,4-tétrahydroquinoléine;
2-carboxy-5,7-dichloro-4-(N,N-diphénylamino)carbonylamino-1,2,3,4-tétrahydroquinoléine;
2-carboxy-5,7-dichloro-4-(4-éthylphényl)aminocarbonylamino-1,2,3,4-tétrahydroquinoléine;
2-carboxy-5,7-dichloro-4-(N-éthyl-N-phényl)-aminocarbonylamino)-1,2,3,4-tétrahydroquinoléine;
5,7-dichloro-2-[2-(N-N-diméthylamino)éthylaminocarbonylamino]-4-phénylaminocarbonylamino-1,2,3,4-tétrahydroquinoléine; et les sels de ceux-ci, la relative orientation des substituants en position 2- et 4- du système cyclique de la tétrahydroquinoléine étant trans.

9. La trans-2-carboxy-5,7-dichloro-4-phénylaminocarbonylamino-1,2,3,4-tétrahydroquinoléine, ou un de ses sels pharmaceutiquement acceptables.

10. Une composition pharmaceutique comportant un composé tel que revendiqué dans l'une quelconque des revendications précédentes en association avec un support pharmaceutiquement acceptable.

11. L'utilisation d'un composé tel que revendiqué dans l'une quelconque des revendications 1 à 9, pour la fabrication d'un médicament pour le traitement et/ou la prévention de désordres neurodégénératifs.

12. Un procédé pour la préparation d'un composé tel que revendiqué dans l'une quelconque des revendications 1 à 9, ce procédé comportant:
(A) la réduction d'un dérivé de la quinoléine de formule IV: 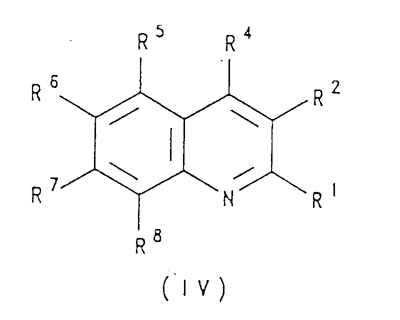 dans laquelle R¹, R²,R⁵, R⁶, R⁷ et R⁸ sont tels que définis dans la revendication 1 et R⁴ représente -NR^{a}COR^{b} ou -NRⁱCXNR^{a}R^{b} tels que définis dans les revendications 1 et 3, respectivement; ou
(B) la réaction d'un composé de formule A-COR^{b} avec un composé de formule V: 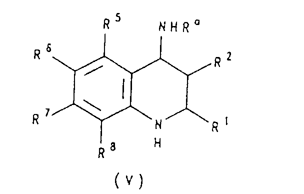 dans laquelle l'orientation relative des substituant R¹ et -NHR^{a} est trans; R¹, R²,R⁵, R⁶, R⁷ et R⁸, R^{a} et R^{b} sont définis comme dans la revendication 1, et A représente un groupe partant; ou
(C) la réaction d'un composé de formule X, ou d'un de ses dérivés protégés, avec un composé de formule XI, ou un de ses dérivés protégés: 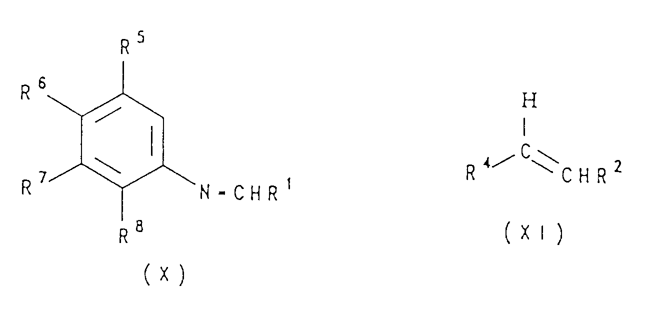 dans lesquels R¹, R² et R⁵ à R⁸, sont tels que définis dans la revendication 1, et R⁴ est tel que défini ci-dessus, suivi, si nécessaire, par une élimination des groupes protecteurs.
